# EUROPEAN PATENT APPLICATION

(11) **EP 4 850 758 A2**
(43) Date of publication of application: **09.12.2026**
(21) Application number: 26205582.5
(22) Date of filing: 23.08.2023
(51) Int. Cl.: A61P 25/04

(54) **BRAIN PENETRATING PEPTIDES AND METHODS OF USE THEREOF**

(62) Document number(s) of the earlier application(s) in accordance with Art. 76 EPC: 23764718.5 / 4 577 229
(71) Applicant: University of Tartu, 50090 Tartu (EE)
(72) Inventor: PÕSNOGRAJEVA, Kristina, 50403 Tartu (EE); HAUGAS, Maarja, 51007 Tartu (EE); TEESALU, Tambet, 67216 Tartumaa (EE); TOOME, Kadri, 60532 Tartu vald (EE)
(74) Representative: Williams Powell

(57) **Abstract**

Polypeptides that traverse the blood-brain-barrier (BBB) are disclosed. The peptide can serve as BBB-traversing transport vehicles for delivering active agents from the circulation into across the BBB to contact cells and tissues within the CNS and/or brain. Therefore, composition of conjugates including polypeptides that traverse the BBB, conjugated with one or more active agents are provided. The BBB-traversing peptides can be a fusion protein that includes one or more additional domains such as a targeting moiety, a linker, an active agent, a purification tag, or any combination thereof. Exemplary BBB-traversing peptides include polypeptides having an amino acid sequence RRVISRAKLAAAL (SEQ ID NO:1), or a functional variant thereof, or the amino acid sequence of CVGTNCY (SEQ ID NO:2).

## Description

### FIELD OF THE INVENTION

The field of the invention generally relates to compositions for traversing the healthy blood-brain-barrier (BBB) and methods of use thereof for delivery of therapeutic agents from the circulation into the brain.

### BACKGROUND OF THE INVENTION

Brain cancer is a devastating disease. Despite surgical and medical advances, the prognosis for most brain cancers remains dismal. The median survival times for glioblastoma - the most common malignant glioma in adults (Scott CB, et al., International Journal of Radiation Oncology, Biology, Physics, 40(1): 51-55 (1998)), diffuse intrinsic pontine glioma - the most common type of brainstem glioma in children (Khatua S, et al., Childs Nerv Syst, 27(9):1391-1397 (2011)), and brain metastasis (Jaboin JJ, et al., Radiat Oncol, 8 (2013)) are 14 months, 9 months, and 12 months, respectively. Novel therapeutic approaches with improved efficacy for these tumors are urgently needed.

Gene therapy is an effective approach for the treatment of a variety of tumors. However, its application of gene therapy to brain tumors is limited by the lack of efficient delivery platforms that are able to simultaneously overcome the blood-brain barrier (BBB) and cellular barriers. Although local BBB disruption is observed in large brain tumors, these "leaky" blood vessels are located primarily in the tumor center and the capillaries feeding the proliferating tumor edge remain impermeable (Blakeley J., Curr Neurol Neurosci Rep, 8(3): 235-241 (2008)).

The BBB can potentially be bypassed using invasive methods, such as surgical implantation of degradable Gliadel^{®} wafers, or locoregional administration of Poly(lactic-co-glycolic acid) (PLGA) brain-penetrating nanoparticles (NPs) that were recently developed (Strohbehn G, et al., Journal of Neuro-oncology, 121(3):441-449 (2015); Zhou J, et al., Proc Natl Acad Sci USA, 110(29):11751-11756 (2013)). Unfortunately, the clinical utility of these approaches is hampered by their highly invasive nature. In addition, restricted drug penetration to distant tumor cells that are separate from the tumor bulk limits their therapeutic efficacy (Fung LK, et al., Pharmaceutical Research, 13(5):671-682 (1996); Fung LK, et al., Cancer Research, 58(4):672-684 (1998)). Therefore, next-generation brain therapy requires the development of novel technologies that are amenable to systemic delivery and able to target tissues throughout the brain.

Nanotechnology represents one of the most promising approaches for intravenous delivery of therapeutic agents to the brain (Deeken JF, et al., Clinical Cancer Research*:* An Official Journal of the American Association for Cancer Research, 13(6):1663-1674 (2007); Patel T, et al., Advanced Drug Delivery Reviews, 64(7):701-705 (2012); Zhou J, et al., Cancer J, 18(1):89-99 (2012)). The primary benefit of nanotechnology is that particles and/or macromolecules can be engineered to take advantage of many mechanisms for brain-targeting delivery including: 1) receptor-mediated transcytosis (Qiao R, et al., ACS Nano, 6(4):3304-3310 (2012)); 2) carrier-mediated transcytosis (Li J, et al., Biomaterials, 34(36):9142-9148 (2013)); 3) adsorptive-mediated transcytosis (Liu L, et al., Biopolymers, 90(5):617-623 (2008)); 4) physical disruption of the BBB (Nance E, et al., Journal of Controlled Release: Official Journal of the Controlled Release Society, 189:123-132 (2014)); and 5) disease microenvironment-targeted delivery (Kievit FM, et al., ACS Nano, 4(8):4587-4594 (2010)).

Despite its promise, nanotechnology for systemic delivery of active agents to the brain is still in its infancy. Existing engineering approaches often fail to enhance systemic delivery of therapeutic agents to the brain to a degree sufficient for treatment purposes (Deeken JF, et al., Clinical Cancer Research: An Official Journal of the American Association for Cancer Research, 13(6):1663-1674 (2007); Patel T, et al., Advanced Drug Delivery Reviews, 64(7):701-705 (2012); Zhou J, et al., Cancer J, 18(1):89-99 (2012)).

Current methods of delivering therapeutic agents across the blood brain barrier to treat the myriad of pathologies associated with diseases and disorders of the brain and CNS are insufficient.

Therefore, it is an object of the invention to provide compositions that traverse the healthy blood brain barrier.

It is also an object of the invention to use compositions that traverse the healthy blood brain barrier for delivering active agents from the circulation to the brain and CNS.

It is a further object of the invention to treat or prevent one or more symptoms of a disease or disorder in the brain or CNS in a subject.

It is also a further object of the invention to identify or monitor one or more physiological markers of a disease or disorder in the brain or CNS in a subject.

It is a further object of the invention to treat or prevent one of more symptoms of neurological disease diseases and disorders in a subject.

### SUMMARY OF THE INVENTION

Polypeptides that specifically and selectively traverse the healthy blood-brain barrier (BBB) with high efficiency and without toxicity have been developed. The BBB-penetrating peptides include a BBB-traversing domain including between 5-50 amino acids inclusive.

Brain penetrating peptides that cross the blood brain barrier (BBB), and conjugates and compositions thereof are provided. In an exemplary form, the compositions include a BBB-traversing peptide domain having an amino acid sequence that includes or is RRVISRAKLAAAL (SEQ ID NO:1), or a functional variant thereof. In another exemplary form, the compositions include a BBB-traversing peptide domain having an amino acid sequence that includes or is CVGTNCY (SEQ ID NO:2) (referred to as "CVG" peptide), or a functional variant thereof.

In some forms, the BBB-traversing domain is at least 13 amino acids in length. For example, in some forms, the brain penetrating peptide includes between 5-20 contiguous amino acids of any one or more of SEQ ID NOs:3-10. In some forms, the brain penetrating peptide domain is any one of SEQ ID NOs:3-10, or a functional variant thereof having an amino acid sequence at least 70%, 75%, 80%, 85%, 90%, 95%, or 99% identical to any one of SEQ ID NOs: 1 or 3-10. In other forms, the BBB-traversing domain is at least 7 amino acids in length. For example, in some forms, the brain penetrating peptide includes between 5-10 contiguous amino acids of one or more of SEQ ID NOs: 11-20, or 22-23, or a functional variant thereof. In some forms, the brain penetrating peptide domain is any one of SEQ ID NOs:11-20, or 22-23. Typically, the amino acid sequence of the BBB-traversing domain is not CAGALCY (SEQ ID NO:21).

In some forms, the brain penetrating peptide, or conjugate or composition thereof selectively homes to the brain parenchyma, endothelial cell, or the whole brain when administered *in vivo*. In some forms, when the BBB-traversing domain is or includes SRRVISRAKLAAALE (SEQ ID NO:3) or MLGDPILASRRVISRAKLAAALE (SEQ ID NO:4), the brain penetrating peptide selectively homes to cells of the brain parenchyma.

BBB-traversing peptide conjugates including (a) a brain penetrating peptide that is or includes the amino acid sequence of any one of SEQ ID NOs:1-20, 22-23, or a functional variant thereof; and (b) a cargo molecule that is directly or indirectly conjugated to or complexed with the brain penetrating peptide are also described. Typically, the cargo molecule does not traverse the BBB in the absence of the brain penetrating peptide. Exemplary cargo molecules include active agents selected from the group including a therapeutic agent, a diagnostic agent, a prophylactic agent, and a nutraceutical agent. In some forms, the cargo molecule is encapsulated within or conjugated to a carrier. In some forms, the carrier encapsulates or is complexed with the one or more active agents and is directly or indirectly conjugated to the brain penetrating peptide. Exemplary carriers include a polymeric particle, a lipid particle, a liposome, a gel, an inorganic particle, a viral particle, a nucleic acid nanostructure, and a virus-like particle. In some forms the one or more active agent(s) and/or the carrier is conjugated to the brain penetrating peptide by one or more linkers. In particular forms, one or more of the linkers are cleavable linkers.

In some forms, the active agent is a therapeutic agent selected from the group including a nucleic acid, peptide, lipid, glycolipid, glycoprotein, and small molecules. Exemplary nucleic acid therapeutic agents include antisense molecules, aptamers, ribozymes, triplex forming oligonucleotides, external guide sequences, RNAi, CRISPR/Cas, zinc finger nucleases, and transcription activator-like effector nucleases (TALEN). In other forms the therapeutic agent is a small molecule. Exemplary therapeutic agents include an anti-cancer agent, an anti-inflammatory agent, an immunomodulatory agent, and an antimicrobial agent.

Pharmaceutical compositions including the peptide conjugates together with and a pharmaceutically acceptable excipient for administration to a subject *in vivo* are also provided. Exemplary pharmaceutical compositions are formulated to be suitable for mucosal, pulmonary, intravenous (*iv*), or intramuscular (*im*) delivery.

Methods of treating or preventing one or more symptoms of a disease or disorder in the brain or CNS of a subject by administering a pharmaceutical composition including the BBB-traversing peptide conjugates to the subject are also provided. Typically, the methods include administering to the subject an effective amount of a pharmaceutical composition including the BBB-traversing peptide conjugates to prevent or reduce one or more symptoms of a disease or disorder in the brain or CNS of the subject.

In some forms, the active agent is a diagnostic agent selected from a dye, a radionuclide, a fluorescent tag, a magnetic tag and a nanoparticle. Pharmaceutical compositions including the BBB-traversing peptide conjugated with a diagnostic agent together with a pharmaceutically acceptable excipient for administration to a subject *in vivo* are also provided. Exemplary pharmaceutical compositions are formulated to be suitable for mucosal, pulmonary, intravenous (*iv*), or intramuscular (*im*) delivery.

Methods of detecting or monitoring a disease or disorder in the brain or CNS of a subject by administering to the subject an effective amount of a pharmaceutical composition including the BBB-traversing peptide conjugated with a diagnostic agent are also provided. The methods detect or monitor a disease or disorder in the brain or CNS of the subject in the subject.

Typically, the subject is a human. In some forms the subject is an infant or a child. In some forms, the subject has, or is suspected as having a disease or disorder. In some, forms the subject has an underlying disease or disorder that exacerbates or presents the disease that is to be treated by the methods. Exemplary diseases and disorders include cancer, an inflammatory disease, a neuronal disorder, HIV/AIDS, a diabetes, a cardiovascular disease, an infectious disease (including a viral, a protozoan, a bacterial disease, and an allergy), an autoimmune disease and an autoimmune disease, Alzheimer's disease, Parkinson's disease, ischemia, a neurodegenerative disorder, and a genetic disorder. In particular forms, the disease is a cancer, such as a glioma. In other forms the disease is a neurodegenerative disease, such as Alzheimer's disease. In particular forms, the disease is Parkinson's disease. In some forms the subject has an infection.

The present invention will be further understood by reference to the following description.

### BRIEF DESCRIPTION OF THE DRAWINGS

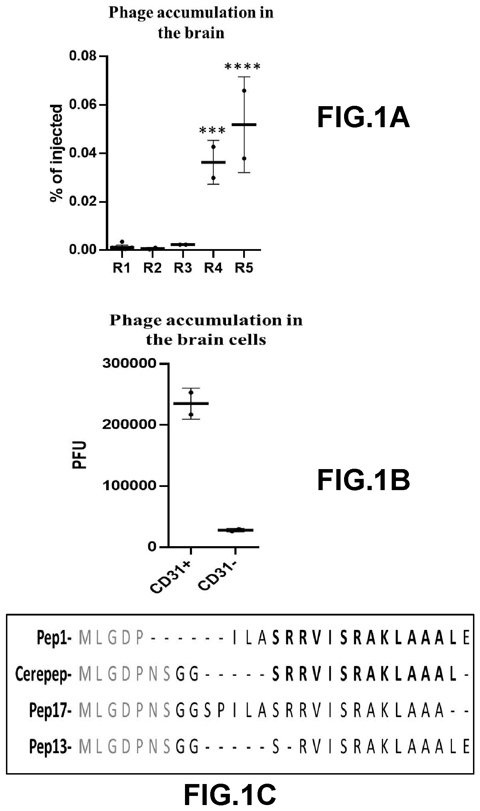

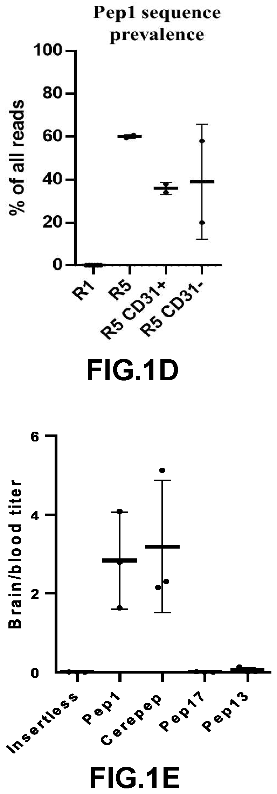

**Figures 1A, 1B****,** **1D, and 1E** are graphs and **Figure 1C** compares sequences. **Fig. 1A** shows the % of injected phage accumulated in the brain throughout the five rounds of bio-panning (R1-R5). Peptide-phages were injected *i.v*. and following 30 min circulation mice were perfused with PBS. The phages rescued from the brain were reamplified and injected for a subsequent round of bio-panning. N=8 in R1 and n=2 in R2-R5. *** p ≤ 0.0001, **** p < 0.0001. **Fig. 1B** shows the total amount of phage rescued from mouse brain (PFU) for each of CD31+ and CD31- cell fractions after R5 with the additional step of isolating brain endothelial (CD31+) cells from all the other brain cells (parenchyma cells; CD31-) N=2. **Fig. 1C** shows an amino acid sequence alignment for peptides identified in screening experiments, including MLGDPILASRRVISRAKLAAALE (SEQ ID NO:4) (the functional fragment SRRVISRAKLAAALE (SEQ ID NO:3) is termed "Pep1"); MLGDPNSGGSRRVISRAKLAAAL (SEQ ID NO:5) (the functional fragment SRRVISRAKLAAAL (SEQ ID NO:9) is termed "Cerepep"); MLGDPNSGGSPILASRRVISRAKLAAA (SEQ ID NO:65) (termed "Pep17"); and MLGDPNSGGSRVISRAKLAAALE (SEQ ID NO:7) (termed "Pep13"). Residues in bold text are a functional component associated with penetration into the brain. **Fig. 1D** shows the prevalence of Pep1 sequence in high throughput sequencing data, shown as a percentage of all reads. **Fig. 1E** shows peptide-phage brain homing (brain/blood titer; 0-6) for each of Insertless, Pep1, Cerepep, Pep17, and Pep13 peptides. N=3, * p ≤ 0.05, ** p ≤ 0.01. All data are shown as mean with SD.

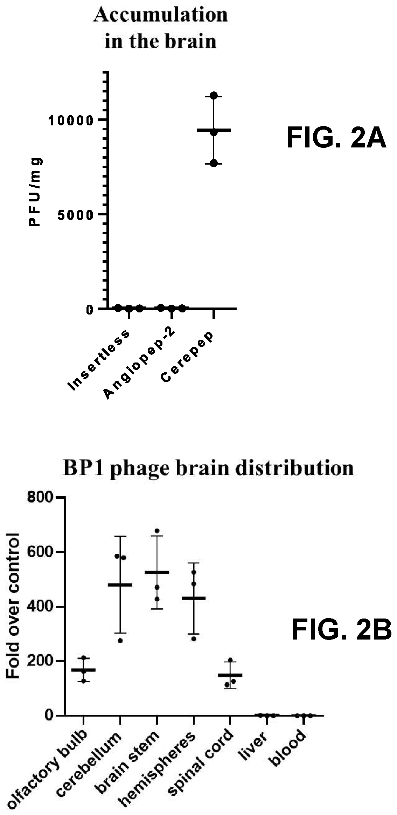

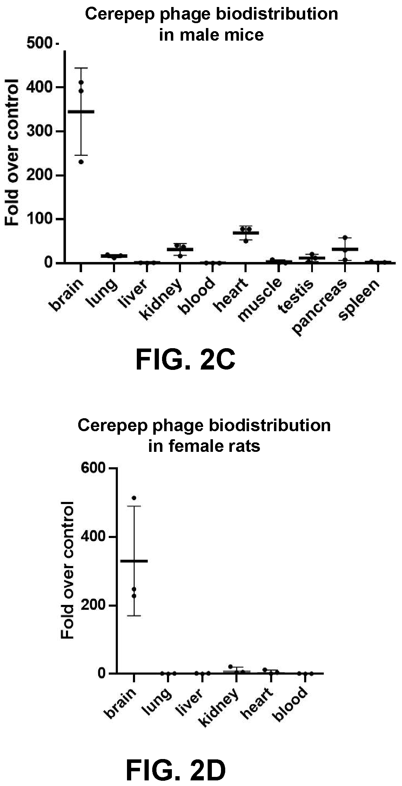

**Figures 2A-2D** are graphs demonstrating accumulation of peptide-phage in brains of male and female mouse and rat brains. **Fig. 2A** shows accumulation in the brain (PFU/mg) for phage expressing no peptide (Insertless), Angiopep-2 and Cerepep (SRRVISRAKLAAAL; (SEQ ID NO:9)) peptides. **Fig. 2B** shows Cerepep phage brain distribution (0-800 fold over control) in each of olfactory bulb, cerebellum, brain stem, hemisphere, spinal cord, liver and blood. **Fig. 2C** shows Cerepep phage brain distribution in male mice (0-800 fold over control) in brain, lung, liver, kidney, blood, heart, muscle, testis, pancreas and spleen. **Fig. 2D** shows Cerepep phage brain distribution in female mice (0-600 fold over control) in brain, lung, liver, kidney, blood, and heart.

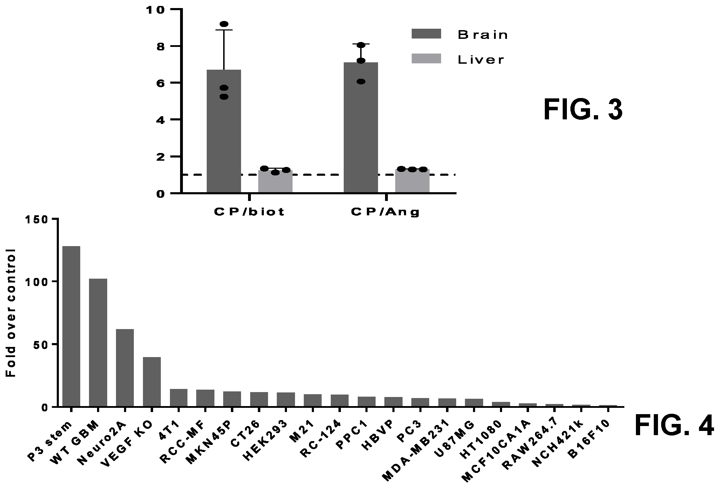

**Figure 3** is a histogram showing the quantification of silver nanoparticles (AgNPs) Cerepep (CP)/biotin accumulation in each of brain and liver compared to biotin-NPs and Cerepep (CP)/Angiopep-2. All data are shown as mean with SD, n=3.
**Figure 4** is a histogram of binding of BP1 peptide-phage to different cell lines shown as fold over insert-less phage, showing the Fold binding over control (0-150) for each of P3 stem, WT GBM, Neuro 2A, VEGF KO, 4T1, RCC-MF, MKN45P, CT26, HEK293, M21, RC124, PPC1, HBVP, PC3, MDA-MB231, U87MG, HT1080, MCF10CA1A, RAW264.7, NCH421K and B16F10.

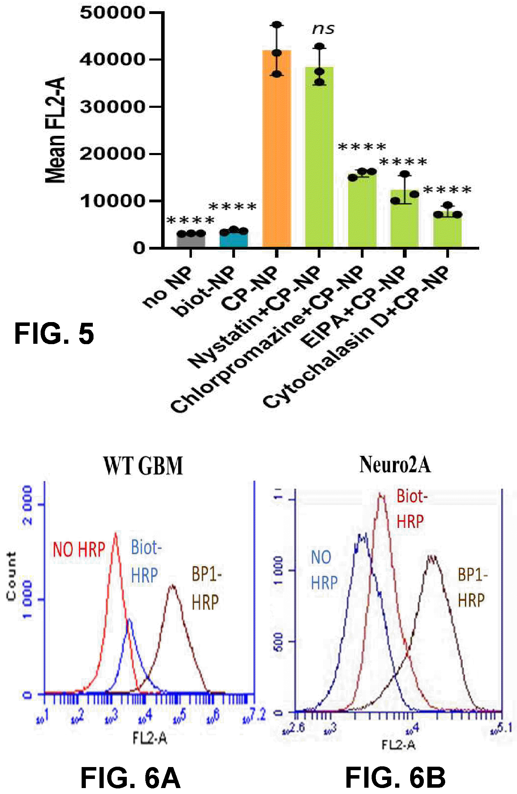

**Figure 5** **is** a graph of data from flow cytometry analyses showing the effect of cell uptake inhibitors on the internalization of Cerepep (BP)-AgNPs into Neuro2A cells incubated with samples including no nanoparticle control (No NP), biotinylated nanoparticles (Biot-NP), Cerepep nanoparticles (BP_NP) and each of Chlorpromazine, Nystatin, cytochalasin D, and 5-(N-ethyl-N-isopropyl)-amiloride (EIPA); respectively. The data are quantified as Mean fluorescence (FL2-A) for each sample.
**Figures 6A and 6B** are data from flow cytometry to determine peptide-guided horseradish peroxidase (HRP)-mediated biotinylation of cellular proteins (proximity ligation assay). Counts for WT GBM (**Fig. 6A**) and Neuro2A (**Fig. 6B**) cells incubated with no HRP, Biot-HRP and Cerepep (BP1)-HRP are shown.

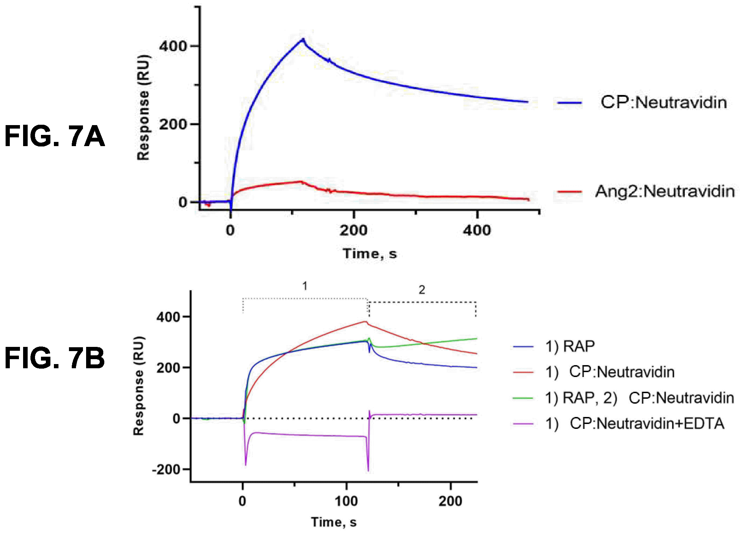

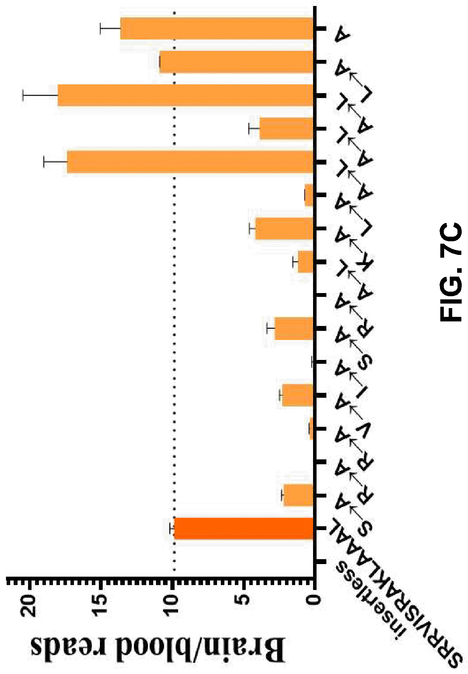

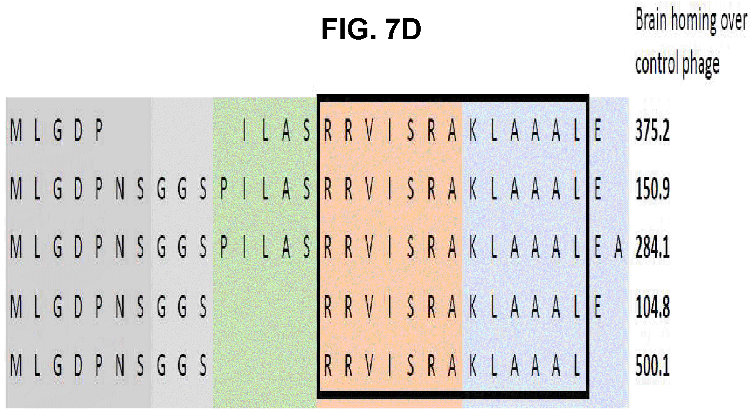

**Figures 7A-7D** are data from Surface plasmon resonance analysis with human LRP-1 immobilized on a BIAcore sensor chip. **Fig. 7A** shows response (0-500 RU) over time (0-500 seconds) of Cerepep (CP)-neutravidin complex and Angiopep-2-neutravidin complex (used as a positive control). **Fig. 7B** shows the binding of Cerepep (CP)-neutravidin complex in the presence or absence of RAP and/or EDTA. RAP and Cerepep (CP)-neutravidin are used as controls. **Fig. 7C** shows brain/blood reads (0-25) for the Cerepep (SRRVISRAKLAAAL; (SEQ ID NO:9)) peptide in which every residue in each position of the peptide is independently mutated to alanine (alanines were changed to leucines), as indicated. The brain homing of each mutated peptide-phage was assessed using *in vivo* play-off method. **Fig. 7D** shows different peptide sequences and the fold brain homing over control phage for each of the peptides listed, including MLGDPILASRRVISRAKLAAALE (SEQ ID NO:4); MLGDPNSGGSPILASRRVISRAKLAAALE (SEQ ID NO:71); MLGDPNSGGSPILASRRVISRAKLAAALEA (SEQ ID NO:72); MLGDPNSGGSRRVISRAKLAAALE (SEQ ID NO:73); and MLGDPNSGGSRRVISRAKLAAAL (SEQ ID NO:5).
   The residues MLGDPNS (SEQ ID NO:69) are part of the phage expression system; the residues GGS are linker residues; residues PILAS (SEQ ID NO:98) are identified in the screen and do not appear essential for brain homing; residues RRVISRAKLAAAL (SEQ ID NO:1) appear to be necessary for brain homing.

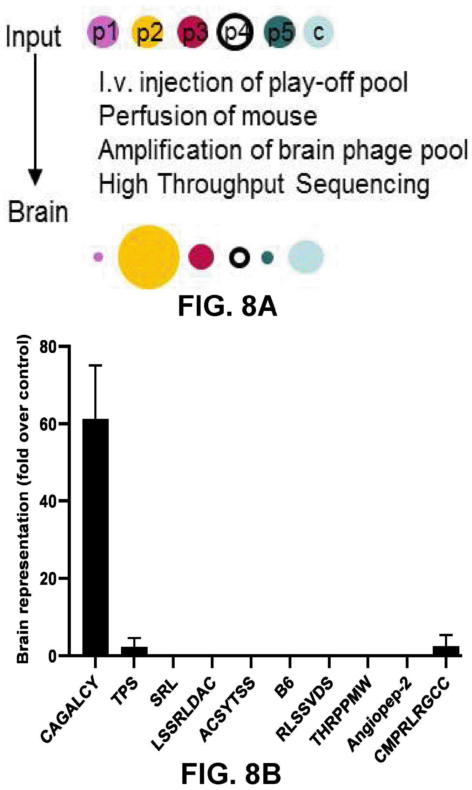

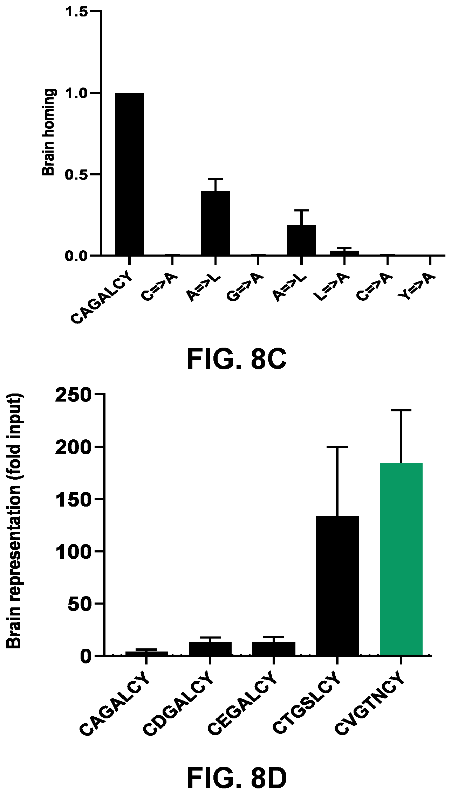

**Figures 8A-8D** show identification of brain homing peptides including the CAGALCY (SEQ ID NO:21) peptide, and variants thereof. **Fig. 8A** is a flow-chart of the experimental design of the internally-controlled in vivo play off experiment, listing the sequence of steps including iv injection of phage pool, perfusion, amplification of phage from brain and high throughput sequencing for each of p1, p2, p3 p4, p5 and control(c) groups, respectively (depicted as circles at top of workflow). The relative amount of each of the pooled sequences is depicted by the relative sizes of the circles at bottom of workflow. **Fig. 8B** is a histogram showing brain representation (fold over control 0-80) for each of the peptides listed, including CAGALCY (SEQ ID NO:21); TPSYDTAAELR (SEQ ID NO:33; labeled "TPS"); CLSSRLDA (SEQ ID NO:25; labeled "SRL"); LSSRLDAC (SEQ ID NO:26); ACSYTSSTMCGGGS (SEQ ID NO:27; labeled "ACSYTSS"); CGHKAKGPRK (SEQ ID NO:28; labeled "B6"); RLSSVDSDLSGC (SEQ ID NO:29; labeled "RLSSVDS"); THRPPMWSPVWP (SEQ ID NO:30; labeled "THRPPMW"); TFFYGGSRGKRNNFKTEEYC (SEQ ID NO:31; labeled "Angiopep-2"); and CMPRLRGCC (SEQ ID NO:32). **Fig. 8C** shows brain homing (0-1.5) as a function of the CAGALCY (SEQ ID NO:21) peptide for each of the mutations listed. **Fig. 8D** shows brain representation (fold input 0-250) as a function of the CAGALCY (SEQ ID NO:21) peptide for each of the peptides listed, including CDGALCY (SEQ ID NO:34); CEGALCY (SEQ ID NO:24); CTGSLCY (SEQ ID NO: 12); and CVGTNCY (SEQ ID NO:2).

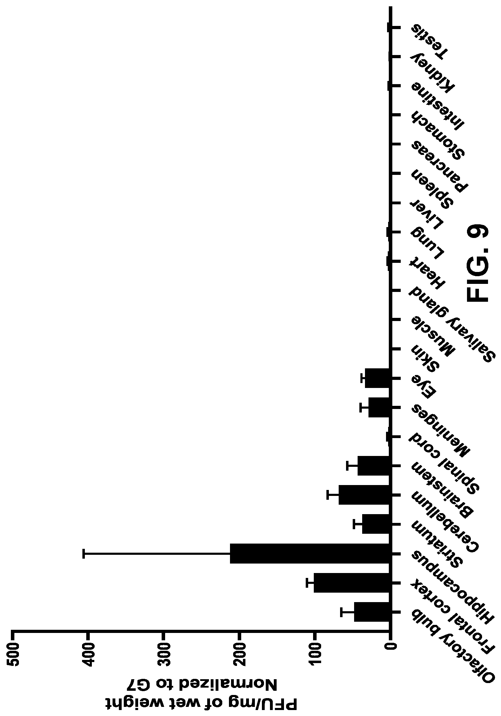

**Figure 9** is a histogram of biodistribution analysis of CVGTNCY (SEQ ID NO:2) peptide-phage in the mouse CNS and in control organs, showing PFU/mg wet weight normalized to G7 for each of the organs listed. The CVGTNCY (SEQ ID NO:2) peptide is referred to as the "CVG" peptide.

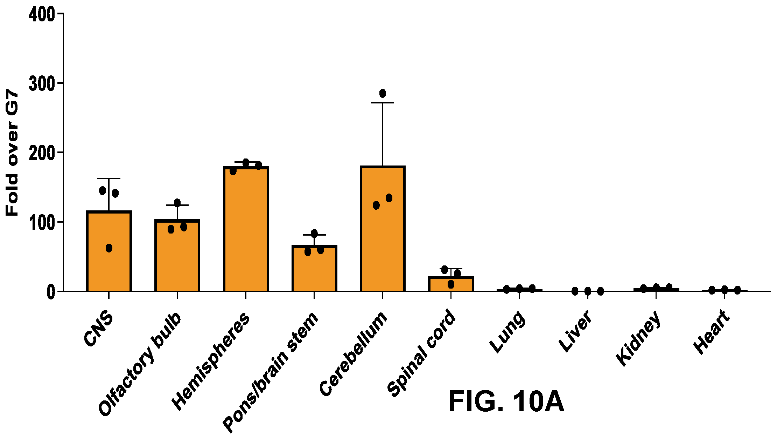

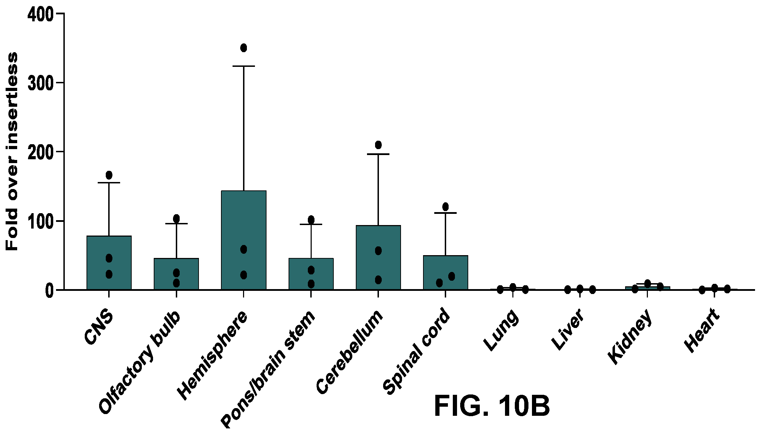

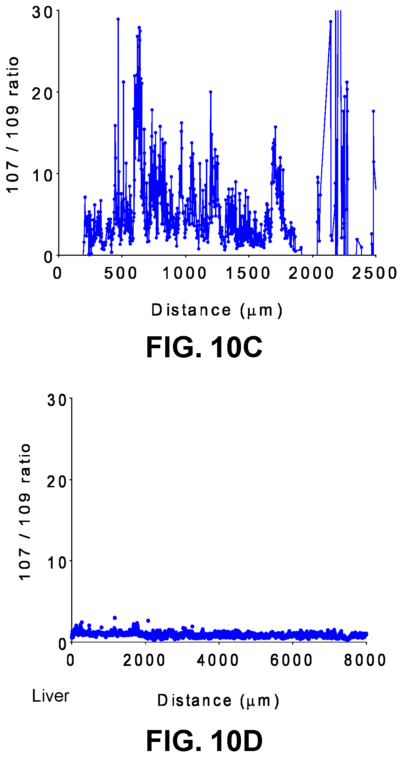

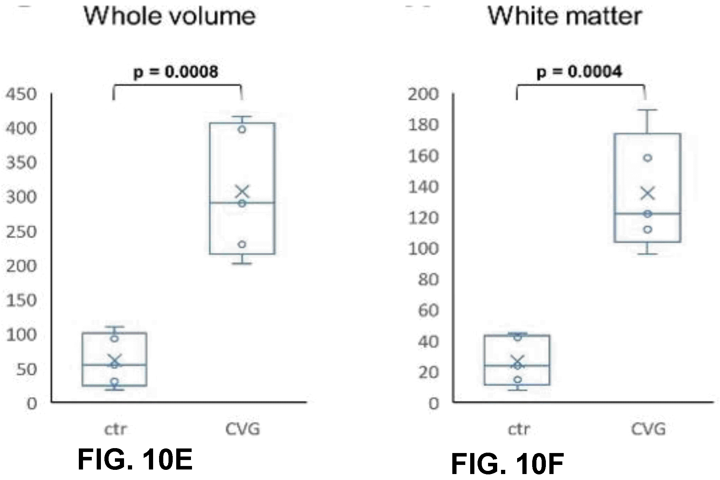

**Figures 10A** and **10B** are histograms showing homing of iv CVGTNCY (SEQ ID NO:2) peptide-displaying phage in mice (expressed fold over G7 peptide displaying phage, **Fig. 10A**) or rats (expressed fold over insert-less, **Fig. 10B**) for each of the organs listed. **Figures 10C** and **10D** are graphs showing ratio of Ag107/109 from mouse brain (**Fig. 10C**) and control organ (liver) (**Fig. 10D**) over distance (µm), respectively. The mice were injected with an equimolar mixture of Ag107 nanoparticles functionalized with CVGTNCY (SEQ ID NO:2) peptide and control nanoparticles made from Ag109 and tissue cryosections were subjected to line scan using laser ablation ICP-MS analysis. **Figures 10E** and **10F** are box plots for distribution of CVGTNCY (SEQ ID NO:2) peptide Ag nanoparticles or controls Ag nanoparticles in the brain whole volume (**Fig. 10E**) and white matter (**Fig. 10F**), respectively.

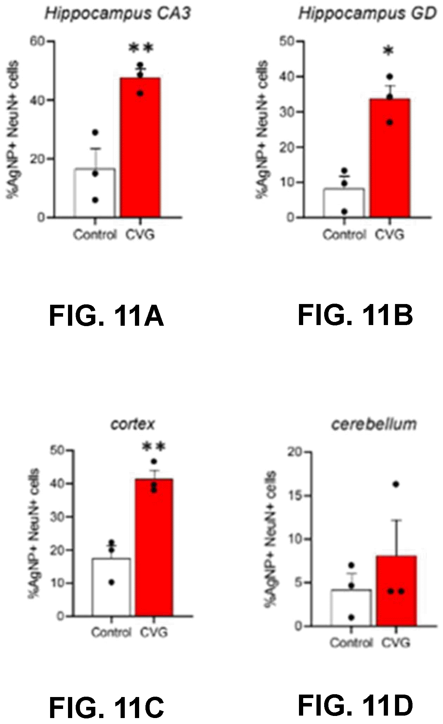

**Figures 11A-11D** are histograms showing %AgNP+NeuN+ cells for control and CVG peptides in each of Hippocampus CA3 (**Fig. 11A**), Hippocampus GD (**Fig. 11B**), cortex (**Fig. 11C**), cerebellum (**Fig. 11D**), respectively.

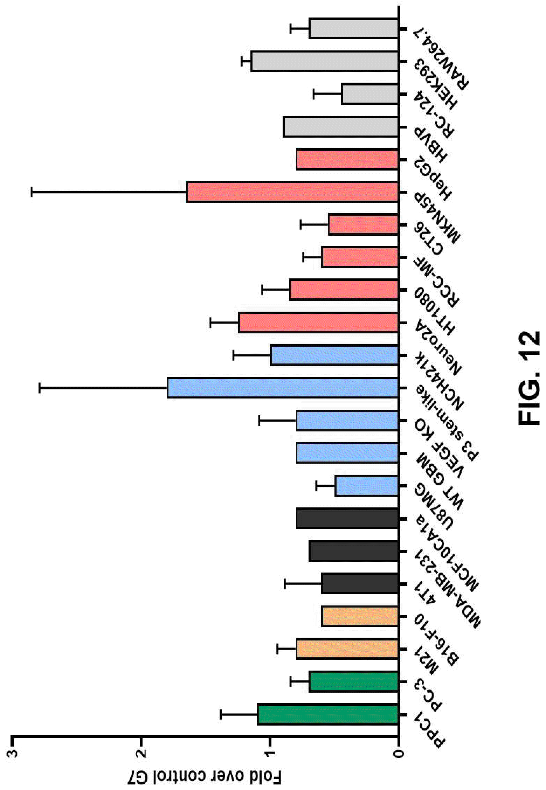

**Figure 12** is a histogram showing Fold over control G7 for each of multiple different tumorigenic and non-tumorigenic cell lines.

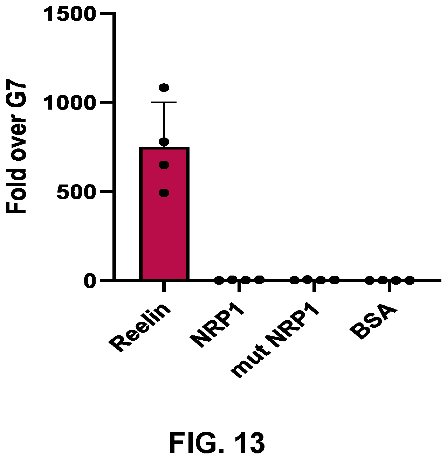

**Figure 13** is a histogram showing 0-1,500 Fold over control G7 for each of Reelin, NRP1, mut NRP1 and BSA, respectively.

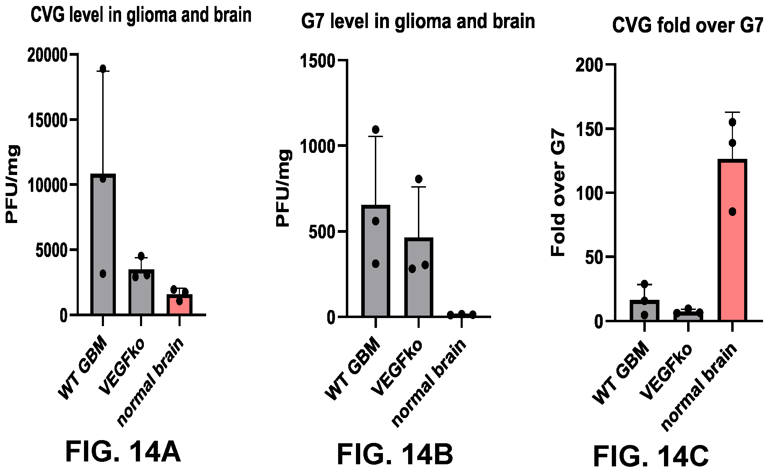

**Figures 14A-14C** are histograms. **Fig. 14A** shows CVG peptide level in glioma and brain, showing 0-20,000 PFU/mg for each of WT GBM, VEGFKo, and normal brain, respectively. **Fig. 14B** shows G7 level in glioma and brain, showing 0-1500 PFU/mg for each of WT GBM, VEGFKo, and normal brain, respectively. **Fig. 14C** shows CVG fold over G7 (0-200) for each of WT GBM, VEGFKo, and normal brain, respectively.

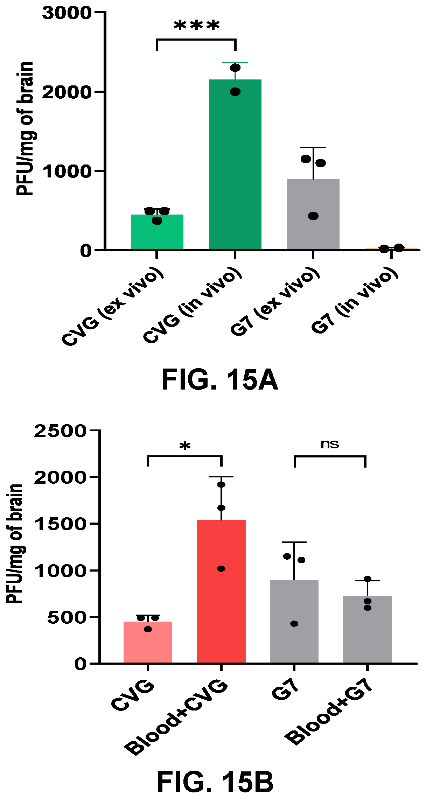

**Figures 15A-15B** are histograms. **Fig. 15A** shows PFU/mg of brain (1,000-3,000) for phage coupled to each of CVGTNCY (CVG; SEQ ID NO:2) (*ex vivo*), CVG (*in vivo*), G7 control phage only (*ex vivo*), and G7 (*in vivo*), respectively; N=3; *** p<0.0005. **Fig. 15B** is a graph demonstrating that CVGTNCY (CVG; SEQ ID NO:2) peptide-phage binding to brain tissue homogenate *ex vivo* is restored in the presence of whole blood sample, showing PFU/mg of brain (1,000-2,500) for phage coupled to each of CVG, Blood+CVG, G7 (control) alone, and Blood+G7, respectively; N=3; * p<0.05.

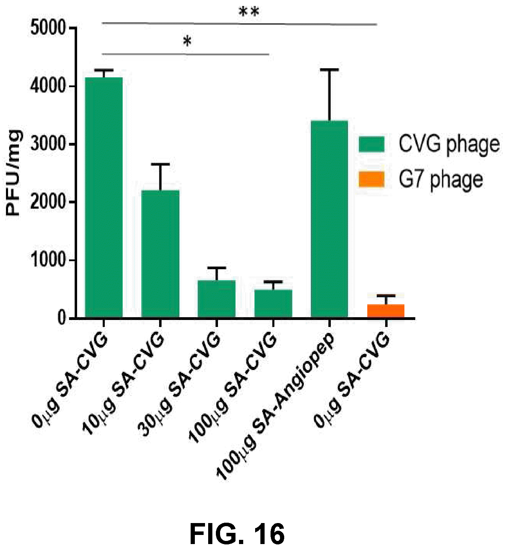

**Figure 16** is a histogram depicting saturation of CVGTNCY (CVG; SEQ ID NO:2) receptor in the brain, showing PFU/mg (0-5,000) for each of several concentrations (0, 10, 30, 100 µg, respectively) of the CVG peptide-streptavidin complex (SA-CVG). Angiopep2-peptide streptavidin complex (SA-Angiopep) and G7 phage were used as controls, respectively; graph shows mean and SD. N=3, * p<0.05; ** p<0.005.

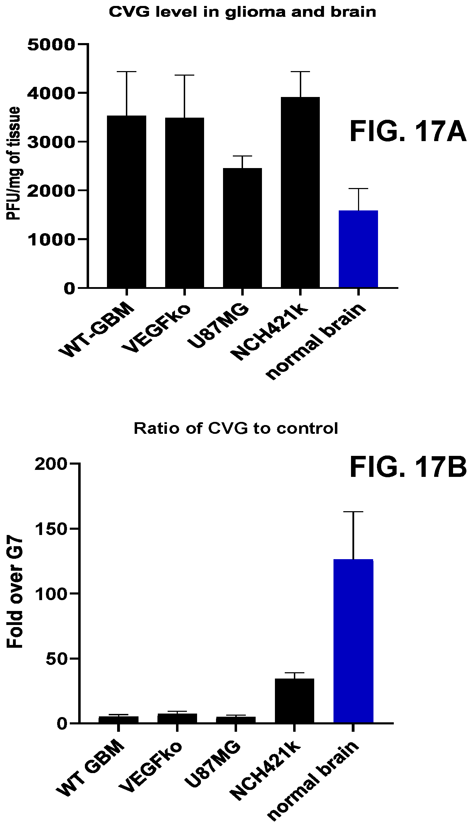

**Figures 17A-17B** are histograms of homing of CVGTNCY peptide in glioblastoma. **Fig. 17A** shows CVGTNCY (CVG; SEQ ID NO:2) level in glioma and brain (1,000-5,000PFU/mg of brain) for each of different mice, including WT-GBM, VEGFko, U87MG or NCH421k GBM, and to Normal brain (mice without a tumor), respectively. **Fig. 17B** shows the ratio of CVGTNCY (CVG; SEQ ID NO:2) to control (0-200 Fold over G7).

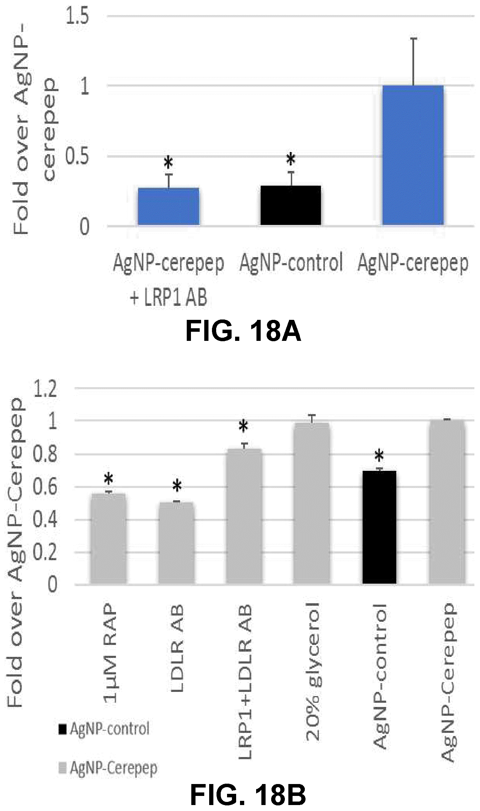

**Figures 18A-18B** are histograms demonstrating that binding of AgNP-Cerepep to P3 human stem cells is reduced by inhibitor of several LDL-receptor proteins, Receptor Associated Protein (RAP) and anti LRP-1 antibodies. **Fig. 18A** shows LRP1 antibody (AB) binding as fold over AgNP-Cerepep binding (0-1.5) for each of AgNP-Cerepep+LRP1 AB, AgNP-control, and AgNP-Cerepep, respectively. **Fig. 18B** shows LRP1 antibody (AB) binding as fold over AgNP-Cerepep binding (0-1.2) for each of RAP, LDLR antibody (AB), and both LRP1 antibody (AB) + LRP1 antibody (AB) and 20% glycerol, AbNP-control and AgNp-Cerepep, respectively; Experiments were performed in three replicates per condition and the error bars show the standard deviation. * Statistical significance, conditions were compared to AgNP-Cerepep.

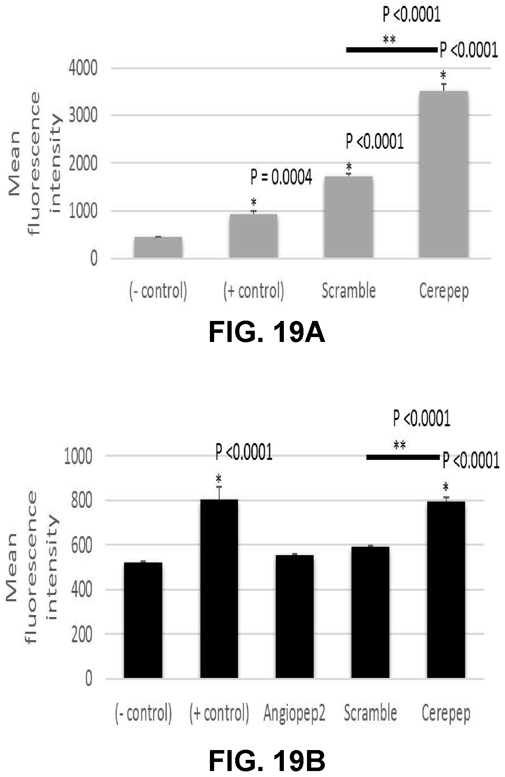

**Figures 19A-19B** are histograms demonstrating that Cerepep interacts with LRP-1 cluster II and cluster III. **Fig. 19A** shows Mean Fluorescence Intensity (0-4,000) for beads incubated overnight with 10 nM LRP-1 cluster II with each of -ve control (biotin), +ve control (beads incubated with biotinylated-Angiopep2 peptide, LRP-1 cluster II protein and Alexa Fluor 647 anti-human LRP-1 antibody), Scramble peptide and Cerepep, respectively. **Fig. 19B** shows Mean Fluorescence Intensity (0-1,000) for beads incubated overnight with 10 nM LRP-1 cluster III with each of each of -ve control (biotin), +ve control (beads incubated with biotinylated-RPAR peptide, Neuropilin-1 b1b2 protein, (1:200) anti-neuropilin-1 antibody, (1:200) and Alexa Fluor 647 anti-rabbit antibody), Angiopep2, Scramble peptide and Cerepep, respectively; Experient was performed in three replicates per condition and the error bars show the standard deviation. * Statistical significance, ANOVA test compared the conditions with the negative control. ** Statistical significance, t-test compared scramble sequence (SARVISRAKLARAL (SEQ ID NO:70)) with Cerepep.

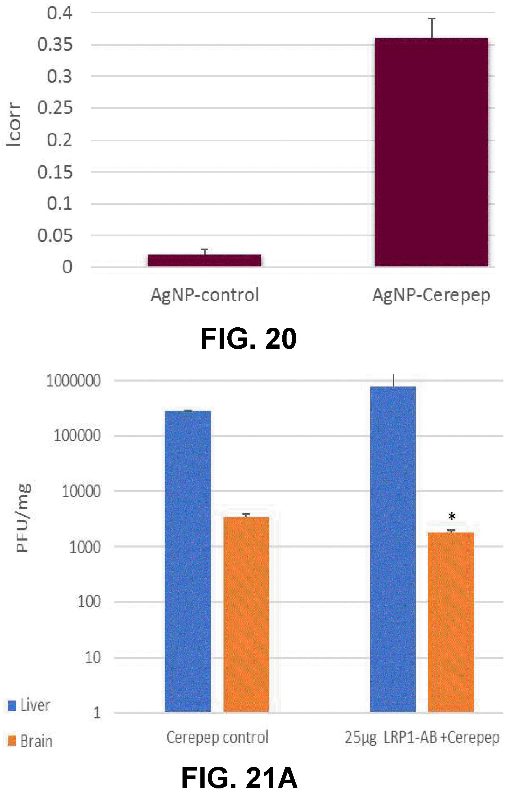

**Figure 20** is a histogram demonstrating that Cerepep-AgNPs colocalize with lysosomes, showing Mean correlation Index (0-45 Icorr) for P3 stem cells, grown on coverslips and incubated with AgNP-control, or AgNP-Cerepep and stained using anti-LAMP1 antibody and DAPI, respectively, then images overlayed/correlated as shown. Bars represent the mean correlation index (Icorr, ImageJ output). Experient was performed in three replicates and the error bars indicate the standard deviation. * Statistical significance.

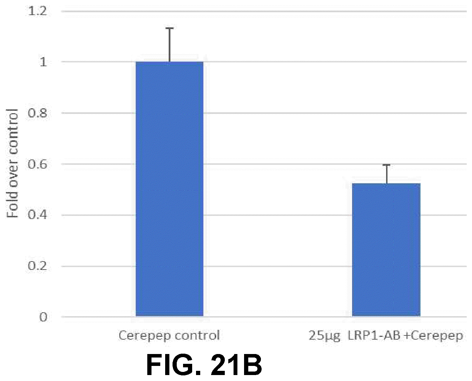

**Figures 21A-21B** are histograms demonstrating that the LRP1 antibody inhibits the binding of Cerepep in the brain. **Fig. 21A** shows PFU/mg (1-100,000,000) for each of Cerepep control in liver, Cerepep control in brain, 25 µg LRP1-AB+Cerepep in liver, and 25 µg LRP1-AB+Cerepep in brain, respectively. **Fig. 21B** shows the fold-over control phage (0-1.2) in brain for each of Cerepep control and 25 µg LRP1-AB+Cerepep, respectively. Experiment was performed in three replicates per condition and the error bars show the standard deviation. * Statistical significance, p value= 0.008.

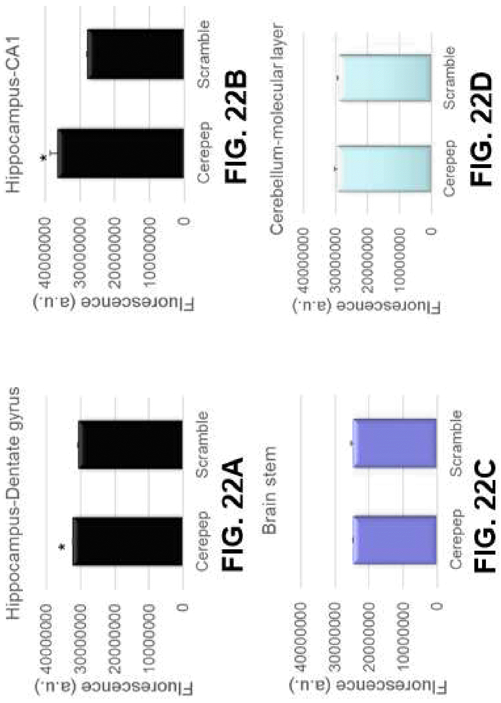

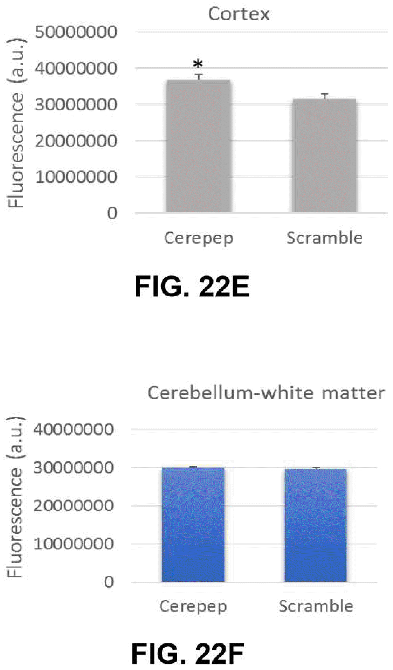

**Figures 22A-22F** are histograms demonstrating the distribution of FAM-Cerepep monomeric peptide in the brain, showing fluorescence intensity (a.u.) for each of Cerepep and Scramble peptides, respectively, in each of the brain sections Hippocampus-Dentate gyrus (**Fig. 22A**); Hippocampus-CA1(**Fig. 22B**); Brain stem (**Fig. 22C**); Cerebellum molecular layer (**Fig. 22D**); Cortex (**Fig. 22E**); and in Cerebellum white matter (**Fig. 22F**), respectively. Fluorescence intensity signal from FAM was measure using ImageJ. Experient was performed in 3 replicates. * Statistical significance.

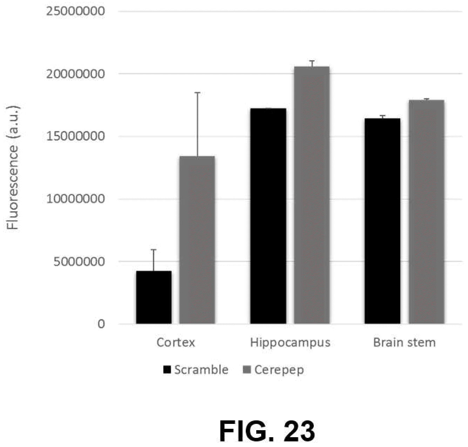

**Figure 23** is a histogram demonstrating that the HER2-FAM-Cerepep antibody crosses the blood-brain barrier, showing fluorescence (a.u.) for each of Cerepep and Scramble peptides, respectively, in each of the brain sections Cortex, Hippocampus, and Brain stem, respectively; * Statistical significance.

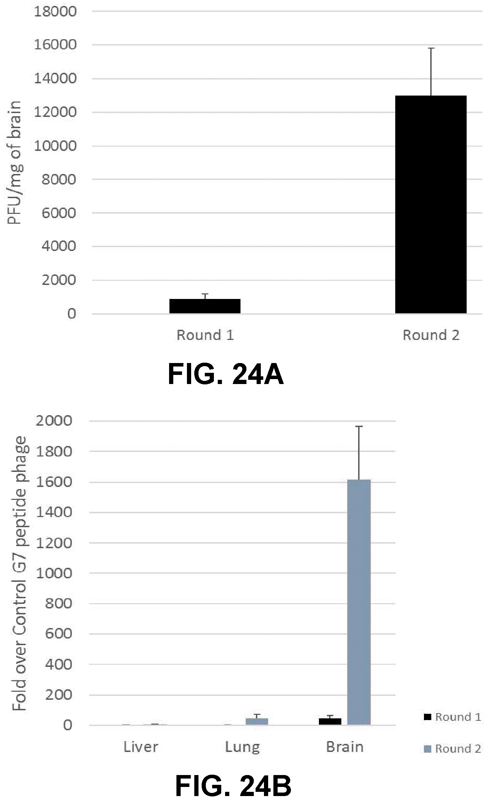

**Figures 24A-24B** are histograms demonstrating accumulation of the Cerepep library in the brain of mice in two second rounds of biopanning. **Fig. 24A** shows phage accumulation in the brain after rounds 1 and 2 of biopanning, as PFU/mg of brain (1-1800) for each of Round 1 and Round 2 biopanning, respectively. **Fig. 24B** shows the fold-over control phage for three organs (liver, lung and brain) for each of Round and Round 2 biopanning, respectively, with highest-value datapoints indicated for each, respectively. Experiment was performed in three replicates per condition and the error bars show the standard deviation.

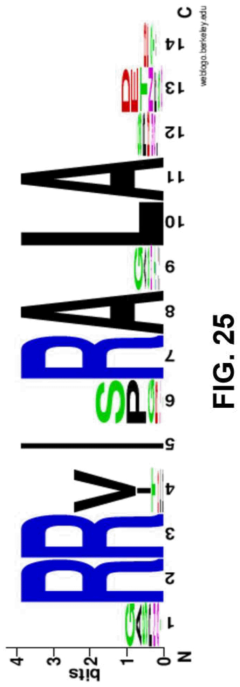

**Figure 25** is a graphical representation of compiled peptides from a constrained Cerepep library screen showing amino acids, ranked on the high throughput DNA sequencing-based representation in the brain.

### DETAILED DESCRIPTION OF THE INVENTION

### I. Definitions

As used herein, "treat" means to prevent, reduce, decrease, or ameliorate one or more symptoms, characteristics or comorbidities of an age-related disease, disorder or condition; to reverse the progression of one or more symptoms, characteristics or comorbidities of an age related disorder; to halt the progression of one or more symptoms, characteristics or comorbidities of an age-related disorder; to prevent the occurrence of one or more symptoms, characteristics or comorbidities of an age-related disorder; to inhibit the rate of development of one or more symptoms, characteristics or comorbidities or combinations thereof.

The terms "individual," "subject," and "patient" are used interchangeably herein, and refer to a mammal, including, but not limited to, rodents, simians, and humans.

The terms "reduce", "inhibit", "alleviate" and "decrease" are used relative to a control. One of skill in the art would readily identify the appropriate control to use for each experiment. For example, a decreased response in a subject or cell treated with a compound is compared to a response in subject or cell that is not treated with the compound.

The terms "increase", "induce", "activate" and "improve" are used relative to a control. One of skill in the art would readily identify the appropriate control to use for each experiment. For example, an increased response in a subject or cell treated with a compound is compared to a response in subject or cell that is not treated with the compound.

The term "polypeptides" includes proteins and fragments thereof. Polypeptides are disclosed herein as amino acid residue sequences. Those sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

"Variant" refers to a polypeptide or polynucleotide that differs from a reference polypeptide or polynucleotide but retains essential properties. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (*e.g*., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

Modifications and changes can be made in the structure of the polypeptides of in disclosure and still obtain a molecule having similar characteristics as the polypeptide (*e.g*., a conservative amino acid substitution). For example, certain amino acids can be substituted for other amino acids in a sequence without appreciable loss of activity. Because it is the interactive capacity and nature of a polypeptide that defines that polypeptide's biological functional activity, certain amino acid sequence substitutions can be made in a polypeptide sequence and nevertheless obtain a polypeptide with like properties.

In making such changes, the hydropathic index of amino acids can be considered. The importance of the hydropathic amino acid index in conferring interactive biologic function on a polypeptide is generally understood in the art. It is known that certain amino acids can be substituted for other amino acids having a similar hydropathic index or score and still result in a polypeptide with similar biological activity. Each amino acid has been assigned a hydropathic index on the basis of its hydrophobicity and charge characteristics. Those indices are: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is believed that the relative hydropathic character of the amino acid determines the secondary structure of the resultant polypeptide, which in turn defines the interaction of the polypeptide with other molecules, such as enzymes, substrates, receptors, antibodies, antigens, and the like. It is known in the art that an amino acid can be substituted by another amino acid having a similar hydropathic index and still obtain a functionally equivalent polypeptide. In such changes, the substitution of amino acids whose hydropathic indices are within ± 2 is preferred, those within ± 1 are particularly preferred, and those within ± 0.5 are even more particularly preferred.

Substitution of like amino acids can also be made on the basis of hydrophilicity, particularly, where the biological functional equivalent polypeptide or peptide thereby created is intended for use in immunological forms. The following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); proline (-0.5 ± 1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent polypeptide. In such changes, the substitution of amino acids whose hydrophilicity values are within ± 2 is preferred, those within ± 1 are particularly preferred, and those within ± 0.5 are even more particularly preferred.

As outlined above, amino acid substitutions are generally based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include (original residue: exemplary substitution): (Ala: Gly, Ser), (Arg: Lys), (Asn: Gln, His), (Asp: Glu, Cys, Ser), (Gln: Asn), (Glu: Asp), (Gly: Ala), (His: Asn, Gln), (Ile: Leu, Val), (Leu: Ile, Val), (Lys: Arg), (Met: Leu, Tyr), (Ser: Thr), (Thr: Ser), (Tip: Tyr), (Tyr: Trp, Phe), and (Val: Ile, Leu). Forms of this disclosure thus contemplate functional or biological equivalents of a polypeptide as set forth above. In particular, forms of the polypeptides can include variants having about 50%, 60%, 70%, 80%, 90%, and 95% sequence identity to the polypeptide of interest.

"Identity," as known in the art, is a relationship between two or more polypeptide sequences, as determined by comparing the sequences. In the art, "identity" also means the degree of sequence relatedness between polypeptide as determined by the match between strings of such sequences. "Identity" can also mean the degree of sequence relatedness of a polypeptide compared to the full-length of a reference polypeptide. "Identity" and "similarity" can be readily calculated by known methods, including, but not limited to, those described in (Computational Molecular Biology, Lesk, A. M., Ed., Oxford University Press, New York, 1988; Biocomputing: Informatics and Genome Projects, Smith, D. W., Ed., Academic Press, New York, 1993; Computer Analysis of Sequence Data, Part I, Griffin, A. M., and Griffin, H. G., Eds., Humana Press, New Jersey, 1994; Sequence Analysis in Molecular Biology, von Heinje, G., Academic Press, 1987; and Sequence Analysis Primer, Gribskov, M. and Devereux, J., Eds., M Stockton Press, New York, 1991; and Carillo, H., and Lipman, D., SIAM J Applied Math., 48: 1073 (1988).

Preferred methods to determine identity are designed to give the largest match between the sequences tested. Methods to determine identity and similarity are codified in publicly available computer programs. The percent identity between two sequences can be determined by using analysis software (*e.g*., Sequence Analysis Software Package of the Genetics Computer Group, Madison Wis.) that incorporates the Needelman and Wunsch, (J. Mol. Biol., 48: 443-453, 1970) algorithm (*e.g*., NBLAST, and XBLAST). The default parameters are used to determine the identity for the polypeptides of the present disclosure.

By way of example, a polypeptide sequence may be identical to the reference sequence, that is be 100% identical, or it may include up to a certain integer number of amino acid alterations as compared to the reference sequence such that the % identity is less than 100%. Such alterations are selected from: at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the reference polypeptide by the numerical percent of the respective percent identity (divided by 100) and then subtracting that product from said total number of amino acids in the reference polypeptide.

As used herein, the term "low stringency" refers to conditions that permit a polynucleotide or polypeptide to bind to another substance with little or no sequence specificity.

As used herein, the term "purified" and like terms relate to the isolation of a molecule or compound in a form that is substantially free (at least 60% free, preferably 75% free, and most preferably 90% free) from other components normally associated with the molecule or compound in a native environment.

As used herein, the term "pharmaceutically acceptable carrier" encompasses any of the standard pharmaceutical carriers, such as a phosphate buffered saline solution, water and emulsions such as an oil/water or water/oil emulsion, and various types of wetting agents.

"Operably linked" refers to a juxtaposition wherein the components are configured so as to perform their usual function. For example, control sequences or promoters operably linked to a coding sequence are capable of effecting the expression of the coding sequence, and an organelle localization sequence operably linked to protein will assist the linked protein to be localized at the specific organelle.

"Localization Signal or Sequence or Domain" or "Targeting Signal or Sequence or Domain" are used interchangeably and refer to a signal that directs a molecule to a specific cell, tissue, organelle, intracellular region or cell state. The signal can be polynucleotide, polypeptide, or carbohydrate moiety or can be an organic or inorganic compound sufficient to direct an attached molecule to a desired location. Exemplary targeting signals include mitochondrial localization signals from the precursor proteins list in U.S. Patent No. 8,039,587, and cell targeting signals known in the art such as those in Wagner et al., Adv. Gen., 53:333-354 (2005). It will be appreciated that the entire sequence need not be included, and modifications including truncations of these sequences are within the scope of the disclosure provided the sequences operate to direct a linked molecule to a specific cell type. Targeting signals of the present disclosure can have 80 to 100% sequence identity to the mitochondrial localize signal or cell targeting signal sequences. One class of suitable targeting signals include those that do not interact with the targeted cell in a receptor-ligand mechanism. For example, targeting signals include signals having or conferring a net charge, for example a positive charge. Positively charged signals can be used to target negatively charged cell types such as neurons and muscle. Negatively charged signals can be used to target positively charged cells.

"Cell surface marker" refers to any molecule such as moiety, peptide, protein, carbohydrate, nucleic acid, antibody, antigen, and/or metabolite presented on the surface or in the vicinity of a cell sufficient to identify the cell as unique in either type or state.

"Small molecule," as used herein, refers to molecules with a molecular weight of less than about 2000 g/mol, more preferably less than about 1500 g/mol, most preferably less than about 1200 g/mol.

"Nanoparticle", as used herein, generally refers to a particle having a diameter from about 1 nm up to, but not including, about 1 micron, preferably from 100 nm to about 1 micron. The particles can have any shape. Nanoparticles having a spherical shape can be referred to as "nanospheres".

"Mean particle size" as used herein, generally refers to the statistical mean particle size (diameter) of the particles in a population of particles. The diameter of an essentially spherical particle may refer to the physical or hydrodynamic diameter. The diameter of a non-spherical particle may refer preferentially to the hydrodynamic diameter. As used herein, the diameter of a non-spherical particle may refer to the largest linear distance between two points on the surface of the particle. Mean particle size can be measured using methods known in the art, such as dynamic light scattering.

"Monodisperse" and "homogeneous size distribution", are used interchangeably herein and describe a population of nanoparticles or microparticles where all of the particles are the same or nearly the same size. As used herein, a monodisperse distribution refers to particle distributions in which 90% of the distribution lies within 15% of the median particle size, more preferably within 10% of the median particle size, most preferably within 5% of the median particle size.

As used herein, the term "carrier" or "excipient" refers to an organic or inorganic ingredient, natural or synthetic inactive ingredient in a formulation, with which one or more active ingredients are combined.

### II. Compositions for Traversing the Blood-Brain-Barrier (BBB)

Compositions including polypeptides that traverse the blood-brain-barrier (BBB) have been developed. The ability of proteins that traverse to BBB to deliver associated agents to the brain and CNS provides strategies for treating diseases and disorders of the brain and CNS. Compositions of brain-penetrating peptides, and conjugates and methods of use thereof to traverse the BBB and deliver active agents across the BBB to treat symptoms of pathologies within the brain or CNS disclosed. Typically, the brain penetrating peptide conjugates include at least one BBB-traversing domain, in addition to one or more active agents, such as therapeutic, diagnostic or prophylactic agents, optionally including one or more linkers and/or one or more carriers.

### A. BBB-traversing Polypeptides

Polypeptides that traverse the blood-brain-barrier (BBB) are described. A BBB-traversing polypeptide is a polypeptide that passes from the circulation into the brain and/or CNS, in the absence of any additional molecular component.

The blood-brain barrier (BBB) is formed of brain endothelial cells (BECs), which form the lumen of the brain microvasculature (Abbott et al., Neurobiol Dis., 37:13-25 (2010)). The barrier function is achieved through tight junctions between endothelial cells that regulate the extravasation of molecules and cells into and out of the central nervous system (CNS). Modulation of receptor signaling at BECs is known to modulate BBB permeability and to facilitate the entry of molecules and cells into the CNS (Carman, et al., The Journal of Neuroscience, 31(37):13272-13280 (2011)). It has been established that receptors at the surface of BEC can bind and/or interact with the described peptides that traverse the BBB to initiate and complete traverse of the BBB-traversing peptides as well as associated cargo molecules from the circulation into the brain. In some forms the BBB-traversing peptides specifically bind to reelin at the surface of cells to initiate transport and/or entry across the BBB. In other forms, the BBB-traversing peptides specifically bind to Low-Density Lipoprotein Receptor-Related Protein 1 (LRP1) at the surface of cells to initiate transport and/or entry across the BBB.

Preferably, the polypeptides are non-toxic in the brain and do not cause damage or otherwise disrupt the integrity of the blood-brain-barrier, or any other tissue or structure in the brain. Therefore, preferably, the peptides traverse the intact functional BBB (*i.e*., "healthy BBB") via one or more mechanisms of active or passive transit. In some forms, the peptides traverse the damaged BBB to the same or different extent to the healthy BBB.

### 1. BBB-traversing Domains

BBB-traversing polypeptides include at least one BBB-traversing domain. The term "BBB-traversing domain" as used herein means a polypeptide that is capable of traversing the BBB of a subject *in vivo* in the absence of another molecule that targets, chaperones or otherwise mediates the passage of the peptide across the BBB. Typically, a BBB-traversing domain is or incudes a polypeptide having a sequence of between about 5 and about 25 contiguous amino acids including any one or more of SEQ ID NOs:1-20, 22-23.

In some forms, the BBB-traversing domain includes the amino acid sequence RRVISRAKLAAAL (SEQ ID NO:1), or a functional fragment, variant or derivative thereof. In other forms, the BBB-traversing domain includes the amino acid sequence CVGTNCY (SEQ ID NO:2) or a functional fragment, variant or derivative thereof. In other forms, the BBB-traversing domain is or includes the amino acid sequence SRRVISRAKLAAALE (SEQ ID NO:3) or a functional fragment, variant or derivative thereof. In other forms, the BBB-traversing domain is or includes the amino acid sequence MLGDPILASRRVISRAKLAAALE (SEQ ID NO:4) or a functional fragment, variant or derivative thereof. In other forms, the BBB-traversing domain is or includes the amino acid sequence MLGDPNSGGSRRVISRAKLAAAL (SEQ ID NO:5) or a functional fragment, variant or derivative thereof. In other forms, the BBB-traversing domain is or includes the amino acid sequence MLGDPNSGGSRRVISRAKLAAALE (SEQ ID NO:6) or a functional fragment, variant or derivative thereof. In other forms, the BBB-traversing domain is or includes the amino acid sequence MLGDPNSGGSRVISRAKLAAALE (SEQ ID NO:7) or a functional fragment, variant or derivative thereof. In other forms, the BBB-traversing domain is or includes the amino acid sequence ILASRRVISRAKLAAALE (SEQ ID NO:8) or a functional fragment, variant or derivative thereof. In other forms, the BBB-traversing domain is or includes the amino acid sequence SRRVISRAKLAAAL (SEQ ID NO:9) or a functional fragment, variant or derivative thereof. In other forms, the BBB-traversing domain is or includes the amino acid sequence GGSPILASRRVISRAKLAAALE (SEQ ID NO:10) or a functional fragment, variant or derivative thereof.

In other forms, the BBB-traversing domain is or includes the amino acid sequence MLGDPNLASRRRVISRAKLAAALE (SEQ ID NO:35) or a functional fragment, variant or derivative thereof. In other forms, the BBB-traversing domain is or includes the amino acid sequence MLGDPILASRRVISRAKLAAAL (SEQ ID NO:36) or a functional fragment, variant or derivative thereof. In other forms, the BBB-traversing domain is or includes the amino acid sequence MLGDPNFAIRRVISRAKLAAALE (SEQ ID NO:37) or a functional fragment, variant or derivative thereof. In other forms, the BBB-traversing domain is or includes the amino acid sequence MLGDPNSSSVDKLAAALE (SEQ ID NO:38) or a functional fragment, variant, or derivative thereof. In other forms, the BBB-traversing domain is or includes the amino acid sequence MLGDPILAGRRIVSRAKLAAALE (SEQ ID NO:39) or a functional fragment, variant, or derivative thereof. In other forms, the BBB-traversing domain is or includes the amino acid sequence NFAIRRVISRAKLAAALE (SEQ ID NO:62) or a functional fragment, variant, or derivative thereof. In other forms, the BBB-traversing domain is or includes the amino acid sequence MLGDPNSGGSPILASRRVISRAKLAAALE (SEQ ID NO:63) or a functional fragment, variant, or derivative thereof. In other forms, the BBB-traversing domain is or includes the amino acid sequence PILASRRVISRAKLAAALE (SEQ ID NO:64) or a functional fragment, variant, or derivative thereof. In other forms, the BBB-traversing domain is or includes the amino acid sequence MLGDPNSGGSPILASRRVISRAKLAAA (SEQ ID NO:65) or a functional fragment, variant, or derivative thereof. In other forms, the BBB-traversing domain is or includes the amino acid sequence SRVISRAKLAAALE (SEQ ID NO:66) or a functional fragment, variant, or derivative thereof. In other forms, the BBB-traversing domain is or includes the amino acid sequence MLGDPNLASRRVISRAKLAAALE (SEQ ID NO:67).

In other forms, the BBB-traversing domain is or includes the amino acid sequence CEGSLCY (SEQ ID NO:11) or a functional fragment, variant or derivative thereof. In other forms, the BBB-traversing domain is or includes the amino acid sequence CTGSLCY (SEQ ID NO:12) or a functional fragment, variant or derivative thereof. In other forms, the BBB-traversing domain is or includes the amino acid sequence CAGSMCY (SEQ ID NO:13) or a functional fragment, variant or derivative thereof. In other forms, the BBB-traversing domain is or includes the amino acid sequence CVGTLCY (SEQ ID NO:14) or a functional fragment, variant or derivative thereof. In other forms, the BBB-traversing domain is or includes the amino acid sequence CKGSNCY (SEQ ID NO:15) or a functional fragment, variant or derivative thereof. In other forms, the BBB-traversing domain is or includes the amino acid sequence CVGQLCY (SEQ ID NO:16) or a functional fragment, variant or derivative thereof. In other forms, the BBB-traversing domain is or includes the amino acid sequence CAGPLCY (SEQ ID NO:17) or a functional fragment, variant or derivative thereof. In other forms, the BBB-traversing domain is or includes the amino acid sequence CQGALCY (SEQ ID NO:18) or a functional fragment, variant or derivative thereof. In other forms, the BBB-traversing domain includes the amino acid sequence CVGANCY (SEQ ID NO:19) or a functional fragment, variant or derivative thereof. In other forms, the BBB-traversing domain includes the amino acid sequence CQGSNCY (SEQ ID NO:20) or a functional fragment, variant or derivative thereof. In other forms, the BBB-traversing domain is or includes a functional fragment, variant or derivative of a polypeptide having the amino acid sequence CAGALCY (SEQ ID NO:21). In other forms, the BBB-traversing domain is or includes the amino acid sequence CTGSMCY (SEQ ID NO:22) or a functional fragment, variant or derivative thereof. In other forms, the BBB-traversing domain is or includes the amino acid sequence CMGDLCY (SEQ ID NO:23) or a functional fragment, variant or derivative thereof.

Typically, the BBB-traversing domain or functional fragment thereof is between about 5 amino acids and about 25 amino acids, inclusive of any one of SEQ ID NOs:1-20 or 22-23 or a homologue such as an orthologue or paralogue thereof; or any combination thereof, or any subrange thereof, or any specific integer number of amino acids therebetween, including, but not limited to 6, 7, 8, 9, 10, 11, 12, 13, 14, 15, 16, 17, 18, 19, 20, 21, 22, 23, 24, or 25 amino acids. Variants can have, for example, at least 65%, 70%, 75%, 80%, 85%, 90%, 95%, 99% or 100% sequence identity to SEQ ID NOs:1-20 or 22-23, or a functional fragment thereof; or the corresponding sequence of a homologue such as an orthologue or paralogue of any of the foregoing sequences; or any combination thereof. In a particular form, a variant BBB traversing domain has at least 75%, 80%, 85%, 90%, 95%, 99% or 100% sequence identity to SEQ ID NO:1. In a particular form, a variant BBB-traversing domain has at least 75%, 80%, 85%, 90%, 95%, 99% or 100% sequence identity to SEQ ID NO:2. A BBB-traversing polypeptide variant is considered to be "functional" if it maintains the ability to cross the BBB in a subject, such as a healthy BBB in a subject, without toxicity to the subject.

In some forms, the BBB-traversing peptides specifically bind to Low-Density Lipoprotein Receptor-Related Protein 1 (LRP1) at the surface of cells to initiate transport and/or entry across the BBB. It may be that brain specificity of the BBB-traversing peptides is due to relative overexpression of LRP-1 in the brain microvascular endothelial cells or may involve another receptor acting in synergy with LRP1. Therefore, in some forms, a "functional" BBB-traversing peptide is one that binds to the LRP1 receptor in a manner sufficient for transport of the peptide across the BBB. For example, in some forms, the BBB-traversing domain is or incudes a polypeptide having a sequence of between about 5 and about 25 contiguous amino acids including any one or more of SEQ ID NOs:1, 3-10 selectively binds the Low-Density Lipoprotein Receptor-Related Protein 1 (LRP1) at the surface of cells to initiate transport and/or entry across the BBB.

In other forms, the BBB-traversing peptides specifically bind to reelin to initiate transport and/or entry across the BBB. Reelin itself binds to cell surface receptors, including apolipoprotein E receptor 2 (ApoER2) and very-low-density lipoprotein receptor (VLDLR), which are mainly expressed on the cell membrane of cortical neurons. Therefore, in some forms, a "functional" BBB-traversing peptide is one that binds to reelin in a manner that maintains the native interaction of reelin with VLDLR and/or ApoER2 sufficient for transport of the peptide across the BBB. For example, in some forms, the BBB-traversing domain is or incudes a polypeptide having a sequence of between about 5 and about 25 contiguous amino acids including any one or more of SEQ ID NOs 2, 11-20, 22-23 selectively binds reelin and enables reelin receptor-mediated transport across the surface of cells to initiate transport and/or entry across the BBB.

In some forms, the BBB-traversing peptides are identified using constrained library screening, for example, identified based on accumulation in the brain. An exemplary constrained library screen is demonstrated in Example 1, using Peptides from constrained library XRRXIXRAXLAXXX (where X is a random amino acid; SEQ ID NO:76; based on the Cerepep peptide), ranked by high throughput DNA sequencing-based representation in the brain (ranked based on Round2 to Round 1 ratio).

In some forms, the BBB-traversing peptide is or includes the amino acid sequence of one or more of NRRVIDRAALASL (SEQ ID NO:77); SRRVISRAGLADNL (SEQ ID NO:78); PRRVIGRAGLASTA (SEQ ID NO:79); GRRVIGRASLAPDS (SEQ ID NO:80); GRRIIERAALALED (SEQ ID NO:81); GRRPISRANLANTD (SEQ ID NO:82); PRRIISRAQLAQTS (SEQ ID NO:83); GRRVISRAGLASDD (SEQ ID NO:84); ARRIINRAILASDP (SEQ ID NO:85); PRRIITRATLAPPV (SEQ ID NO:86); TRRVIDRAGLANEK (SEQ ID NO:87); ARRVISRAGLAQDT (SEQ ID NO:88); ARRIIPRAPLADR (SEQ ID NO:89); ARRVISRAELARNG (SEQ ID NO:90); ARRTISRAALAQ (SEQ ID NO:91); QRRVIPRAKLALEE (SEQ ID NO: 92); ARRVISRANLANPT (SEQ ID NO:93); NRRVIPRAGLAENQ (SEQ ID NO: 94); GRRVIGRASLAQDE (SEQ ID NO:95); and NRRVIPRAGLASND (SEQ ID NO:96), or a or a functional fragment, variant or derivative thereof of any one of SEQ ID NOs:77-96.

### 2. BBB-traversing Domain Fusion Peptides

Fusion proteins and polypeptides, including one or more additional polypeptide sequences fused to one or more BBB-traversing domain polypeptides are described.

The term "BBB-traversing domain" is used exclusively in the context of fusion peptides that include one or more BBB-traversing domain, to refer to the component of the fusion peptide that includes the BBB-traversing domain(s). In some forms, the BBB-traversing domain of a fusion peptide includes 5-25 amino acids of any one or more of SEQ ID NOs:1-20, 22-23. Therefore, in some forms, a BBB-traversing domain of a fusion peptide includes all of the amino acids of any one or more of SEQ ID NOs:1-20, 22-23. Therefore, in some forms, the BBB-traversing domain fusion peptide includes 5-25 amino acids of any one or more of SEQ ID NOs:1-20, 22-23, contiguous with one or more additional heterologous polypeptide sequences.

The term "additional heterologous polypeptide sequences" refers to a heterologous sequence that is directly or indirectly fused to a BBB-traversing domain. Typically, an additional heterologous polypeptide sequences does not traverse the BBB in the absence of BBB-traversing domain. Therefore, the additional heterologous polypeptide sequence of a BBB-traversing domain fusion peptide does not include 5-25 amino acids of any one or more of SEQ ID NOs:1-20, 22-23. The additional heterologous polypeptide sequences are typically between two and two thousand contiguous amino acids in length, such as 5, 10, 20, 50, 100, 150, 250, 500, 600, 750, 1000 or 1,500 amino acids, or more than 1,500 amino acids.

In some forms, a BBB-traversing domain fusion protein includes one or more additional heterologous polypeptide sequence fused to the amino (N) or carboxyl (C) terminus of the BBB-traversing domain. Exemplary schematics for the domain structure of a fusion protein include:
N-[BBB-traversing domain(s)]-[heterologous polypeptide]-C; or
N-[heterologous polypeptide]-[BBB-traversing domain(s)]-C; or
N-[ heterologous polypeptide]-[BBB-traversing domain(s)]-[ heterologous polypeptide]-C, where "N" and "C" refer to the ammino (NH2) and Carboxyl (COOH) termini, respectively.

The number of functional domains and BBB-traversing domain(s) can vary according to the requirements of the fusion peptide. For example, the domain structure of a fusion protein can include:
N-[BBB-traversing domain(s)]X-[heterologous polypeptide]Y-C; or
N-[heterologous polypeptide]X-[BBB-traversing domain(s)]Y-C; or
N-[ heterologous polypeptide]X-[BBB-traversing domain(s)]Y-[ heterologous polypeptide]Z-C, where "N" and "C" refer to the ammino (NH2) and Carboxyl (COOH) termini, respectively, and where X, Y and Z are independently integers of between 1 and 10, respectively.

In exemplary forms, X and Y and Z are, independently, 1, 2 or 3.

Exemplary heterologous polypeptides include any known proteins or fragments thereof, such as enzymes, immunoglobulins, cell surface receptors, viral capsids, immune receptors, membrane proteins, *etc*. In some forms the heterologous polypeptide is or forms an active agent, such as a therapeutic agent, diagnostic agent or prophylactic agent. Therefore, in some forms, the heterologous polypeptide of a BBB-traversing domain is a cargo molecule.

### B. BBB-Traversing Polypeptide Conjugates

Conjugates of BBB-Traversing Polypeptides in complex or conjugated with one or more additional molecules are described.

Typically, the conjugates include one or more additional molecules that is a cargo molecule. Cargo molecules include active agents for delivery across the BBB. For example, in some forms one or more cargo molecules is a therapeutic, diagnostic or prophylactic molecule for treating or diagnosing one or more diseases or disorders in the brain or CNS of a subject. Typically, the one or more agents are covalently attached to one or more groups on the BBB-traversing polypeptide. In some forms, the BBB-traversing polypeptide conjugates include one or more therapeutic, prophylactic or diagnostic agents conjugated or complexed with the BBB-traversing polypeptide via one or more linking moieties. In further forms, the linking moieties incorporate or are conjugated with one or more spacer moieties. The linking and/or spacer moieties can be cleavable, for example, by exposure to the intracellular compartments of target cells in vivo. The therapeutic, prophylactic or diagnostic agent and/or targeting moiety can be either covalently attached or intra-molecularly dispersed or encapsulated.

### 1. Cargo Molecules

Conjugates of BBB-Traversing Polypeptides in complex or conjugated with one or more cargo molecules for delivery to the brain or CNS across the BBB. Cargo molecules typically include one or more therapeutic, prophylactic, or diagnostic active agents. Typically, the cargo molecules do not cross the BBB in the absence of the BBB-Traversing Polypeptide.

Exemplary cargo molecules are tethered to, complexed with, or otherwise conjugated to the BBB traversing polypeptide. In some forms, two, three, four, or more cargo molecules are attached to and/or conjugated with the BBB traversing polypeptide. Two or more different cargo molecules can be loaded into, attached to the surface of, or conjugated with the same BBB traversing polypeptide, or different BBB traversing polypeptides. For example, in some forms, the compositions include two or more different types of BBB traversing polypeptide conjugates having the same or different cargo molecule(s) associated therewith. In some forms, additional cargo molecules are co-administered to the subject but are not loaded into, attached to the surface of, and/or enclosed within the disclosed BBB traversing polypeptide conjugates, and can be, for example, free or soluble, or in a different carrier or dosage form. For example, such cargo molecules can be free or soluble active agent(s), or active agent(s) in a different carrier or dosage form but are nonetheless part of the same pharmaceutical composition as the nanocarrier composition.

The compositions disclosed herein typically include one or more therapeutic, prophylactic, or diagnostic active agents loaded onto, attached to the surface of, complexed and/or conjugated with BBB traversing polypeptide conjugates. In some forms, two, three, four, or more active agents are loaded onto, attached to the surface of, complexed and/or conjugated with BBB traversing polypeptide conjugates. The two or more agents can be loaded onto, attached to the surface of, complexed and/or conjugated with BBB traversing polypeptide conjugates. In some forms, compositions include two or more different types of BBB traversing polypeptide conjugates having the same or different active agent(s) associated therewith. In some forms, additional active agents are co-administered to the subject but are not loaded into, attached to the surface of, and/or enclosed within the disclosed BBB traversing polypeptide conjugates, and can be, for example, free or soluble, or in a different carrier or dosage form. For example, such active agents can be free or soluble active agent(s), or active agent(s) in a different carrier or dosage form but are nonetheless part of the same pharmaceutical composition as the BBB traversing polypeptide conjugates composition.

The active agents can be small molecule active agents or biomacromolecules, such as proteins, polypeptides, or nucleic acids. In some forms, the nucleic acid is an expression vector encoding a protein or a functional nucleic acid. In some forms, vectors are suitable for integration into a cells genome or expressed extra-chromosomally. In other forms, the nucleic acid is a functional nucleic acid. Suitable small molecule active agents include organic and organometallic compounds. The small molecule active agents can be hydrophilic, hydrophobic, or amphiphilic compounds. The active agent can be a therapeutic, nutritional, diagnostic, or prophylactic agent.

Exemplary active agents include, but are not limited to, chemotherapeutic agents, neurological agents, tumor antigens, CD4+ T-cell epitopes, cytokines, imaging agents, radionuclides, small molecule signal transduction inhibitors, photothermal antennas, immunologic danger signaling molecules, other immuno-therapeutics, enzymes, antibiotics, antivirals, anti-parasites (helminths, protozoans), growth factors, growth inhibitors, hormones, hormone antagonists, antibodies and bioactive fragments thereof (including humanized, single chain, and chimeric antibodies), antigen and vaccine formulations (including adjuvants), peptide drugs, anti-inflammatories, immunomodulators (including ligands that bind to Toll-Like Receptors (including but not limited to CpG oligonucleotides) to activate the innate immune system, molecules that mobilize and optimize the adaptive immune system, molecules that activate or up-regulate the action of cytotoxic T lymphocytes, natural killer cells and helper T-cells, and molecules that deactivate or down-regulate suppressor or regulatory T-cells), agents that promote uptake of the nanocarrier into cells (including dendritic cells and other antigen-presenting cells), nutraceuticals such as vitamins, oligonucleotide drugs (including DNA, RNAs, antisense, aptamers, small interfering RNAs, ribozymes, external guide sequences for ribonuclease P, and triplex forming agents) and other gene modifying agents such as ribozymes, CRISPR/Cas, zinc finger nuclease, and transcription activator-like effector nucleases (TALEN).

Exemplary diagnostic agents include paramagnetic molecules, fluorescent compounds, magnetic molecules, and radionuclides, x-ray imaging agents, and contrast agents.

The optimal drug loading will necessarily depend on many factors, including the choice of drug, BBB traversing polypeptide structure and size, and tissues to be treated. In some forms, the one or more therapeutic, prophylactic, or diagnostic agents are encapsulated, associated, and/or conjugated to the BBB traversing polypeptide at a concentration between about 50% and about 95%, inclusive; preferably between about 50% and about 80%, inclusive; between about 40% and about 70%, inclusive; between about 50% and about 80%, inclusive; between about 1% and about 20%, inclusive; between about 1% and about 5%, inclusive; between about 3% and about 20% by weight, inclusive; and between about 3% and about 10% by weight, inclusive. However, optimal drug loading for any given drug, BBB traversing polypeptide, and site of target can be identified by routine methods, such as those described.

### a. Therapeutic Agents

In certain forms, the cargo molecule(s) include one or more therapeutic agents. Exemplary therapeutic agents that can be cargo molecules conjugated to or complexed with the BBB traversing peptides include anticancer molecular agents, such as monoclonal antibody (mAb)Herceptin, neuroactive agents, such as neuroprotective growth factors and interfering peptides, and immune modulators, such as anti-inflammatories.

In some forms, the therapeutic agent is a monoclonal antibody specific for one or more molecular targets associated with a disease or disorder, or an antigen-binding fragment of a mAb. In other forms, the therapeutic agent is a small molecule. In other forms, the agent is a designer interference peptide (iPep). Ipeps are natural or synthetic pharmacological agents created to block selective interactions between protein partners that are difficult to target with conventional small molecule chemicals or with large biologicals. Protein-protein interactions (PPIs) are promising therapeutic targets and, in some instances, interfering peptides (*i.e*., natural or synthetic peptides capable of interfering with PPIs) are better suited than small molecules to interfere with the large surfaces implicated in PPIs. In some forms, the therapeutic agent is a neurotrophic factors, such as brain-derived neurotrophic factor (BDNF), ciliary neurotrophic factor (CNTF), glial cell-line derived neurotrophic factor (GDNF) and nerve growth factor (NGF).

Supramolecular agents, such as anticancer agents, neuroactive agents, and immune modulators loaded in biological and synthetic nano-assemblies are also described. Vectors and cells including the conjugates are also described. Exemplary cells include CAR-T cells.

### (a) Chemotherapeutic Agents

In certain forms, the cargo molecule(s) include one or more anti-cancer agents, such as cytotoxic agents (*e.g*. potent cytotoxic drug monomethyl auristatin A, paclitaxel), signaling pathway modulators, antibodies and their fragments (*e.g*. HER2 and immune checkpoint inhibitor antibodies),.proteins/protein fragments/peptides (such as interfering peptides, proapoptotic peptides, secondary targeting peptides for targets behind the BBB, cytokines and interleukins), polysaccharides, photosenzitizers (*e.g*. verteporfin), haptens to be used as targets for immunotherapy (FAM/FITC), nucleic acids (RNA and DNA), and therapeutic radioisotopes.

Representative anti-cancer agents include, but are not limited to, alkylating agents (such as cisplatin, carboplatin, oxaliplatin, mechlorethamine, cyclophosphamide, chlorambucil, dacarbazine, lomustine, carmustine, procarbazine, chlorambucil and ifosfamide), antimetabolites (such as fluorouracil (5-FU), gemcitabine, methotrexate, cytosine arabinoside, fludarabine, and floxuridine), antimitotics (including taxanes such as paclitaxel and decetaxel and vinca alkaloids such as vincristine, vinblastine, vinorelbine, and vindesine), anthracyclines (including doxorubicin, daunorubicin, valrubicin, idarubicin, and epirubicin, as well as actinomycins such as actinomycin D), cytotoxic antibiotics (including mitomycin, plicamycin, and bleomycin), topoisomerase inhibitors (including camptothecins such as camptothecin, irinotecan, and topotecan as well as derivatives of epipodophyllotoxins such as amsacrine, etoposide, etoposide phosphate, and teniposide), antibodies to vascular endothelial growth factor (VEGF) such as bevacizumab (AVASTIN^{®}), other anti-VEGF compounds; thalidomide (THALOMID^{®}) and derivatives thereof such as lenalidomide (REVLIMID^{®}); endostatin; angiostatin; receptor tyrosine kinase (RTK) inhibitors such as sunitinib (SUTENT^{®}); tyrosine kinase inhibitors such as sorafenib (NEXAVAR^{®}), erlotinib (TARCEVA^{®}), pazopanib, axitinib, and lapatinib; transforming growth factor-α or transforming growth factor-β inhibitors, and antibodies to the epidermal growth factor receptor such as panitumumab (VECTIBIX^{®}) and cetuximab (ERBITUX^{®}).

In some forms, particularly those for treating cancer, one or more of the active agents can be a chemotherapeutic agent that has immune signaling properties. In some forms, the agent is the Herceptin monoclonal antibody (mAb). Herceptin is a mAb specifically designed to target HER2 receptors, which are transmembrane receptors on both normal cells and HER2+ tumor cells. HER2 plays an important role in the signaling network that drives cell proliferation.

### (b) Neuroactive Agents

In some form that active agent is a conventional treatment for neurodegeneration, or for increasing or enhancing neuroprotection. Exemplary neuroprotective agents are known in the art in include, for example, glutamate antagonists, antioxidants, and NMDA receptor stimulants. In some forms, cargo molecules for treatment of neurodegenerative disease include neurostimulatory growth factors (CDNF, GDNF), therapeutic antibodies, and interfering peptides, as well as neuropeptides, such as galanin and neurotensin. Other neuroprotective agents and treatments include caspase inhibitors, trophic factors, anti-protein aggregation agents, therapeutic hypothermia, and erythropoietin. Amantadine and anticholinergics are used for treating motor symptoms, clozapine for treating psychosis, cholinesterase inhibitors for treating dementia. Treatment strategies can also include administration of modafinil.

For subjects with Huntington's disease, dopamine blocker is used to help reduce abnormal behaviors and movements, and drugs such as amantadine and tetrabenazine are used to control movement, etc. Drugs that help to reduce chorea include neuroleptics and benzodiazepines. Compounds such as amantadine or remacemide have shown preliminary positive results. Hypokinesia and rigidity, especially in juvenile cases, can be treated with antiparkinsonian drugs, and myoclonic hyperkinesia can be treated with valproic acid. Psychiatric symptoms can be treated with medications similar to those used in the general population. Selective serotonin reuptake inhibitors and mirtazapine have been recommended for depression, while atypical antipsychotic drugs are recommended for psychosis and behavioral problems.

Treatments for Parkinson's disease, include, but are not limited to, levodopa (usually combined with a dopa decarboxylase inhibitor or COMT inhibitor), dopamine agonists, and MAO-B inhibitors.

The only compound yielding borderline significance with respect to survival time in subjects with ALS is riluzole (RILUTEK^{®}) (2-amino-6-(trifluoromethoxy) benzothiazole), an antiexcitotoxin. Other medications, most used off-label, and interventions can reduce symptoms due to ALS. Some treatments improve quality of life and a few appear to extend life. Common ALS-related therapies are reviewed in Gordon, Aging and Disease, 4(5):295-310 (2013), which is specifically incorporated by reference herein in its entirety. Exemplary ALS treatments and interventions are also discussed in Gordon, Aging and Disease, 4(5):295-310 (2013), listed in a table provided therein.

A number of other agents have been tested in one or more clinical trials with efficacies ranging from non-efficacious to promising. Exemplary agents are reviewed in Carlesi, et al., Archives Italiennes de Biologie, 149:151-167 (2011) and include, for example, agents that reduces excitotoxicity such as talampanel (8-methyl-7H-1,3-dioxolo(2,3)benzodiazepine), a cephalosporin such as ceftriaxone, or memantine; agents that reduce oxidative stress such as coenzyme Q10, manganoporphyrins, KNS-760704 [(6R)-4,5,6,7-tetrahydro-N6-propyl-2,6-benzothiazole-diamine dihydrochloride, RPPX], and edaravone (3-methyl-1-phenyl-2-pyrazolin-5-one, MCI-186); agents that reduces apoptosis such as histone deacetylase (HDAC) inhibitors including valproic acid, TCH346 (Dibenzo(b,f)oxepin-10-ylmethyl-methylprop-2-ynylamine), minocycline, or tauroursodeoxycholic Acid (TUDCA); agents that reduce neuroinflammation such as thalidomide and celastol; neurotropic agents such as insulin-like growth factor 1 (IGF-1) and vascular endothelial growth factor (VEGF); heat shock protein inducers such as arimoclomol; or an autophagy inducer such as rapamycin or lithium.

Exemplary neurological drugs include, but are not limited to, ABSTRAL^{®} (fentanyl), AGGRENOX^{®} (aspirin/extended-release dipyridamole), AMERGE^{®} (naratriptan), AMPYRA^{®} (dalfampridine), AMRIX^{®} (cyclobenzaprine hydrochloride extended release), ANEXSIA^{®}, APOKYN^{®} (apomorphine hydrochloride), APTIOM^{®} (eslicarbazepine acetate), ARICEPT^{®} (donepezil hydrochloride), aspirin, AVINZA^{®} (morphine sulfate), AVONEX^{®} (Interferon Beta 1-A), AXERT^{®} (almotriptan malate), AXONA^{®} (caprylidene), BANZEL^{®} (rufinamide), BELSOMRA^{®} (suvorexant), BOTOX^{®} (onabotulinumtoxinA), BROMDAY^{®} (bromfenac), BUTRANS^{®} (buprenorphine), CAMBIA^{®} (diclofenac potassium), CARBAGLU^{®} (carglumic acid), CARBATROL^{®} (Carbamazepine), CENESTIN^{®} (synthetic conjugated estrogens, A), CIALIS^{®} (tadalafil), KLONOPIN^{®} (clonazepam), COMTAN^{®} (Entacapone), COPAXONE^{®} (glatiramer acetate), CUVPOSA^{®} (glycopyrrolate), CYLERT^{®}, DEPAKOTE^{®} (divalproex sodium), DEPAKOTE^{®} (divalproex sodium), DEPAKOTE ER^{®} (divalproex sodium), DUOPA^{®} (carbidopa and levodopa), DUREZOL^{®} (difluprednate), DYLOJECT^{®} (diclofenac sodium), EDLUAR^{®} (zolpidem tartrate), ELIQUIS^{®} (apixaban), EMBEDA^{®} (morphine sulfate and naltrexone hydrochloride), EXALGO^{®} (hydromorphone hydrochloride), EXELON^{®} (rivastigmine tartrate), EXELON^{®} (rivastigmine tartrate), EXPAREL^{®} (bupivacaine liposome injectable suspension), EXTAVIA^{®} (Interferon beta-l b), FETZIMA^{®} (levomilnacipran), FOCALIN^{®} (dexmethylphenidate HCl), FROVA^{®} (frovatriptan succinate), FYCOMPA^{®} (perampanel), GALZIN^{®} (zinc acetate), GRALISE^{®} (gabapentin), HETLIOZ^{®} (tasimelteon), HORIZANT^{®} (gabapentin enacarbil), HORIZANT^{®} (gabapentin enacarbil), IMITREX^{®} (sumatriptan), IMITREX^{®} (sumatriptan), INTERMEZZO^{®} (zolpidem tartrate sublingual tablet), INTUNIV^{®} (guanfacine extended-release), INVEGA^{®} (paliperidone), NUMBY^{®} (iontocaine), KADIAN^{®} (Morphine Sulfate), KAPVAY^{®} (clonidine hydrochloride), LEVETIRACTAM^{®} (keppra), LAMICTAL^{®} (lamotrigine), LAZANDA^{®} (fentanyl citrate), LEMTRADA^{®} (alemtuzumab), LEVITRA^{®} (vardenafil), LUNESTA^{®} (eszopiclone), LUPRON DEPOT^{®} (leuprolide acetate), LUSEDRA^{®} (fospropofol disodium), LYRICA^{®} (pregabalin), MAXALT^{®} (rizatriptan benzoate), MERREM I.V.^{®} (meropenem), METADATE CD^{®} (methylphenidate HCl), MIGRANAL^{®} (dihydroergotamine), MIRAPEX^{®} (pramipexole), MOVANTIK^{®} (naloxegol), MYOBLOC^{®} (rimabotulinumtoxinB), REVIA^{®} (naltrexone hydrochloride), NAMENDA^{®} (memantine HCl), NAMZARIC^{®} (memantine hydrochloride extended-release + donepezil hydrochloride), NEUPRO^{®} (Rotigotine Transdermal System), NEUPRO^{®} (rotigotine), NEURONTIN^{®} (gabapentin), NORCO^{®} (Hydrocodone Bitartrate/Acetaminophen 10 mg/325 mg), NORTHERA^{®} (droxidopa), NOVANTRONE^{®} (mitoxantrone hydrochloride), NUCYNTA^{®} (tapentadol), NUEDEXTA^{®} (dextromethorphan hydrobromide and quinidine sulfate), NUVIGIL^{®} (armodafinil), NYMALIZE^{®} (nimodipine), ONFI^{®} (clobazam), ONSOLIS^{®} (fentanyl buccal), OXECTA^{®} (oxycodone HCl), OXTELLAR XR^{®} (oxcarbazepine extended release), OXYCONTIN^{®} (oxycodone), PERCODAN^{®} (oxycodone/aspirin), PERCOCET^{®} (oxycodone with acetaminophen), PLEGRIDY^{®} (peginterferon beta-1a), POSICOR^{®} (mibefradil), POTIGA^{®} (ezogabine), QUADRAMET^{®} (samarium lexidronam), QUDEXY XR^{®} (topiramate), QUILLIVANT XR^{®} (methylphenidate hydrochloride), QUTENZA^{®} (capsaicin), REBIF^{®} (interferon beta-1a), REDUX^{®} (dexfenfluramine hydrochloride), RELPAX^{®} (eletriptan hydrobromide), REMINYL^{®} (galantamine hydrobromide), REQUIP^{®} (ropinirole hydrochloride), RILUTEK^{®} (riluzole), ROZEREM^{®} (ramelteon), RYTARY^{®} (carbidopa and levodopa), SABRIL^{®} (vigabatrin), ZELAPAR^{®} (selegiline), SILENOR^{®} (doxepin), SONATA^{®} (zaleplon), SPRIX^{®} (ketorolac tromethamine), STAVZOR^{®} (valproic acid delayed release), STRATTERA^{®} (atomoxetine HCl), SUBSYS^{®} (fentanyl sublingual spray), TARGINIQ ER^{®} (oxycodone hydrochloride + naloxone hydrochloride), TASMAR^{®} (tolcapone), TEGRETOL^{®} (carbamazepine), TIVORBEX^{®} (indomethacin), TOPAMAX^{®} (topiramate), TRILEPTAL^{®} (oxcarbazepine), TROKENDI XR^{®} (topiramate), TYSABRI^{®} (natalizumab), ULTRACET^{®} (acetaminophen and tramadol HCl), ULTRAJECT VERSED^{®} (midazolam HCI), VIIBRYD^{®} (vilazodone hydrochloride), VIMPAT^{®} (lacosamide), VISIPAQUE^{®} (iodixanol), VIVITROL^{®} (naltrexone), VPRIV^{®} (velaglucerase alfa), VYVANSE^{®} (Lisdexamfetamine Dimesylate), XARTEMIS XR^{®} (oxycodone hydrochloride and acetaminophen), XENAZINE^{®} (tetrabenazine), XIFAXAN^{®} (rifaximin), XYREM^{®} (sodium oxybate), ZANAFLEX^{®} (tizanidine hydrochloride), ZINGO^{®} (lidocaine hydrochloride monohydrate), ZIPSOR^{®} (diclofenac potassium), ZOHYDRO ER^{®} (hydrocodone bitartrate), ZOMIG^{®} (zolmitriptan), ZONEGRAN^{®} (zonisamide), ZUBSOLV^{®} (buprenorphine and naloxone).

Treatment for Dementia with Lewy Bodies can include, for example, acetylcholinesterase inhibitors such as tacrine, rivastigmine, galantamine or donepezil; the N-methyl d-aspartate receptor antagonist memantine; dopaminergic therapy, for example, levodopa or selegiline; antipsychotics such as olanzapine or clozapine; REM disorder therapies such as clonazepam, melatonin, or quetiapine; anti-depression and antianxiety therapies such as selective serotonin reuptake inhibitors (citalopram, escitalopram, sertraline, paroxetine, *etc.*) or serotonin and noradrenaline reuptake inhibitors (venlafaxine, mirtazapine, and bupropion) (see, *e.g*., Macijauskiene, et al., Medicina (Kaunas), 48(1):1-8 (2012)).

In some forms, the therapeutic agent is a neuroprotective factor. Neuroprotective factors generally prevent neuronal cell death by intervening and inhibiting the pathogenetic process that causes neuronal dysfunction and death. Neuroleptics, antidepressants, sedatives/hypnotics, and anxiolytics are frequently prescribed for the treatment of neuropsychiatric diseases. Exemplary neuroprotective agents include, for example, glutamate antagonists, antioxidants, and NMDA receptor stimulants. Other neuroprotective agents and treatments include caspase inhibitors, trophic factors, anti-protein aggregation agents, therapeutic hypothermia, and erythropoietin. In some forms, the agent is a neuroprotective growth factor. Exemplary neuroprotective growth factors include brain-derived neurotrophic factor (BDNF), ciliary neurotrophic factor (CNTF), glial cell-line derived neurotrophic factor (GDNF) and nerve growth factor (NGF).

Other common therapeutic, prophylactic or diagnostic agents for treating neurological dysfunction include amantadine and anticholinergics for treating motor symptoms, clozapine for treating psychosis, cholinesterase inhibitors for treating dementia, and modafinil for treating daytime sleepiness.

### (c) Immune Modulators

The active agent can be an immunomodulator such as an immune response stimulating agent or an agent that blocks immunosuppression. In particularly preferred forms, the active agents target tumor checkpoint blockade or costimulatory molecules.

The immune system is composed of cellular (T-cell driven) and humoral (B-cell driven) elements. It is generally accepted that for cancer, triggering of a powerful cell-mediated immune response is more effective than activation of humoral immunity. Cell-based immunity depends upon the interaction and co-operation of a number of different immune cell types, including antigen-presenting cells (APC; of which dendritic cells are an important component), cytotoxic T cells, natural killer cells and T-helper cells. Therefore, the active agent can be an agent that increases a cell (T-cell driven) immune response, a humoral (B-cell driven) immune response, or a combination thereof. For example, in some forms, the agent enhances a T cell response, increases T cell activity, increases T cell proliferation, reduces a T cell inhibitory signal, enhances production of cytokines, stimulates T cell differentiation or effector function, promotes survival of T cells or any combination thereof.

Exemplary immunomodulatory active agents include cytokines, xanthines, interleukins, interferons, oligodeoxynucleotides, glucans, growth factors (*e.g*., TNF, CSF, GM-CSF and G-CSF), hormones such as estrogens (diethylstilbestrol, estradiol), androgens (testosterone, HALOTESTIN^{®} (fluoxymesterone)), progestins (MEGACE^{®} (megestrol acetate), PROVERA^{®} (medroxyprogesterone acetate)), and corticosteroids (prednisone, dexamethasone, hydrocortisone).

In some forms the active agent is an inflammatory molecule such as a cytokine, metalloprotease or other molecule including, but not limited to, IL-1 β, TNF-α, TGF-beta, IFN-γ, IL- 17, IL-6, IL-23, IL-22, IL-21, and MMPs.

### (1) Cytokines

In a preferred form, at least one of the active agents is a proinflammatory cytokine. Cytokines typically act as hormonal regulators or signaling molecules at nano-to- picomolar concentrations and help in cell signaling. The cytokine can be a protein, peptide, or glycoprotein. Exemplary cytokines include, but are not limited to, interleukins (*e.g*., IL-1, IL-2, IL-4, IL-5, IL-6, IL-7, IL-12, IL-15, *etc.*), interferons (*e.g*., interferon-γ), macrophage colony stimulating factor, granulocyte colony stimulating factor, tumor necrosis factor, Leukocyte Inhibitory Factor (LIF), chemokines, SDF-1α, and the CXC family of cytokines.

### (2) Chemokines

In another form, at least one of the active agents is a proinflammatory chemokine. Chemokines are a family of small cytokines. Their name is derived from their ability to induce directed chemotaxis in nearby responsive cells. Therefore, they are chemotactic cytokines. Proteins are classified as chemokines according to shared structural characteristics such as small size (they are all approximately 8-10 kDa in size), and the presence of four cysteine residues in conserved locations that are key to forming their 3-dimensional shape. Chemokines have been classified into four main subfamilies: CXC, CC, CX3C and XC. Chemokines induce cell signaling by binding to G protein-linked transmembrane receptors (*i.e*., chemokine receptors).

### (d) Agents that Block Immune Suppression

At least one of the active agents can be an agent that blocks, inhibits or reduces immune suppression or that that blocks, inhibits or reduces the bioactivity of a factor that contributes to immune suppression. It has become increasingly clear that tumor-associated immune suppression not only contributes greatly to tumor progression but is also one of the major factors limiting the activity of cancer immunotherapy. Antigen-specific T-cell tolerance is one of the major mechanisms of tumor escape, and the antigen-specific nature of tumor non-responsiveness indicates that tumor-bearing hosts are not capable of maintaining tumor-specific immune responses while still responding to other immune stimuli (Willimsky, et al., Immunol. Rev., 220:102-12 (2007), Wang, et al. Semin Cancer Biol., 16:73-9 (2006), Frey, et al., Immunol. Rev., 222:192-205 (2008), Nagaraj, et al., Clinical Cancer Research, 16(6):1812-23 (2010)).

### b. Diagnostic Agents

In some cases, the active agents conjugated with BBB-traversing polypeptides are diagnostic agents. When the conjugates include diagnostic agents, the diagnostic agents can be tumor imaging agents alone or in combination with one or more therapeutic agents. Exemplary reported agents include fluorescent reporter dyes (including near infrared dyes), radio-reporters, and contrast agents for MRI (such as iron oxide nanoparticles).

Examples of diagnostic agents that can be delivered to the brain by BBB traversing polypeptide conjugates include paramagnetic molecules, fluorescent compounds, magnetic molecules, and radionuclides, x-ray imaging agents, and contrast media. BBB traversing polypeptide conjugates can include agents useful for determining the location of administered compositions. Agents useful for this purpose include fluorescent tags, radionuclides and contrast agents.

Exemplary diagnostic agents include dyes, fluorescent dyes, near infra-red dyes, SPECT imaging agents, PET imaging agents and radioisotopes. Representative dyes include carbocyanine, indocarbocyanine, oxacarbocyanine, thüicarbocyanine and merocyanine, polymethine, coumarin, rhodamine, xanthene, fluorescein, boron-dipyrromethane (BODIPY), Cy5, Cy5.5, Cy7, VivoTag-680, VivoTag-S680, VivoTag-S750, AlexaFluor660, AlexaFluor680, AlexaFluor700, AlexaFluor750, AlexaFluor790, Dy677, Dy676, Dy682, Dy752, Dy780, DyLight547, Dylight647, HiLyte Fluor 647, HiLyte Fluor 680, HiLyte Fluor 750, IRDye 800CW, IRDye 800RS, IRDye 700DX, ADS780WS, ADS830WS, and ADS832WS.

Exemplary SPECT or PET imaging agents include chelators such as di-ethylene tri-amine penta-acetic acid (DTPA), 1,4,7,10-tetra-azacyclododecane-1,4,7,10-tetraacetic acid (DOTA), di-amine dithiols, activated mercaptoacetyl-glycyl-glycyl-gylcine (MAG3), and hydrazidonicotinamide (HYNIC).

Exemplary isotopes include Tc-94m, Tc-99m, In-111, Ga-67, Ga-68, Gd3+, Y-86, Y-90, Lu-177, Re-186, Re-188, Cu-64, Cu-67, Co-55, Co-57, F-18, Sc-47, Ac-225, Bi-213, Bi-212, Pb-212, Sm-153, Ho-166, and Dy-166.

In some forms, the BBB traversing polypeptide conjugates include one or more radioisotopes suitable for positron emission tomography (PET) imaging. Exemplary positron-emitting radioisotopes include carbon-11 (11C), copper-64 (64Cu), nitrogen-13 (13N), oxygen-15 (15O), gallium-68 (68Ga), and fluorine-18 (18F), *e.g*., 2-deoxy-2-18F-fluoro-β-D-glucose (18F-FDG).

In further forms, a singular BBB-traversing polypeptide conjugate composition can simultaneously treat and/or diagnose a disease or a condition at one or more locations in the body.

### 2. Linkers and Coupling Agents

Conjugates of BBB-Traversing Polypeptides in complex or conjugated with one or more cargo molecules for delivery to the brain or CNS across the BBB can include one or more linkers. As noted above, the targeting agent, the BBB-Traversing Polypeptides and/or the cargo molecule can be linked directly or indirectly via one or more linkers.

An exemplary coupling agent is biotin. For example, the BBB-traversing polypeptide can be a biotinylated polypeptide. As demonstrated in the Examples, when the BBB-traversing polypeptide is biotinylated, the conjugate can be formed through specific interaction with a streptavidin-conjugated active agent.

As used herein, the term "linker" refers to a carbon chain that can contain heteroatoms (*e.g*., nitrogen, oxygen, sulfur, *etc*.) and which may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 atoms long. Linkers may be substituted with various substituents including, but not limited to, hydrogen atoms, alkyl, alkenyl, alkynl, amino, alkylamino, dialkylamino, trialkylamino, hydroxyl, alkoxy, halogen, aryl, heterocyclic, aromatic heterocyclic, cyano, amide, carbamoyl, carboxylic acid, ester, thioether, alkylthioether, thiol, and ureido groups. Those of skill in the art will recognize that each of these groups may in turn be substituted. Examples of linkers include, but are not limited to, pH-sensitive linkers, protease-cleavable peptide linkers, nuclease sensitive nucleic acid linkers, lipase sensitive lipid linkers, glycosidase sensitive carbohydrate linkers, hypoxia sensitive linkers, photo-cleavable linkers, heat-labile linkers, enzyme cleavable linkers (*e.g*., esterase cleavable linker), ultrasound-sensitive linkers, and x-ray cleavable linkers.

In some forms, the linking moiety is designed to be cleaved *in vivo*. The linking moiety can be designed to be cleaved hydrolytically, enzymatically, or by a combination thereof, to provide for the sustained release of the attached agents *in vivo*. Both the composition of the linking moiety and its point of attachment to the agent are selected so that cleavage of the linking moiety releases either an therapeutic, prophylactic or diagnostic agent or a prodrug thereof. The composition of the linking moiety can also be selected in view of the desired release rate of the agents.

In some forms, the attachment of one or more agents occurs via one or more of disulfide, ester, ether, thioester, carbamate, carbonate, hydrazine, or amide linkages. In preferred forms, the attachment occurs via an appropriate spacer that provides an ester bond or an amide bond between the agent and the polypeptide depending on the desired release kinetics of the agent. In an exemplary form, the linker is a valine citrulline (VCit) dipeptide linker. For example, in some forms a VCit linker is used as an enzymatically cleavable linker between a cargo molecule and a BBB-traversing polypeptide. In some forms, compositions including VCit linkers are internalized by cells, such as target cells, and are cleaved by cathepsins within the cells resulting in traceless release of the cargo molecule(s).

### 3. Targeting Moieties

In some forms, conjugates of BBB-Traversing Polypeptides include one or more targeting moieties for targeting of the conjugate to one or more specific tissues or cells *in vivo*. The targeting moiety is distinct from the BBB-Traversing domain.

In an exemplary form, one or more targeting moieties target the conjugate to one or more specific tissues or cells within the brain to the brain or CNS. In other forms the one or more targeting moieties target the conjugate to the brain epithelial cells and/or to the BBB. As noted above, the targeting agent, the BBB-Traversing Polypeptides and/or the cargo molecule can be linked directly or indirectly via one or more linkers.

In some forms, the targeting moiety directs the conjugate to the BBB, or to tissues/cells associated with the BBB. In other forms, the targeting moiety directs the conjugate to a specific location within the brain. For example, in some forms the targeting moiety directs the conjugate to a specific location that following traversal of the BBB.

Typically, targeting moieties exploit the surface-markers specific to a group of cells to be targeted. Exemplary targeting elements include proteins, peptides, nucleic acids, lipids, saccharides, or polysaccharides that bind to one or more targets associated with cell, or extracellular matrix, or specific type of tumor or infected cell. Targeting molecules can be selected based on the desired physical properties, such as the appropriate affinity and specificity for the target. Exemplary targeting molecules having high specificity and affinity include antibodies, or antigen-binding fragments thereof. The specificity of antibodies lends particularly well to the active targeting of the compositions described herein. One of skill in the art will appreciate that any antibody that specifically binds to a desired target/antigen can be used in accordance with the disclosed compositions. As such, antibodies which specifically recognize one or more types of cells, tissues, organs, or microenvironments are known in the art, and their use in the preferential targeting of the BBB-traversing polypeptide conjugates is contemplated herein. Therefore, in some forms, the compositions of BBB-traversing polypeptides include one or more antibodies or antigen binding fragments specific to an epitope. The epitope can be a linear epitope. The epitope can be specific to one cell type or can be expressed by multiple different cell types. In other forms, the antibody or antigen binding fragment thereof can bind a conformational epitope that includes a 3-D surface feature, shape, or tertiary structure at the surface of the target cell. In an exemplary form, the targeting moiety binds selectively to a cell, tissue or structure that is associated with the BBB. In some forms the targeting moiety binds to a cell surface receptor, such as the Lipoprotein Receptor-Related Protein 1 (LRP1), or the apolipoprotein E receptor 2 (ApoER2) and very-low-density lipoprotein receptor (VLDLR). In other forms, the targeting moiety selectively binds to a protein, such as reelin.

In certain forms the targeting moiety is a polypeptide. For example, In some forms, BBB-traversing polypeptides described herein are conjugated with polypeptide-based ligands which contribute to their preferential targeting to one or more types of cells, tissues, organs, or microenvironments. Non-limiting examples of polypeptide-based ligands include homing peptides, protein domains, and antibodies (including antibody fragments and derivatives, *e.g*., Fab, Fab', F(ab')2, Fv fragments, diabodies, affibodies, nanobodies, linear antibodies, and single-chain antibody molecules). Smaller than antibodies but larger than small molecules, short homing peptides offer additional targeting options. In some forms, the targeting moiety is an amino acid sequence that is contiguous with that of the BBB-traversing domain. Therefore, in some forms the BBB-Traversing Polypeptide includes a targeting motif sequence at the carboxyl or amino terminus relative to the position of the BBB-traversing polypeptide.

In other forms, the targeting molecule is an aptamer. Aptamers are short single-stranded DNA or RNA oligonucleotides (6 ~ 26 kDa) that fold into well-defined 3D structures that recognize a variety of biological molecules including transmembrane proteins, sugars and nucleic acids with high affinity and specificity (Yu B, et al., Mol Membr Biol., 27(7):286-98 (2010)). The high sequence and conformational diversity of naïve aptamer pools (not yet selected against a target) makes the discovery of target binding aptamers highly likely. The selection of aptamers capable of binding a target of interest is called 'Systematic Evolution of Ligands by EXponential enrichment' (SELEX). SELEX involves iterative rounds of target binding, partitioning binding from non-binding sequences, and amplification of the enriched binding sequences. Given their unique conformations with ligand-binding characteristics, typical non-immunogenicity and non-toxicity, and ability to be modified for stability in circulation, aptamers are suited to the active targeting of the BBB-traversing polypeptide and conjugates thereof described herein.

In some forms, the aptamers are nuclease resistant. In some forms, the aptamer is an RNA aptamer that is 2'-modified (*e.g*., 2'-fluro and 2'-O-methyl). In some forms, the aptamer (*e.g*., RNA aptamer) exhibits fluorescence upon binding small molecules. For example, the Spinach and Spinach2 aptamers bind and activate the fluorescence of fluorophores similar to that found in green fluorescent protein, and Broccoli is a 49-nt-long aptamer that exhibits bright green fluorescence upon binding DFHBI or DFHBI-1T (Filonov GS, et al., J Am Chem Soc.,136(46):16299-308 (2014)).

In some forms, the targeting moiety is directed to cells of the nervous system, including the brain and peripheral nervous system, or for the blood-brain barrier itself. Cells in the brain include several types and states and possess unique cell surface molecules specific for the type. Furthermore, cell types and states can be further characterized and grouped by the presentation of common cell surface molecules.

The targeting moiety can be directed to specific neurotransmitter receptors expressed on the surface of cells of the nervous system. The distribution of neurotransmitter receptors is well known in the art and one so skilled can direct the compositions described by using neurotransmitter receptor specific antibodies as targeting signals. Furthermore, given the tropism of neurotransmitters for their receptors, in one form the targeting signal includes a neurotransmitter or ligand capable of specifically binding to a neurotransmitter receptor.

The targeting moiety can be specific to cells of the nervous system which may include astrocytes, microglia, neurons, such as mature neorons, oligodendrocytes and Schwann cells. These cells can be further divided by their function, location, shape, neurotransmitter class and pathological state. Cells of the nervous system can also be identified by their state of differentiation, for example stem cells. Exemplary markers specific for these cell types and states are well known in the art and include but are not limited to CD133 and Neurosphere. Specific preferred brain targeting moieties include, but are not limited to, the peptide mHph2 and the peptide chlorotoxin (CTX), insulin receptor (which can be target by, for example, 83-14 antibody or insulin), EGF receptor (which can be targeted by, for example, cetuximab and fragments (*e.g*., Fab') thereof), low-density lipoprotein receptor (which can be targeted by, for example, apolipoproteins such as ApoA, ApoE, *etc*.), thiamine receptor (which can be targeted with, for example, thiamine), transferrin receptor (which can be targeted with, for example, transferrin and OX26 antibody, and 8D3 antibody), folate receptor (which can be targeted with, for example, folate and with its derivatives), glycoside receptor (which can be targeted with, for example, glycosides), lactoferrin receptor (which can be targeted with, for example, lactoferrin), insulin-like growth factor receptors (IGF1R & IGF2R) (which can be targeted with, for example, insulin like growth factor 1 & 2 (IGF-1 & IGF-2), , leptin receptor (LEPR) (which can be targeted with, for example, leptin), Fc-like growth factor receptor(FCGRT) (which can be target with, for example, IgG), scavenger receptor type B1 (SCARB1) (which can be targeted with, for example, (modified lipoproteins, like acetylated low density lipoprotein (LDL)), and others targets and targeting moieties discussed in Alam, et al., European Journal of Pharmaceutical Sciences, 40:385-403 (2010), and Wong, et al., Adv Drug Deliv Rev., 64(7):686-700 (2012)).

In other forms, targeting moieties and markers are related to, or specific for, the condition being treated. For example, in some forms, the targeting moiety targets a marker of cancer, or cancer-associated stromal cells such as M2-skewed macrophages (discussed in more detail below), demyelinating diseases such as sclerosis multiplex (tPA and other extracellular proteases), traumatic brain injury (e.g. lectican family of chondroitin sulfate proteoglycans), stroke (*e.g*., MMP2, thrombin), epilepsy (*e.g*., MMP2), injury, or a neurological or neurodegenerative disease or disorder.

### 4. Carriers

Conjugates of BBB-Traversing Polypeptides can include one or more carriers. For example, in some forms, the conjugates include one or more BBB-Traversing Polypeptides and optionally one or more active agents encapsulated within, associated with, or otherwise bound to a carrier. In some forms, the carrier is an inorganic carrier, such as a metal particle. Exemplary metal particles include silver or gold particles. In other forms the carrier is an organic carrier, such as a nanogel, nanolipogels, polymeric particles, lipid particles, hybrid lipid-polymer particles, inorganic particles, liposomes (*e.g*., nanoliposomes), nanosuspensions, nanoemulsions, multilamellar vesicles, nanofibers, nanorobots, solid lipid nanoparticles (SLN), nanostructured lipid carriers (NLC), and lipid drug conjugates (LDC). In some forms, the particulate nanocarriers are nanoscale compositions, for example, 10 nm up to, but not including, about 1 micron, more preferably up to about 500 nm, as discussed below. However, it will be appreciated that in some forms, and for some uses, the particles can be smaller, or larger (*e.g*., microparticles, *etc*.). It will be appreciated that in some forms and for some uses the conjugate/carrier compositions can have nanoscale or microscale dimensions. Such compositions can be referred to as nanoparticles or microparticles.

In preferred forms for treating diseases and disorders of the brain, it is desirable that the conjugate/carrier be of a size suitable to cross the blood-brain barrier. Therefore, in some forms, the conjugate/carrier is in the range of about 25 nm to about 500 nm inclusive, more preferably in the range of about 50 nm to about 350 nm inclusive, most preferably between about 70 nm and about 300 nm inclusive. In some forms, the carrier acts as a drug carrier (*e.g*., submicroscopic colloidal systems such as nanospheres with a matrix system in which the drug is dispersed) or nanocapsules (*e.g*., reservoirs in which the drug is confined surrounded by a single polymeric membrane)).

The carrier can contain one or more lipids or amphiphilic compounds. For example, the particles can be liposomes, lipid micelles, solid lipid particles, or lipid-stabilized polymeric particles. The lipid particle can be made from one or a mixture of different lipids. Lipid particles are formed from one or more lipids, which can be neutral, anionic, or cationic at physiologic pH. The lipid particle is preferably made from one or more biocompatible lipids. The lipid particles may be formed from a combination of more than one lipid, for example, a charged lipid may be combined with a lipid that is non-ionic or uncharged at physiological pH.

### a. Moieties for Enhanced Stability and/or Half-life

In some forms, the conjugates of BBB-traversing peptides associated with carrier molecules include one or more molecules to enhance stability and/or half-life of the conjugates *in vivo*. Particle size, size distribution, shape, and surface characteristic are important characteristics of BBB-traversing peptide conjugates/carrier molecules. In some forms, these features impact the *in vivo* distribution, biological fate, toxicity, clearance, uptake and targeting ability of BBB-traversing peptide conjugates/carrier molecules to act as delivery systems for cargo molecules. In addition, they can influence cargo (*e.g*., drug) loading and release, and stability of the composition (Singh R and Lillard JW Jr. Exp Mol Pathol. 86(3):215-23 (2009); Bamrungsap S, et al., Nanomedicine. 7(8):1253-1271 (2012)). Accordingly, in some forms, the size, size distribution, shape, geometry, surface characteristics (e.g., surface charge, surface chemistry) of the BBB-traversing peptide conjugates/carrier molecules are modified and/or selected to enhance stability and/or half-life of the compositions *in vivo*.

Besides size and shape, surface characteristics of compositions can also determine their lifespan during circulation in the blood stream. A major discovery was the finding that compositions (e.g., BBB-traversing peptides associated with carrier molecules) coated with hydrophilic polymer molecules, such as polyethylene glycol (PEG), can resist serum protein adsorption, prolonging the systemic circulation of the particle. Numerous variations of PEG and other hydrophilic polymers have been tested for improved circulation. The surface charge on the particle also affects other functions, such as internalization by macrophages. Positively charged particles have been shown to exhibit higher internalization by macrophages and dendritic cells compared with neutral or negatively charged particles, although surface charge effect could also be cell-type dependent. Therefore, in some forms, the BBB-traversing peptides associated with carrier molecules are conjugated or complexed with one or more molecules having a negative charge, to provide a molecule having a negative overall surface charge *in vivo*. Suitable molecules include polyethylene glycol (PEG) molecules, lipids, polar groups, charged groups, amphipathic groups, and albumin binding molecules.

To enhance half-life of the disclosed BBB-traversing peptide conjugates/ carrier molecules, minimized opsonization and prolonged circulation *in vivo* are desired. This can be achieved, for example, by coating BBB-traversing peptides associated with carrier molecules with hydrophilic polymers/surfactants or formulating the BBB-traversing peptides with biodegradable copolymers with hydrophilic characteristics, *e.g*., PEG, polyethylene oxide, dextran, polyoxamer, poloxamine, and polysorbate 80 (Tween 80). Studies show that PEG on nanoparticle surfaces prevents opsonization by complement and other serum factors. PEG molecules with brush-like and intermediate configurations reduce phagocytosis and complement activation, whereas surfaces comprised of PEG with mushroom-like structures are potent complement activators and favor phagocytosis (Singh R and Lillard JW Jr. Exp Mol Pathol. 86(3):215-23 (2009)).

In some forms, the BBB-traversing peptides are coated with a hydrophilic layer (*e.g*., PEG, polyethylene oxide, polyoxamer, poloxamine, and polysorbate 80 (Tween 80)) to enhance stability and/or half-life of the compositions in vivo. Non-PEG based alternatives such as polyoxazolines, poly(amino acids), polybetaines, polyglycerols, and polysaccharide derivatives may also be used to enhance stability and/or half-life (Amoozgar Z, and Yeo Y. Wiley Interdiscip Rev Nanomed Nanobiotechnol., 4(2):219-33 (2012)). In some forms, the BBB-traversing peptides are coated with polyoxazolines (POZ), poly(amino acids) such as poly(hydroxyethyl l-glutamine) and poly(hydroxyethyl-l-asparagine), N-(2-hydroxypropyl)methacrylamide (HPMA) and its derivatives, polybetaines such as sulfobetaine and carboxybetaine, polyglycerols (also known as polyglycidols), and polysaccharides such as, derivatives of chitosan, dextran, hyaluronic acid, and heparin.

In some forms, the BBB-traversing peptide conjugates/carrier molecules include a plurality of effector molecules which may contribute to their physiochemical properties (*e.g*., enhanced stability and/or half-life). In some forms, an effector molecule is any of the above-mentioned molecules that can be used to coat the nucleic acid assembly (*e.g*., PEG, polyethylene oxide, polyoxamer, poloxamine, and polysorbate 80 (Tween 80), polyoxazolines, poly(amino acids), HPMA, polybetaines, polyglycerols, and polysaccharide derivatives). In some forms, an effector molecule is a polyethylene glycol molecule, lipid, polar group, charged group, amphipathic group, or albumin binding molecule.

### III. Pharmaceutical Compositions

Pharmaceutical compositions including conjugates of BBB-Traversing polypeptides are also disclosed. The pharmaceutical compositions can be formulated for administration by parenteral (intramuscular, intraperitoneal, intravenous (IV) or subcutaneous injection), by instillation, or in a depo, formulated in dosage forms appropriate for each route of administration.

### A. Formulations for Parenteral Administration

In some forms the compositions are administered in an aqueous solution, by parenteral injection. The formulation can be in the form of a suspension or emulsion. In general, pharmaceutical compositions are provided including effective amounts of one or more active agents optionally include pharmaceutically acceptable diluents, preservatives, solubilizers, emulsifiers, adjuvants and/or carriers. Such compositions can include diluents such as sterile water, buffered saline of various buffer content (*e.g*., Tris-HCl, acetate, phosphate), pH and ionic strength; and optionally, additives such as detergents and solubilizing agents (*e.g*., TWEEN^{®} 20, TWEEN^{®} 80 also referred to as polysorbate 20 or 80), anti-oxidants (*e.g*., ascorbic acid, sodium metabisulfite), and preservatives (*e.g*., Thimersol, benzyl alcohol). Examples of non-aqueous solvents or vehicles are propylene glycol, polyethylene glycol, vegetable oils, such as olive oil and corn oil, gelatin, and injectable organic esters such as ethyl oleate. The formulations may be lyophilized and resuspended immediately before use. The formulation may be sterilized by, for example, filtration through a bacteria-retaining filter, by incorporating sterilizing agents into the compositions, by irradiating the compositions, or by heating the compositions.

### B. Formulations for Mucosal Administration

In some forms, the compositions are formulated for mucosal administration, for example via pulmonary or intranasal delivery.

These methods of administration can be made effective by formulating the composition with mucosal transport elements. Compositions can be delivered to the lungs while inhaling and traverse across the lung epithelial lining to the blood stream when delivered either as an aerosol or spray dried particles having an aerodynamic diameter of less than about 5 microns.

A wide range of mechanical devices designed for pulmonary delivery of therapeutic products can be used, including but not limited to nebulizers, metered dose inhalers, and powder inhalers, all of which are familiar to those skilled in the art.

Formulations for administration to the mucosa will typically be spray dried drug particles, which may be incorporated into a tablet, gel, capsule, suspension or emulsion. Standard pharmaceutical excipients are available from any formulator.

Mucosal formulations may include one or more agents for enhancing delivery through the nasal mucosa. Agents for enhancing mucosal delivery are known in the art, see, for example, U.S. Patent Application No. 2009/0252672 to Eddington, and U.S. Patent Application No. 2009/0047234 to Touitou.

Acceptable agents include, but are not limited to, chelators of calcium (EDTA), inhibitors of nasal enzymes (boro-leucin, aprotinin), inhibitors of muco-ciliar clearance (preservatives), solubilizers of nasal membrane (cyclodextrin, fatty acids, surfactants) and formation of micelles (surfactants such as bile acids, Laureth 9 and taurodehydrofusidate (STDHF)). Compositions may include one or more absorption enhancers, including surfactants, fatty acids, and chitosan derivatives, which can enhance delivery by modulation of the tight junctions (TJ) (B. J. Aungst, et al., J. Pharm. Sci. 89(4):429-442 (2000)). In general, the optimal absorption enhancer should possess the following qualities: its effect should be reversible, it should provide a rapid permeation enhancing effect on the cellular membrane of the mucosa, and it should be non-cytotoxic at the effective concentration level and without deleterious and/or irreversible effects. Intranasal compositions maybe administered using devices known in the art, for example a nebulizer.

### C. Formulations for Enteral Administration

Pharmaceutical compositions for oral administration can be liquid or solid. Liquid dosage forms suitable for oral administration include, but are not limited to, pharmaceutically acceptable emulsions, microemulsions, solutions, suspensions, syrups and elixirs. In addition to the encapsulated or unencapsulated compound, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor and sesame oils), glycerol, tetrahydrofuryl alcohol, polyethylene glycols and fatty acid esters of sorbitan and mixtures thereof. Besides inert diluents, the oral compositions can also include adjuvants, wetting agents, emulsifying and suspending agents, sweetening, flavoring and perfuming agents.

Solid dosage forms for oral administration include, but are not limited to, capsules, tablets, caplets, dragees, powders and granules. In such solid dosage forms, the encapsulated or unencapsulated compound is typically mixed with at least one inert, pharmaceutically acceptable excipient or carrier such as sodium citrate or dicalcium phosphate and/or (a) fillers or extenders such as starches, lactose, sucrose, glucose, mannitol and silicic acid, (b) binders such as, for example, carboxymethylcellulose, alginates, gelatin, polyvinylpyrrolidinone, sucrose and acacia, (c) humectants such as glycerol, (d) disintegrating agents such as agar-agar, calcium carbonate, potato or tapioca starch, alginic acid, certain silicates and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay and (i) lubricants such as talc, calcium stearate, magnesium stearate, solid polyethylene glycols, sodium lauryl sulfate and mixtures thereof. In the case of capsules, tablets and pills, the dosage form may also contain buffering agents.

Solid compositions of a similar type may also be employed as fill materials in soft and hard-filled gelatin capsules using such excipients as lactose or milk sugar as well as high molecular weight polyethylene glycols and the like. The solid dosage forms of tablets, dragees, capsules, pills and granules can be prepared with coatings and shells such as enteric coatings and other coatings well known in the pharmaceutical formulating art, which can confer enteric protection or enhanced delivery through the GI tract, including the intestinal epithelia and mucosa (see Samstein, et al. Biomaterials. .29(6):703-8 (2008)).

### IV. Methods of Use

Methods of using Polypeptides that specifically and selectively traverse the healthy blood-brain barrier (BBB) to deliver conjugated active agents to the brain from the circulation have been established.

As described in the Examples, the BBB-traversing peptide domain "SRRVISRAKLAAAL" homes preferentially to the eyes/brain/CNS, particularly to neurons within compartments of the brain that express the Low-density lipoprotein receptor-related protein 1 (LRP1) endocytic cell surface receptor.

As described in the Examples, the BBB-traversing peptide domain "CVGTNCY" (also referred to as the "CVG" peptide) homes preferentially to the eyes/brain/CNS, particularly to neurons within compartments of the brain including the hippocampus, frontal cortex and cerebellum.

In some forms, the BBB traversing polypeptide conjugates cross the BBB and selectively target or enriched within neurons, preferably within the nucleus of neurons of injured/hyperactive neurons. In further forms, the BBB traversing polypeptide conjugates accumulate in one or more cell types including oligodendroglia, microglia and astrocytes.

Typically, an effective amount of the BBB-Traversing polypeptide conjugates including one or more therapeutic, prophylactic, and/or diagnostic active agents are administered to an individual in need thereof. The conjugates may also include a targeting agent, but as demonstrated by the examples, these are not required for delivery to neurons in the brain, spinal cord or other components of CNS.

In some forms, the BBB-Traversing polypeptide conjugates are capable of releasing the therapeutic, prophylactic or diagnostic agents intracellularly under the conditions found *in vivo*. The amount of BBB traversing polypeptide conjugates administered to the subject is selected to deliver an effective amount to reduce, prevent, or otherwise alleviate one or more clinical or molecular symptoms of the disease or disorder to be treated compared to a control, for example, a subject treated with the therapeutic, prophylactic or diagnostic agent without BBB traversing polypeptide conjugates.

In some forms, the methods including a step of selecting a subject who is likely to benefit from treatment with the BBB-traversing polypeptide conjugate compositions.

### A. Methods of Treatment

The compositions are suitable for treating one or more diseases, conditions, and injuries in the brain, and the nervous system, such as cancer and diseases associated with pathological activation of neurons. The compositions can also be used for treatment of neurological diseases and treatment of other tissues where the nerves play a role in the disease or disorder. The compositions and methods are also suitable for prophylactic use.

Methods of treating or preventing one or more symptoms of a disease or disorder in the brain or CNS of a subject are described. Typically, the methods include administering to the subject an effective amount of a BBB-traversing polypeptide conjugate including one or more therapeutic agents for treating or preventing one or more symptoms of a disease or disorder in the brain or CNS, or a pharmaceutical composition thereof, in an amount effective to prevent or reduce one or more symptoms of a disease or disorder in the brain or CNS of the subject.

Uses of BBB-traversing polypeptide conjugates including one or more therapeutic agents in the manufacture of a medicament for treating or preventing one or more symptoms of a disease or disorder in the brain or CNS of a subject are also provided. Formulations of a BBB-traversing polypeptide conjugate, or a pharmaceutical composition thereof, including one or more therapeutic agents for treating or preventing one or more symptoms of a disease or disorder in the brain or CNS of a subject, or a pharmaceutical composition thereof for use in treating or preventing one or more symptoms of a disease or disorder in the brain or CNS of a subject are also provided.

The BBB-traversing polypeptide conjugate compositions selectively deliver active agents into the brain to treat the causes and symptoms of many disorders and conditions including neurodevelopmental, neurodegenerative diseases, and brain cancer. Thus, in some forms, the BBB-traversing polypeptide conjugate compositions are administered in a dosage unit amount effective to treat or alleviate conditions associated with the pathological conditions of neurons. Generally, by targeting neurons, the BBB-traversing polypeptide conjugate compositions deliver agent specifically to treat the diseased neurons with minimal toxicity. Therefore, in some forms, the BBB-traversing polypeptide conjugate compositions are administered in an amount effective to treat diseased neuron-mediated pathology in the subject in need thereof without any associated toxicity.

Typically, the subject to be treated is a human. In some forms, the subject to be treated is a child, or an infant. All the methods can include the step of identifying and selecting a subject in need of treatment, or a subject who would benefit from administration with the described compositions.

### 1. Neurological and Neurodegenerative Diseases

The BBB-traversing polypeptide conjugate compositions and formulations thereof can be used to diagnose and/or to treat one or more neurological and neurodegenerative diseases. The compositions and methods are particularly suited for treating one or more neurological, or neurodegenerative diseases associated with defective or diseased neurons. In some forms, the disease or disorder is selected from, but not limited to, some psychiatric (*e.g*., depression, schizophrenia (SZ), alcohol use disorder, and morphine antinociceptive tolerance), neurological and neurodegenerative (*e.g*., Alzheimer's disease (AD), Parkinson disease (PD), Amyotrophic Lateral Sclerosis (ALS)) disorders. In one form, the BBB-traversing polypeptide conjugate compositions are used to treat Alzheimer's Disease (AD) or dementia).

Neurodegenerative diseases are chronic progressive disorders of the nervous system that affect neurological and behavioral function and involve biochemical changes leading to distinct histopathologic and clinical syndromes (Hardy H, et al., Science. 1998;282:1075-9). Abnormal proteins resistant to cellular degradation mechanisms accumulate within the cells. The pattern of neuronal loss is selective in the sense that one group gets affected, whereas others remain intact. Often, there is no clear inciting event for the disease. The diseases classically described as neurodegenerative are Alzheimer's disease, Huntington's disease, and Parkinson's disease.

The BBB-traversing polypeptide conjugate compositions and methods can also be used to deliver agents for the treatment of a neurological or neurodegenerative disease or disorder, or central nervous system disorder. In preferred forms, the BBB-traversing polypeptide conjugate compositions and methods are effective in treating, and/or alleviating neuroinflammation associated with a neurological or neurodegenerative disease or disorder or central nervous system disorder.

In some forms the methods include administering to the subject an effective amount of the BBB-traversing polypeptide conjugate compositions to increase cognition or reduce a decline in cognition, increase a cognitive function or reduce a decline in a cognitive function, increase memory or reduce a decline in memory, increase the ability or capacity to learn or reduce a decline in the ability or capacity to learn, or a combination thereof.

Neurodegeneration refers to the progressive loss of structure or function of neurons, including death of neurons. For example, in some forms, the methods administer BBB-traversing polypeptide conjugate compositions to treat subjects with a disease or disorder, such as Parkinson's Disease (PD) and PD-related disorders, Huntington's Disease (HD), Amyotrophic Lateral Sclerosis (ALS), Alzheimer's Disease (AD) and other dementias, Prion Diseases such as Creutzfeldt-Jakob Disease, Corticobasal Degeneration, Frontotemporal Dementia, HIV-Related Cognitive Impairment, Mild Cognitive Impairment, Motor Neuron Diseases (MND), Spinocerebellar Ataxia (SCA), Spinal Muscular Atrophy (SMA), Friedreich's Ataxia, Lewy Body Disease, Alpers' Disease, neuronal ceroid lipofuscinoses, Batten Disease, Cerebro-Oculo-Facio-Skeletal Syndrome, Corticobasal Degeneration, Gerstmann-Straussler-Scheinker Disease, Kuru, Leigh's Disease, Monomelic Amyotrophy, Multiple System Atrophy, Multiple System Atrophy With Orthostatic Hypotension (Shy-Drager Syndrome), Multiple Sclerosis (MS), Duchenne muscular dystrophy (DMD), Neurodegeneration with Brain Iron Accumulation, Opsoclonus Myoclonus, Posterior Cortical Atrophy, Primary Progressive Aphasia, Progressive Supranuclear Palsy, Vascular Dementia, Progressive Multifocal Leukoencephalopathy, Dementia with Lewy Bodies (DLB), Lacunar syndromes, Hydrocephalus, Wernicke-Korsakoff's syndrome, post-encephalitic dementia, cancer and chemotherapy-associated cognitive impairment and dementia, and depression-induced dementia and pseudodementia.

In some forms, the disease or disorder is spinal muscular atrophy. In such cases, HDAC inhibitors, antisense oligonucleotide (ASO) drug nusinersen, or gene therapy drug ZOLGENSMA^{®} can be conjugated to BBB-traversing polypeptides for delivery to neurons or nucleus of neurons for the treatment of spinal muscular atrophy.

In other forms, the disease or disorder is injection-localized amyloidosis, cerebral amyloid angiopathy, myopathy, neuropathy, brain trauma, frontotemporal dementia, Pick's disease, multiple sclerosis, prion disorders, diabetes mellitus type 2, fatal familial insomnia, cardiac arrhythmias, isolated atrial amyloidosis, atherosclerosis, rheumatoid arthritis, familial amyloid polyneuropathy, hereditary non-neuropathic systemic amyloidosis, Finnish amyloidosis, lattice corneal dystrophy, systemic AL amyloidosis, neuronopathic Gaucher disease, or Down syndrome. In preferred forms, the disease or disorder is Alzheimer's disease or dementia.

Criteria for assessing improvement in a particular neurological factor include methods of evaluating cognitive skills, motor skills, memory capacity or the like, as well as methods for assessing physical changes in selected areas of the central nervous system, such as magnetic resonance imaging (MRI) and computed tomography scans (CT) or other imaging methods. Such methods of evaluation are well known in the fields of medicine, neurology, psychology and the like, and can be appropriately selected to diagnosis the status of a particular neurological impairment. To assess a change in Alzheimer's disease, or related neurological changes, the selected assessment or evaluation test, or tests, are given prior to the start of administration of the BBB-traversing polypeptide conjugate compositions. Following this initial assessment, treatment methods for the administration of the BBB traversing polypeptide conjugates are initiated and continued for various time intervals. At a selected time interval subsequent to the initial assessment of the neurological defect impairment, the same assessment or evaluation test (s) is again used to reassess changes or improvements in selected neurological criteria.

### a. Alzheimer's Disease and Dementia

The BBB-traversing polypeptide conjugate compositions are suitable for reducing or preventing one or more pathological processes associated with the development and progression of neurological diseases such as Alzheimer's disease and dementia. Thus, methods for treatment, reduction, and prevention of the pathological processes associated with Alzheimer's disease include administering the BBB-traversing polypeptide conjugate compositions in an amount and dosing regimen effective to reduce brain and/or serum exosomes, brain and/or serum ceramide levels, serum anti-ceramide IgG, glial activation, total Aβ42 and plaque burden, tau phosphorylation/propagation, and improved cognition in a learning task, such as a fear-conditioned learning task, in an individual suffering from Alzheimer's disease or dementia are provided. Methods for reducing, preventing, or reversing the learning and/or memory deficits in an individual suffering from Alzheimer's disease or dementia are provided.

In some forms, the BBB-traversing polypeptide conjugate compositions are administered in an amount and dosing regimen effective to induce neuro-enhancement in a subject in need thereof. Neuro-enhancement resulting from the administration of the BBB-traversing polypeptide conjugate compositions includes the stimulation or induction of neural mitosis leading to the generation of new neurons, *i.e*., exhibiting a neurogenic effect, prevention or retardation of neural loss, including a decrease in the rate of neural loss, *i.e*., exhibiting a neuroprotective effect, or one or more of these modes of action. The term "neuroprotective effect" includes prevention, retardation, and/or termination of deterioration, impairment, or death of an individual's neurons, neurites, and neural networks. Administration of the BBB-traversing polypeptide conjugate compositions leads to an improvement, or enhancement, of neurological function in an individual with a neurological disease, neurological injury, or age-related neuronal decline or impairment.

Neural deterioration can be the result of any condition which compromises neural function which is likely to lead to neural loss. Neural function can be compromised by, for example, altered biochemistry, physiology, or anatomy of a neuron, including its neurite. Deterioration of a neuron may include membrane, dendritic, or synaptic changes, which are detrimental to normal neuronal functioning. The cause of the neuron deterioration, impairment, and/or death may be unknown. Alternatively, it may be the result of age-, injury-and/or disease-related neurological changes that occur in the nervous system of an individual.

In Alzheimer's patients, neural loss is most notable in the hippocampus, frontal, parietal, and anterior temporal cortices, amygdala, and the olfactory system. The most prominently affected zones of the hippocampus include the CA1 region, the subiculum, and the entorhinal cortex. Memory loss is considered the earliest and most representative cognitive change because the hippocampus is well known to play a crucial role in memory.

Neural loss through disease, age-related decline or physical insult leads to neurological disease and impairment. The BBB-traversing polypeptide conjugate compositions can counteract the deleterious effects of neural loss by promoting development of new neurons, new neurites and/or neural connections, resulting in the neuroprotection of existing neural cells, neurites and/or neural connections, or one or more these processes. Thus, the neuro-enhancing properties of the compositions provide an effective strategy to generally reverse the neural loss associated with degenerative diseases, aging and physical injury or trauma.

Administration of the BBB traversing polypeptide conjugate compositions to an individual who is undergoing or has undergone neural loss, as a result of Alzheimer's disease reduces any one or more of the symptoms of Alzheimer's disease, or associated cognitive disorders, including dementia. Clinical symptoms of AD or dementia that can be treated, reduced or prevented include clinical symptoms of mild AD, moderate AD, and/or severe AD or dementia.

In mild Alzheimer's disease, a person may seem to be healthy but has more and more trouble making sense of the world around him or her. The realization that something is wrong often comes gradually to the person and their family. Exemplary symptoms of mild Alzheimer's disease/mild dementia include memory loss; poor judgment leading to bad decisions; loss of spontaneity and sense of initiative; taking longer to complete normal daily tasks; repeating questions; trouble handling money and paying bills; wandering and getting lost; losing things or misplacing them in odd places; mood and personality changes, and increased anxiety and/or aggression.

Symptoms of moderate Alzheimer's disease/moderate dementia include forgetfulness; increased memory loss and confusion; inability to learn new things; difficulty with language and problems with reading, writing, and working with numbers; difficulty organizing thoughts and thinking logically; shortened attention span; problems coping with new situations; difficulty carrying out multistep tasks, such as getting dressed; problems recognizing family and friends; hallucinations, delusions, and paranoia; impulsive behavior such as undressing at inappropriate times or places or using vulgar language; inappropriate outbursts of anger; restlessness, agitation, anxiety, tearfulness, wandering (especially in the late afternoon or evening); repetitive statements or movement, occasional muscle twitches.

Symptoms of severe Alzheimer's disease/severe dementia include inability to communicate; weight loss; seizures; skin infections; difficulty swallowing; groaning, moaning, or grunting; increased sleeping; loss of bowel and bladder control.

Physiological symptoms of Alzheimer's disease/dementia include reduction in brain mass, for example, reduction in hippocampal volume. Therefore, in some forms, methods of administering the BBB traversing polypeptide conjugate compositions to increase the brain mass, and/or reduce or prevent the rate of decrease in brain mass of a subject; increase the hippocampal volume of the subject, reduce or prevent the rate of decrease of hippocampal volume, as compared to an untreated control subject.

The BBB-traversing polypeptide conjugate compositions are administered to provide an effective amount of one or more therapeutic agents upon administration to an individual. As used in this context, an "effective amount" of one or more therapeutic agents is an amount that is effective to improve or ameliorate one or more symptoms associated with Alzheimer's disease or dementia, including neurological defects or cognitive decline or impairment. Such a therapeutic effect is generally observed within about 12 to about 24 weeks of initiating administration of a composition containing an effective amount of one or more neuro-enhancing agents, although the therapeutic effect may be observed in less than 12 weeks or greater than 24 weeks.

The individual is preferably an adult human, and more preferably, a human over the age of 30, who has lost some amount of neurological function as a result of Alzheimer's disease or dementia. Generally, neural loss implies any neural loss at the cellular level, including loss in neurites, neural organization or neural networks.

In other forms, the methods including selecting a subject who is likely to benefit from treatment with the BBB-traversing polypeptide conjugate compositions. For example, ceramide levels in the CSF of a patient are first determined and compared to that of a healthy control. In some forms, the BBB-traversing polypeptide conjugate compositions are administered to a patient having an elevated concentration of ceramide in the CSF or in the serum relative to that of a healthy control. In other forms, the BBB-traversing polypeptide conjugate compositions are administered to a patient with increased quantity of brain and/or serum exosomes relative to that of a healthy control. In other forms, the BBB-traversing polypeptide conjugate compositions are administered to a patient with increased levels of serum anti-ceramide IgG relative to that of a healthy control.

In some forms, the subject has a nervous system disorder or is in need of neuroprotection. Exemplary conditions and/or subjects include, but are not limited to, subjects having had, subjects with, or subjects likely to develop or suffer from a stroke, a traumatic brain injury, a spinal cord injury, post-traumatic stress syndrome, or a combination thereof.

In some forms, methods administer to a subject in need thereof in an effective amount to reduce or prevent one or more molecular or clinical symptoms of a neurodegenerative disease, or one or more mechanisms that cause neurodegeneration.

Agents for the treatment of neurodegenerative diseases are well known in the art and can vary based on the symptoms and disease to be treated. For example, conventional treatment for Parkinson's disease can include levodopa (usually combined with a dopa decarboxylase inhibitor or COMT inhibitor), a dopamine agonist, or an MAO-B inhibitor.

### b. Huntington's Disease

In some forms, the methods administer BBB-traversing polypeptide conjugate compositions to treat Huntington's disease. Treatment for Huntington's disease can include a dopamine blocker to help reduce abnormal behaviors and movements, or a drug such as amantadine and tetrabenazine to control movement, *etc*. Other drugs that help to reduce chorea include neuroleptics and benzodiazepines. Compounds such as amantadine or remacemide have shown preliminary positive results. Hypokinesia and rigidity, especially in juvenile cases, can be treated with antiparkinsonian drugs, and myoclonic hyperkinesia can be treated with valproic acid. Psychiatric symptoms can be treated with medications similar to those used in the general population. Selective serotonin reuptake inhibitors and mirtazapine have been recommended for depression, while atypical antipsychotic drugs are recommended for psychosis and behavioral problems.

### c. Amyotrophic Lateral Sclerosis (ALS)

In some forms, the methods administer BBB-traversing polypeptide conjugate compositions to treat Amyotrophic Lateral Sclerosis (ALS). Riluzole (RILUTEK^{®}) (2-amino-6-(trifluoromethoxy) benzothiazole), an antiexcitotoxin, has yielded improved survival time in subjects with ALS. Other medications, most used off-label, and interventions can reduce symptoms due to ALS. Some treatments improve quality of life and a few appear to extend life. Common ALS-related therapies are reviewed in Gordon, Aging and Disease, 4(5):295-310 (2013). A number of other agents have been tested in one or more clinical trials with efficacies ranging from non-efficacious to promising. Exemplary agents are reviewed in Carlesi, et al., Archives Italiennes de Biologie, 149:151-167 (2011). For example, therapies may include an agent that reduces excitotoxicity such as talampanel (8-methyl-7H-1,3-dioxolo(2,3)benzodiazepine), a cephalosporin such as ceftriaxone, or memantine; an agent that reduces oxidative stress such as coenzyme Q10, manganoporphyrins, KNS-760704 [(6R)-4,5,6,7-tetrahydro-N6-propyl-2,6-benzothiazole-diamine dihydrochloride, RPPX], or edaravone (3-methyl-1-phenyl-2-pyrazolin-5-one, MCI-186); an agent that reduces apoptosis such as histone deacetylase (HDAC) inhibitors including valproic acid, TCH346 (Dibenzo(b,f)oxepin-10-ylmethyl-methylprop-2-ynylamine), minocycline, or tauroursodeoxycholic Acid (TUDCA); an agent that reduces neuroinflammation such as thalidomide and celastol; a neurotropic agent such as insulin-like growth factor 1 (IGF-1) or vascular endothelial growth factor (VEGF); a heat shock protein inducer such as arimoclomol; or an autophagy inducer such as rapamycin or lithium.

Treatment for Dementia with Lewy Bodies can include, for example, acetylcholinesterase inhibitors such as tacrine, rivastigmine, galantamine or donepezil; the N-methyl d-aspartate receptor antagonist memantine; dopaminergic therapy, for example, levodopa or selegiline; antipsychotics such as olanzapine or clozapine; REM disorder therapies such as clonazepam, melatonin, or quetiapine; anti-depression and antianxiety therapies such as selective serotonin reuptake inhibitors (citalopram, escitalopram, sertraline, paroxetine, *etc.*) or serotonin and noradrenaline reuptake inhibitors (venlafaxine, mirtazapine, and bupropion) (see, *e.g*., Macijauskiene, et al., Medicina (Kaunas), 48(1):1-8 (2012)).

Other common therapeutic, prophylactic or diagnostic agents for treating neurological dysfunction include amantadine and anticholinergics for treating motor symptoms, clozapine for treating psychosis, cholinesterase inhibitors for treating dementia, and modafinil for treating daytime sleepiness.

### d. Pain

In some forms, the methods administer BBB-traversing polypeptide conjugate compositions to treat pain in a subject. The pain can be acute pain, or chronic pain. The The pain can be associated with one or more diseases that causes pain due to excessive tissue swelling or growth, or due to nerve damage or death. In some forms, the methods administer BBB-traversing polypeptide conjugate compositions to treat or prevent pain in the brain or CNS or eyes of the subject. In other forms, the methods administer BBB-traversing polypeptide conjugate compositions to treat or prevent pain in one or more other regions of the body, for example, referred pain and/or neuropathic pain that derives within the brain, or CNS, or eyes of the subject.

There are three broad categories of analgesic medications, including: (1) nonopioid analgesics, which includes the nonsteroidal anti-inflammatory drugs (NSAIDs), acetaminophen, dipyrone, and others; (2) the "adjuvant analgesics," which are defined as "drugs that have primary indications other than pain but may be analgesic in selected circumstances;" and (3) opioid analgesics. Therefore, in some forms, the methods administer one or more of non-opioid analgesics, adjuvant analgesics or opioid analgesics to a subject to prevent or reduce pain in the subject. Methods of scoring and assessing pain are known in the art. Therefore, in some forms, the methods reduce or prevent pain by administering one or more BBB-traversing polypeptide conjugate compositions including one or more analgesics to a subject in need thereof to reduce or prevent pain in the subject.

### (a) Neuropathic Pain

In some forms, the methods administer BBB-traversing polypeptide conjugate compositions to treat neuropathic pain in a subject. In some forms, the methods treat or prevent neuropathic pain resulting from a disease or disorder including diabetic neuropathy, shingles, post herpetic neuralgia, neuromas, phantom limb pain and trigeminal neuralgia. In some forms, the disease or condition is an established or idiopathic chronic pain syndrome and/or conditions, including fibromyalgia and complex regional pain syndrome. In some forms, the disease of condition is demyelinating myelinoclastic disease or a demyelinating leukodystrophic disease. In some forms, the disease or condition is inflammatory demyelination, viral demyelination, acquired metabolic demyelination, hypoxic-ischemic demyelination, or compression-induced demyelination. In some forms, the disease or condition is diabetic neuropathy, shingles, post herpetic neuralgia, neuromas, phantom limb pain, trigeminal neuralgia, multiple sclerosis, acute multiple sclerosis, neuromyelitis optica, concentric sclerosis, acute-disseminated encephalonyelitis, acute hemorrhagic leucoencephalitis, progressive multifocal leucoencephalopathy, human immunodeficiency virus infection, subacute sclerosing panencephalitis, central pontine myelinlysis, extrapontine myelinolysis, fibromyalgia, or complex regional pain syndrome.

### 2. Neurodevelopmental Disorders

Neurodevelopmental disorder generally implies that the brain is not formed normally from the beginning. Abnormal regulation of fundamental neurodevelopmental processes may occur, or there may be disruption by insult that may take various forms. Autism and attention deficit hyperactivity disorder have been classically described as neurodevelopmental disorders.

Cerebral palsy (CP) is one of the most common pediatric neurological/neurodevelopmental disorder, currently estimated to affect approximately 2 to 3 per thousand live births (Kirby, RS et al., Research in Developmental Disabilities, 32, 462 (2011)). CP is recognized in early childhood and the condition persists throughout the life. The most common causes of CP include prematurity, hypoxia-ischemia and placental insufficiency, birth asphyxia and maternal-fetal inflammation (Dammann, O. Acta Pædiatrica 2007, 96, 6; Yoon, BH et al., American Journal of Obstetrics and Gynecology 2000, 182, 675; and O'Shea, TM et al., Journal of child neurology 2012, 27, 22). Although CP is heterogeneous in etiology and mechanism of disease is very complex, however, neuroinflammation is a common pathophysiologic mechanism that is involved irrespective of the etiology. Targeting neuroinflammation and delivering drugs directly at the injured site can be beneficial.

The compositions and methods can also be used to deliver therapeutic, prophylactic or diagnostic agents for the treatment of a neurodevelopmental disorder, such as cerebral palsy. In preferred forms, the compositions and methods are effective in treating, and/or alleviating neuroinflammation associated with a neurodevelopmental disorder, such as cerebral palsy.

In some forms, the BBB-traversing polypeptide conjugate compositions are effective to treat, image, and/or prevent inflammation of the brain in neurodevelopmental disorders, including, for example Rett syndrome. In a preferred form, the BBB-traversing polypeptide conjugate compositions would be used to deliver an anti-inflammatory agent (D-NAC) and anti-excitotoxic and D-anti-glutamate agents. Exemplary candidates are: MK801, Memantine, 1-MT.

In some forms, the BBB-traversing polypeptide conjugate compositions are effective to treat, image, and/or prevent inflammation of the brain in autism spectrum disorders. The term "spectrum" refers to the wide range of symptoms, skills, and levels of impairment or disability that children with ASD can have. Some children are mildly impaired by their symptoms, while others are severely disabled. The latest edition of the Diagnostic and Statistical Manual of Mental Disorders (DSM-5) no longer includes Asperger's syndrome; although the characteristics of Asperger's syndrome are included within the broader category of ASD.

At this time, the only medications approved by the FDA to treat aspects of ASD are the antipsychotics risperidone (Risperdal) and aripripazole (Abilify). Some medications that may be prescribed off-label for children with ASD include the following:
Antipsychotic medications are more commonly used to treat serious mental illnesses such as schizophrenia. These medicines may help reduce aggression and other serious behavioral problems in children, including children with ASD. They may also help reduce repetitive behaviors, hyperactivity, and attention problems.

Antidepressant medications, such as fluoxetine or sertraline, are usually prescribed to treat depression and anxiety but are sometimes prescribed to reduce repetitive behaviors. Some antidepressants may also help control aggression and anxiety in children with ASD.

Stimulant medications, such as methylphenidate (RITALIN^{®}), are safe and effective in treating people with attention deficit hyperactivity disorder (ADHD). Methylphenidate has been shown to effectively treat hyperactivity in children with ASD as well. But not as many children with ASD respond to treatment, and those who do have shown more side effects than children with ADHD and not ASD.

### 3. Brain Tumors

In some forms, the methods treat benign or malignant tumors by delaying or inhibiting the growth of a tumor in a subject, reducing the growth or size of the tumor, inhibiting, or reducing metastasis of the tumor, and/or inhibiting or reducing symptoms associated with tumor development or growth. In preferred forms, the tumors to be treated are neuronal and mixed neuronal-glial tumors. Neuronal and mixed neuronal-glial tumors are types of rare tumors that occur in the brain or spinal cord. In most cases the tumor is not cancerous (benign), but a tumor can press on nearby brain tissue and cause problems such as seizures.

Thus, in some forms, BBB-traversing polypeptide conjugate compositions are administered in combination with one or more additional therapeutically active agents, which are known to be capable of treating brain tumors or one or more symptoms associated therewith. For example, the BBB-traversing polypeptide conjugate compositions may be administered to the brain via intravenous administration or during surgery to remove all or a part of the tumor. The BBB-traversing polypeptide conjugate compositions may be used to deliver chemotherapeutic agents, agents to enhance adjunct therapy such as of a subject undergoing radiation therapy, wherein the BBB-traversing polypeptides are linked to at least one radiosensitizing agent, in an amount effective to suppress or inhibit the activity of DDX3 in the proliferative disease in the brain.

It will be understood by those of ordinary skill in the art, that in addition to chemotherapy, surgical intervention and radiation therapy are also used in treatment of cancers of the nervous system. Radiation therapy means administering ionizing radiation to the subject in proximity to the location of the cancer in the subject. In some forms, the radiosensitizing agent is administered in two or more doses and subsequently, ionizing radiation is administered to the subject in proximity to the location of the cancer in the subject. In further forms, the administration of the radiosensitizing agent followed by the ionizing radiation can be repeated for 2 or more cycles.Typically, the dose of ionizing radiation varies with the size and location of the tumor, but is dose is in the range of 0.1 Gy to about 30 Gy, preferably in a range of 5 Gy to about 25 Gy.

In some forms, the ionizing radiation is in the form of stereotactic ablative radiotherapy (SABR) or stereotactic body radiation therapy (SBRT).

### B. Methods of Diagnosis

It has been established that BBB-traversing polypeptide conjugate compositions including one or more diagnostic agents provide means for selective and specific labeling of one or more structures in the brain or CNS. In some forms, the BBB-traversing polypeptide conjugate includes a diagnostic agent that is a dye or a label specific for a certain cell type or condition. For example, in some forms, the diagnostic agent is a dye or label that selectively binds to and labels a tumor cell within the brain. Therefore, in some forms, methods of labeling a cell type or condition within the brain of a subject include administering to a subject an effective amount of a BBB-traversing polypeptide conjugate including a label or dye to label the cell type or condition in the brain of the subject. In some forms the methods include one or more steps of identifying or viewing the label or dye, for example, *via* one or more imaging techniques. In some forms the BBB-traversing polypeptide conjugate including diagnostic agents are administered in combination with one or more additional therapeutically active agents, for example, which are known to be capable of treating a brain disease or disorder or one or more symptoms associated therewith.

The methods of diagnosis can be coupled to a method of treatment, for example, the methods disclosed herein.

### C. Dosage and Effective Amounts

Dosage and dosing regimens are dependent on the severity and location of the disorder or injury and/or methods of administration, as well as the therapeutic or prophylactic agent being delivered. This can be determined by those skilled in the art. A therapeutically effective amount of the BBB-traversing polypeptide conjugate compositions used in the treatment of a proliferative disease or disorder in the brain is typically sufficient to reduce or alleviate one or more symptoms of brain cancer and/or proliferative disorder in the brain. Typically, doses would be in the range from microgram/kg up to about 100 mg/kg of body weight.

Preferably, the therapeutic, prophylactic or diagnostic agents do not target or otherwise modulate the activity or quantity of healthy cells not within or associated with the diseased/damaged tissue or do so at a reduced level compared to cells associated with a disease or disorder such as a cancer and/or proliferative disorder. In this way, byproducts and other side effects associated with the compositions are reduced. Therefore, in preferred forms, BBB traversing polypeptide conjugate compositions are administered in an amount that leads to an improvement, or enhancement, function in an individual with a disease or disorder, such as a cancer and/or proliferative disorder.

The actual effective amounts of BBB-traversing polypeptide conjugate compositions can vary according to factors including the specific agent administered, the particular composition formulated, the mode of administration, and the age, weight, condition of the subject being treated, as well as the route of administration and the disease or disorder. Generally, for intravenous injection or infusion, the dosage will be lower than for oral administration.

Dosage can vary and can be administered in one or more dose administrations daily, for one or several days. Guidance can be found in the literature for appropriate dosages for given classes of pharmaceutical products. Optimal dosing schedules can be calculated from measurements of drug accumulation in the body of the subject or patient. Persons of ordinary skill in the art can easily determine optimum dosages, dosing methodologies and repetition rates. Optimum dosages can vary depending on the relative potency of individual pharmaceutical compositions and can generally be estimated based on EC50s found to be effective in *in vitro* and *in vivo* animal models.

Dosage forms of the pharmaceutical composition including the BBB traversing polypeptide conjugate compositions are also provided. "Dosage form" refers to the physical form of a dose of a therapeutic compound, such as a capsule or vial, intended to be administered to a patient. The term "dosage unit" refers to the amount of the therapeutic compounds to be administered to a patient in a single dose.

In general, the timing and frequency of administration will be adjusted to balance the efficacy of a given treatment or diagnostic schedule with the side effects of the given delivery system. Exemplary dosing frequencies include continuous infusion, single and multiple administrations such as hourly, daily, weekly, monthly, or yearly dosing.

In some forms, dosages are administered daily, biweekly, weekly, every two weeks or less frequently in an amount to provide a therapeutically effective increase in the blood level of the therapeutic agent. Where the administration is by other than an oral route, the compositions may be delivered over a period of more than one hour, *e.g*., 3-10 hours, to produce a therapeutically effective dose within a 24-hour period. Alternatively, the compositions can be formulated for controlled release, wherein the composition is administered as a single dose that is repeated on a regimen of once a week, or less frequently.

It will be understood by those of ordinary skill that a dosing regimen can be any length of time sufficient to treat the disorder in the subject. In some forms, the regimen includes one or more cycles of a round of therapy followed by a drug holiday (*e.g*., no drug). The drug holiday can be 1, 2, 3, 4, 5, 6, or 7 days; or 1, 2, 3, 4 weeks, or 1, 2, 3, 4, 5, or 6 months.

In preferred forms, methods for treating or preventing one or more symptoms of an injury, a disorder, or a disease in the brain/CNS of a subject in need thereof include administering to the subject a formulation including BBB-traversing polypeptides covalently conjugated to, or complexed with or encapsulated with one or more therapeutic or prophylactic agents in an amount effective to treat or prevent one or more symptoms of the injury, disorder, or disease in the brain/CNS of the subject. It will be understood by those of ordinary skill that a dosing regimen will be for an amount and for a length of time sufficient to treat an injury, a disorder, or a disease in the brain/CNS to alleviate one or more symptoms such as swelling, pain, or seizures. Physicians routinely determine the length and amounts of therapy to be administered.

Typically, the BBB traversing polypeptide conjugates including the one or more therapeutic, prophylactic or diagnostic agents are administered systemically, and are transported across the blood-brain-barrier (BBB) to enter the brain and are selectively taken up by injured and/or diseased neurons. Typically, the BBB-traversing polypeptide conjugate compositions accumulate within nucleus of the neurons and deliver the therapeutic, prophylactic or diagnostic agents to these cells.

Typically, the conjugation of active agents with BBB-traversing polypeptides reduces the amount of the active agent that must be administered to a subject for treatment or diagnosis of a disease or disorder in the brain of the subject, as compared to the amount of the same active agent that must be administered in the absence of the BBB-traversing polypeptide. In some forms, the effective amount of therapeutic, prophylactic or diagnostic agent required for treatment or prevention of an injury, a disorder, or a disease in the brain/CNS when using BBB-traversing polypeptide conjugate compositions is up to one hundredth (100 times less) than the amount of the therapeutic, prophylactic or diagnostic agent alone, for example one quarter, one half, one fifth, one tenth, one twentieth, on thirtieth, one fortieth, one fiftieth, one sixtieth, one seventieth, one eightieth, one ninetieth, or one hundredth of the amount required when using the therapeutic, prophylactic or diagnostic agent alone.

### D. Combination Therapies and Procedures

The BBB-traversing polypeptide conjugate compositions can be administered alone or in combination with one or more conventional therapies. In some forms, the conventional therapy includes administration of one or more of the compositions in combination with one or more additional therapeutic, prophylactic or diagnostic agents. The combination therapies can include administration of the therapeutic, prophylactic or diagnostic agents together in the same admixture, or in separate admixtures. Therefore, in some forms, the pharmaceutical composition contains more than one therapeutic, prophylactic or diagnostic agent. Such formulations typically include an effective amount of an agent targeting the site of treatment. The additional therapeutic, prophylactic or diagnostic agent(s) can have the same or different mechanisms of action. In some forms, the combination results in an additive effect on the treatment of the disease or condition. In some forms, the combinations result in a more than additive effect on the treatment of the disease or disorder.

In some forms, the BBB-traversing polypeptide conjugate composition is administered prior to, in conjunction with, subsequent to, or in alternation with, treatment with one or more additional therapies or procedures. In some forms, the additional therapy is performed between drug cycles or during a drug holiday that is part of the composition dosage regime. For example, in some forms, the additional therapy or procedure is surgery, a radiation therapy, or chemotherapy. Examples of preferred additional therapeutic agents include other conventional therapies known in the art for treating the desired disease, disorder or condition.

In the context of Alzheimer's disease, the other therapeutic agents can include one or more of acetylcholinesterase inhibitors (such as tacrine, rivastigmine, galantamine or donepezil), beta-secretase inhibitors such as JNJ-54861911, antibodies such as aducanumab, agonists for the 5-HT2A receptor such as pimavanserin, sargramostim, AADvac1, CAD106, CNP520, gantenerumab, solanezumab, and memantine.

In the context of Dementia with Lewy Bodies, the other therapeutic agents can include one or more of acetylcholinesterase inhibitors such as tacrine, rivastigmine, galantamine or donepezil; the N-methyl d-aspartate receptor antagonist memantine; dopaminergic therapy, for example, levodopa or selegiline; antipsychotics such as olanzapine or clozapine; REM disorder therapies such as clonazepam, melatonin, or quetiapine; anti-depression and antianxiety therapies such as selective serotonin reuptake inhibitors (citalopram, escitalopram, sertraline, paroxetine, *etc*.) or serotonin and noradrenaline reuptake inhibitors (venlafaxine, mirtazapine, and bupropion) (see, *e.g*., Macijauskiene, et al., Medicina (Kaunas), 48(1):1-8 (2012)). Exemplary neuroprotective agents are also known in the art in include, for example, glutamate antagonists, antioxidants, and NMDA receptor stimulants. Other neuroprotective agents and treatments include caspase inhibitors, trophic factors, anti-protein aggregation agents, therapeutic hypothermia, and erythropoietin.

Other common therapeutic, prophylactic or diagnostic agents for treating neurological dysfunction include amantadine and anticholinergics for treating motor symptoms, clozapine for treating psychosis, cholinesterase inhibitors for treating dementia, and modafinil for treating daytime sleepiness.

In the context of cancer treatment, the other therapies include one or more of conventional chemotherapy, inhibition of checkpoint proteins, adoptive T cell therapy, radiation therapy, and surgical removal of tumors.

The composition and the additional therapeutic agent or treatment can be administered to the subject together or separately. The composition and the additional therapeutic agent or treatment can be administered on the same day, on different days, or combinations thereof.

For example, the subject can be administered a disclosed composition 0, 1, 2, 3, 4, 5, or more days before administration of or exposure to the additional therapeutic agent or treatment. In some forms, the subject can be administered one or more doses of the composition every 1, 2, 3, 4, 5, 6 7, 14, 21, 28, 35, or 48 days prior to a first administration of or exposure to the additional therapeutic agent or treatment.

The subject can also be administered the composition for 0, 1, 2, 3, 4, 5, or more days after administration of or exposure to the additional therapeutic agent or treatment. The subject can also be administered the composition during administration of or exposure to the additional therapeutic agent or treatment. The subject can be administered one or more doses of the composition every 1, 2, 3, 4, 5, 6, 7, 14, 21, 28, 35, or 48 days during or after administration of the additional therapeutic agent or treatment.

### 1. Methods of Administration

The described methods administer BBB-traversing polypeptide conjugate composition compositions prophylactically, therapeutically, or for a diagnostic/investigative method, or combinations thereof. Therefore, the composition can be administered during a period before, during, or after onset of a disease or disorder, or one or more symptoms of a pathology, disorder, or disorder associated therewith. In some forms, the composition is administered with one or more additional therapeutic agents as part of a co-therapy (*e.g*., a combination therapy including a BBB traversing polypeptide conjugate composition and one or more other therapeutic agents), one or more second treatments (*e.g*., an exercise regime, surgery, *etc*.,), or combinations thereof.

Exemplary methods of administration include systemic or local administration to the subject, or by direct administration to cells. The compositions can be administered to a cell or subject, as is generally known in the art for protein therapies and diagnostics. Exemplary forms of administration include systemic administration, for example, via injection, such as intravenous (i.v.), intramuscular (i.m.), intradermal (i.d.), subcutaneous (s.c.), intravitreal, intraarticular, intraperitoneal, intraocular and intrathecal injection. Other forms of administration include oral administration, mucosal administration and trans-dermal administration.

### E. Controls

The therapeutic result of the BBB-traversing polypeptide conjugate compositions including one or more therapeutic, prophylactic, or diagnostic agents can be compared to a control. Suitable controls are known in the art and include, for example, an untreated subject, or a placebo-treated subject. A typical control is a comparison of a condition or symptom of a subject prior to and after administration of BBB-traversing polypeptide conjugate. The condition or symptom can be a biochemical, molecular, physiological, or pathological readout. For example, the effect of the composition on a particular symptom, pharmacologic, or physiologic indicator can be compared to an untreated subject, or the condition of the subject prior to treatment. In some forms, the symptom, pharmacologic, or physiologic indicator is measured in a subject prior to treatment, and again one or more times after treatment is initiated. In some forms, the control is a reference level, or average determined based on measuring the symptom, pharmacologic, or physiologic indicator in one or more subjects that do not have the disease or condition to be treated (*e.g*., healthy subjects). In some forms, the effect of the treatment is compared to a conventional treatment that is known the art. In some forms, an untreated control subject suffers from the same disease or condition as the treated subject.

In some forms, a control includes an equivalent amount of therapeutic, prophylactic or diagnostic agent delivered alone, or bound to a carrier without BBB-traversing polypeptide conjugates.

### V. Kits

The compositions can be packaged in kit. The kit can include a single dose or a plurality of doses of a composition including one or more therapeutic, prophylactic, or diagnostic agents, encapsulated in, associated with, or conjugated to a BBB traversing polypeptide conjugates (*e.g*., one or more BBB traversing polypeptides as described in the Examples), and instructions for administering the compositions. Specifically, the instructions direct that an effective amount of the BBB-traversing polypeptide and therapeutic, prophylactic or diagnostic agents be administered to an individual with a particular disease/disorder as indicated. The composition can be formulated as described above with reference to a particular treatment method and can be packaged in any convenient manner.

The invention will be better understood by reference to the following paragraphs:
1. A brain penetrating peptide that is capable of traversing the blood brain barrier (BBB) of a subject *in vivo*, including a BBB-traversing domain including the amino acid sequence RRVISRAKLAAAL (SEQ ID NO:1), or a functional variant thereof.
2. A brain penetrating peptide that is capable of traversing the blood brain barrier (BBB) of a subject *in vivo*, including a BBB-traversing domain including the amino acid sequence of CVGTNCY (SEQ ID NO:2), or a functional variant thereof.
3. The brain penetrating peptide of paragraph 1, wherein the BBB-traversing domain is at least 13 amino acids in length.
4. The brain penetrating peptide of paragraph 1 or 3, wherein the BBB-traversing domain includes between 5-20 contiguous amino acids of one or more of SEQ ID NOs:3-10, or a functional variant thereof.
5. The brain penetrating peptide of paragraph 1 or 3 or 4, wherein the BBB-traversing domain includes a functional variant of any one of SEQ ID NOs:1, or 3-10, having an amino acid sequence at least 70%, 75%, 80%, 85%, 90%, 95%, or 99% identical to any one of SEQ ID NOs:1 or 3-10.
6. The brain penetrating peptide of paragraph 2, wherein the BBB-traversing domain is at least 7 amino acids in length.
7. The brain penetrating peptide of paragraph 2 or 6, wherein the BBB-traversing domain includes between 5-10 contiguous amino acids of one or more of SEQ ID NOs:11-20, 22-23, or a functional variant thereof.
8. The brain penetrating peptide of any one of paragraphs 1-7, wherein the amino acid sequence of the BBB-traversing domain is not CAGALCY (SEQ ID NO:21).
9. The brain penetrating peptide of any one of paragraphs 1-8, wherein the brain penetrating peptide selectively homes to the brain parenchyma, endothelial cell, or the whole brain.
10. The brain penetrating peptide of paragraph 9, wherein the BBB-traversing domain is or includes SRRVISRAKLAAAL (SEQ ID NO:9); SRRVISRAKLAAALE (SEQ ID NO:3); or MLGDPILASRRVISRAKLAAALE (SEQ ID NO:4), and
   wherein the brain penetrating peptide selectively homes to the brain parenchyma cells.
11. A peptide conjugate including
   (a) the brain penetrating peptide of any one of paragraphs 1-10; and
   (b) a cargo molecule,

   wherein the cargo molecule is directly or indirectly conjugated to or complexed with the brain penetrating peptide, and
   wherein the cargo molecule does not traverse the BBB in the absence of the brain penetrating peptide.
12. The peptide conjugate of paragraph 11, wherein the cargo molecule includes one or more active agent selected from the group including a therapeutic agent, a diagnostic agent, a prophylactic agent and a nutraceutical agent.
13. The peptide conjugate of paragraph 11 or 12, wherein the cargo molecule is encapsulated within or conjugated to a carrier, optionally wherein the carrier is conjugated to the brain penetrating peptide.
14. The peptide conjugate of paragraph 13, wherein the carrier includes a polymeric particle, a lipid particle, a liposome, a gel, an inorganic particle, a viral particle, a nucleic acid nanostructure, and a virus-like particle.
15. The peptide conjugate of paragraph 13, wherein the carrier is conjugated to the brain penetrating peptide by one or more linkers,
   optionally wherein the one or more linkers are cleavable linkers.
16. The peptide conjugate of any one of paragraphs 12-15, wherein the active agent is a therapeutic agent selected from the group including a nucleic acid, a peptide, a lipid, a glycolipid, a glycoprotein, and a small molecule.
17. The peptide conjugate of paragraph 16, wherein the therapeutic agent is a nucleic acid selected from the group including antisense molecules, aptamers, ribozymes, triplex forming oligonucleotides, external guide sequences, RNAi, CRISPR/Cas, zinc finger nucleases, and transcription activator-like effector nucleases (TALENs).
18. The peptide conjugate of paragraph 16, wherein the therapeutic agent is a small molecule.
19. The peptide conjugate of any one of paragraphs 16-18, wherein the therapeutic agent is selected from the group including an anti-cancer agent, an anti-inflammatory agent, and an antimicrobial agent.
20. A pharmaceutical composition including the peptide conjugate of any one of paragraphs 16-19, and a pharmaceutically acceptable excipient for administration.
21. The pharmaceutical composition of paragraph 20, wherein the composition is suitable for mucosal, pulmonary, intravenous, or intramuscular delivery.
22. A method of treating or preventing one or more symptoms of a disease or disorder in the brain or CNS of a subject including
   administering to the subject an effective amount of a pharmaceutical composition of paragraph 20 or 21 to prevent or reduce one or more symptoms of a disease or disorder in the brain or CNS of a subject in the subject.
23. The peptide conjugate of any one of paragraphs 12-15, wherein the active agent is a diagnostic agent selected from the group including a dye, a radionuclide, a fluorescent tag, a magnetic tag, and a nanoparticle.
24. A pharmaceutical composition including the peptide conjugate of paragraph 23 and a pharmaceutically acceptable excipient for administration.
25. The pharmaceutical composition of paragraph 24, wherein the composition is suitable for mucosal, pulmonary, intravenous, or intramuscular delivery.
26. A formulation of the peptide conjugate of any one of paragraphs 11-19 for treating or preventing one or more symptoms of a disease or disorder in the brain or CNS of a subject, or a pharmaceutical composition thereof for use in treating or preventing one or more symptoms of a disease or disorder in the brain or CNS of a subject are also provided.
27. Use of the peptide conjugate of any one of paragraphs 11-19 in the manufacture of a medicament for treating or preventing one or more symptoms of a disease or disorder in the brain or CNS of a subject, wherein the conjugates including one or more therapeutic agents.
28. A method of detecting or monitoring a disease or disorder in the brain or CNS of a subject including
   administering to the subject an effective amount of a pharmaceutical composition of paragraph 24 or 25 to detect or monitor a disease or disorder in the brain or CNS of a subject in the subject.
29. The method of any one of paragraphs 22 or 28, wherein the subject is a human.
30. The method of any one of paragraphs 22 or 28 or 29, wherein the subject has, or is suspected as having a disease selected from the group including cancer, an inflammatory disease, a neuronal disorder, HIV/AIDS, a diabetes, a cardiovascular disease, an infectious disease (including a viral, a protozoan, a bacterial disease, and an allergy), an autoimmune disease and an autoimmune disease, Alzheimer's disease, Parkinson's disease, ischemia, a neurodegenerative disorder, and a genetic disorder.
31. The method of paragraph 30, wherein the disease is a cancer.
32. The method of paragraph 30, wherein the disease is Alzheimer's disease.
33. The method of paragraph 30, wherein the disease is Parkinson's disease.
34. The method of paragraph 30, wherein the disease is a neurodegenerative disorder.
35. The method of any one of paragraphs 22 or 28-34, wherein the subject has an infection.
36. A brain penetrating peptide conjugate that is capable of traversing the blood brain barrier (BBB) of a subject *in vivo*, including
   (a) a BBB-traversing domain including the amino acid sequence RRVISRAKLAAAL (SEQ ID NO:1), or a functional variant thereof; and
   (b) an active agent selected from the group including a therapeutic agent, a diagnostic agent and a prophylactic agent;
   wherein the active agent is conjugated with the BBB-traversing domain, optionally wherein the active agent is conjugated via a linker.
37. A brain penetrating peptide conjugate that is capable of traversing the blood brain barrier (BBB) of a subject *in vivo*, including
   (a) a BBB-traversing domain including the amino acid sequence CVGTNCY (SEQ ID NO:2); and
   (b) an active agent selected from the group including a therapeutic agent, a diagnostic agent and a prophylactic agent;
   wherein the active agent is conjugated with the BBB-traversing domain, optionally wherein the active agent is conjugated via a linker.
38. A brain penetrating peptide conjugate that is capable of traversing the blood brain barrier (BBB) of a subject *in vivo*, comprising
   (a) a BBB-traversing domain comprising the amino acid sequence RRVISRAKLAAAL (SEQ ID NO:1), or a functional variant thereof; and
   (b) an active agent selected from the group consisting of a therapeutic agent, a diagnostic agent and a prophylactic agent;
   wherein the active agent is conjugated with the BBB-traversing domain, optionally wherein the active agent is conjugated *via* a linker.
39. A brain penetrating peptide conjugate that is capable of traversing the blood brain barrier (BBB) of a subject *in vivo*, comprising
   (a) a BBB-traversing domain comprising the amino acid sequence CVGTNCY (SEQ ID NO:2); and
   (b) an active agent selected from the group consisting of a therapeutic agent, a diagnostic agent and a prophylactic agent;
   wherein the active agent is conjugated with the BBB-traversing domain, optionally wherein the active agent is conjugated *via* a linker.
40. A brain penetrating peptide conjugate that is capable of traversing the blood brain barrier (BBB) of a subject *in vivo*, comprising
   (a) a BBB-traversing domain comprising the amino acid sequence of any one of (SEQ ID NO:77-96); and
   (b) an active agent selected from the group consisting of a therapeutic agent, a diagnostic agent and a prophylactic agent;
   wherein the active agent is conjugated with the BBB-traversing domain, optionally wherein the active agent is conjugated *via* a linker.
41. A method of delivering an active agent selectively to one or more structures of the brain of a subject, comprising administering to the subject the composition of any one of paragraphs 38-40,
   wherein the composition is administered to the subject *via* a systemic route.
42. The method of paragraph 41, wherein the active agent comprises a therapeutic and/or prophylactic agent in an amount effective to treat or prevent one or more symptoms of a disease or disorder in the subject.
43. The method of paragraph 42, wherein the therapeutic and/or prophylactic agent comprises one or more of a protein, a carbohydrate, a lipid, a small molecule or a nucleic acid.
44. The method of paragraph 43, wherein the protein comprises an immunoglobulin, or an antigen-binding fragment thereof.
45. The method of any one of paragraphs 42-44, wherein the therapeutic and/or prophylactic agent does not traverse the BBB in the absence of the brain penetrating peptide.
46. The method of any one of paragraphs 42-45, wherein the one or more structures of the brain is selected from the group consisting of the cortex, the hippocampus and the brain stem.
47. The method of any one of paragraphs 42-46, wherein the effective amount of the therapeutic and/or prophylactic when administered to the subject as the conjugate with the BBB-traversing domain is less than the effective amount of the same therapeutic and/or prophylactic agent in the absence of the BBB-traversing domain.
48. The method of any one of paragraphs 42-47, wherein the BBB-traversing domain comprises the amino acid sequence RRVISRAKLAAAL (SEQ ID NO:1), and
   wherein the active agent is selectively delivered to one or more of astrocytes, microglia or mature neurons in the brain of the subject.
49. The method of any one of paragraphs 42-47, wherein the BBB-traversing domain comprises the amino acid sequence CVGTNCY (SEQ ID NO:2); and
   wherein the active agent is selectively delivered to activated microglia in the brain of the subject.
50. The method of paragraph 49, wherein the subject has cancer, and
   wherein the active agent is selectively delivered to the region of a tumor and/or brain damage within the brain of the subject.
52. The method of paragraph 49 or 50, wherein the tumor is a glioblastoma.
53. The method of any one of paragraphs 49-52, wherein the active agent is a chemotherapeutic agent.

The present invention will be further understood by reference to the following non-limiting examples.

### Examples

### Example 1. Identification and Development of Brain Penetrating Peptide Cerepep Methods

### Animal procedures

The animal procedures were approved by the Committee of Animal Experimentation, Estonian Ministry of Agriculture in accordance with Estonian legislation and Directive 2010/63/EU of the European Parliament and of the Council of 22 September 2010 on the protection of animals used for scientific purposes (permits #48 and #159).

### In vivo phage display

The *in vivo* phage screening in male Balb/C mice was performed as described in (Põšnograjeva et al., 2022) with some alterations. To reduce the background of unspecific phages in the brain, 4.5×10¹⁰ pfu of UV-inactivated control insertless T7 phage was injected *i.p*.. 30 min later, CX7C naïve T7 phage library or the phage pool recovered from the brain in the previous round of biopanning was injected *i.v*. and allowed to circulate for 30 min. The mice were anesthetized and perfused with PBS intracardially. The brain was removed, homogenized in LB-NP40 (1%) and the phages in the lysate were rescued by amplification in semi-solid media in *E.coli* strain BLT5403. Peptide-encoding portion of the phage genome was sequenced using Ion Torrent high throughput sequencing (HTS). 8 mice were used in the first round of biopanning (R1) to prevent creating a narrow bottleneck of selection in the first round of biopanning. 2 mice were used for all subsequent rounds (R2...R5). R5 was repeated with the additional step of isolating brain CD31+ cells from CD31- cells by magnetic-activated cell sorting. The mouse brains were dissociated using Dounce tissue grinder and Adult Brain Dissociation Kit (# 130-107-677) and CD31+ cells were isolated from all the other brain cells using CD31 MicroBeads (# 130-097-418) following the manufacturer's protocol for isolation and cultivation of endothelial cells from adult mouse brain. The cells were lysed in LB-NP40 (1%) and the samples were further processed following the same steps with samples of whole brain.

### Phage biodistribution studies

For titering-based biodistribution studies, nucleotide sequences coding for peptides of interest were cloned into T7 phage genomic DNA to be expressed on the phage surface as C-terminal fusions to capsid protein. The cloning was performed using complementary oligonucleotides, the phage clones were amplified, and purified as described in (Põšnograjeva, *et al.,* 2022). 5×10⁹ pfu of peptide-phage clone was injected *i.v*. into male Balb/C mice or 5×10¹⁰ pfu of phage was injected into female Sprague-Dawley rats (n = 3). 60 min later, the animals were anesthetized and perfused with PBS. The tissues were collected, homogenized, and the amount of phage in tissue lysates was determined by titering using *E*. *coli* strain BLT5615.

### Laser ablation ICP-MS-based AgNP biodistribution studies

Isotopically pure ¹⁰⁷AgNPs and ¹⁰⁹AgNPs were prepared as described in (Braun et al., 2014; Toome et al., 2017; Pleiko et al., 2021) and functionalized with biotinylated "Cerepep" peptide (Biotin-Ahx-SRRVISRAKLAAAL-OH (SEQ ID NO:9), where Ahx - aminohexanoic acid), Angiopep-2 peptide (Biotin-Ahx-LGDPNSTFFYGGSRGKRNNFKTEEY-OH (SEQ ID NO:75)) or biotin (biotin-¹⁰⁷AgNPs). ¹⁰⁷AgNPs and ¹⁰⁹AgNPs were mixed at 1:1 ratio and injected *i.v*. in Balb/C mice. Following 3 h circulation time, the mice were intracardially perfused PBS. The brain and liver were collected, frozen in OCT, sectioned at 30 µm on Superfrost+ slides, and dried in a vacuum desiccator.

Laser ablation inductively coupled plasma mass spectrometry (LA-ICP-MS) analysis of the tissue samples was performed using Agilent 8800 ICP-MS/MS coupled to a Cetac LSX-213 G2+ laser ablation unit equipped with HelEx II ablation cell and connected using ARIS (Aerosol Rapid Introduction System) sample introduction system. The system was tuned using NIST 610 glass. Ablation was performed as line scans using 65 µm square spot, scan speed of 260 µm/s, 20 Hz and fluence of 13.5 J/cm². Five parallel ablations with 65 µm spacing were performed on each tissue. ICP-MS was operated in single quad mode. Data collection was performed in TRA mode with dwell times of 9.5 ms on mass 13C and 14 ms on mass ¹⁰⁷Ag and ¹⁰⁹Ag corresponding to a total duty cycle of 50 ms. Data reduction was performed using Iolite v3.62. Median with MAD error 2 SD outlier reject was used for data selection. Multiple parallel line raster scans were performed to generate distribution maps of the whole brain sections and a part of the liver. The raster lines were directly adjacent to each other (with 65 µm offset).

### In vitro phage binding studies

To assess the binding of Cerepep phage, the cells were cultured in 24-well plates on polylysine coated glass coverslips until 80-90% confluency. The cells were incubated with Cerepep or control insertless peptide-phage at a final concentration of 5×10⁷ pfu in 1 ml of DMEM+BSA (1%) at 4°C for 1h on a rocking platform. The wells were washed 4 times with 1 ml of DMEM+BSA (1%), the cells were lysed in 1 ml LB+NP40 and the amount of phage bound to the cells was determined by titering.

### In vitro peptide binding and uptake studies

To assess the binding and uptake of Cerepep peptide, Neuro2A were cultured in 24-well plates on polylysine coated glass coverslips until 80-90% confluency. FAM-Cerepep (FAM-Ahx-SRRVISRAKLAAAL-OH (SEQ ID NO:9)) or control FAM-RPAR (FAM-RPARPAR-OH (SEQ ID NO:74)) peptide with the final concentration of 30 µM was added to Neuro2A cells in growth media and incubated at 4°C (to study the binding) or 37°C (to study the uptake) for 1h. The cells were washed 4 times with growth media and fixed in methanol. The samples were stained with anti-fluorescein antibody to enchance the FAM signal. The fixed cells were first blocked with PBST (PBS+ 0.05% Tween 20), 5% BSA, 5% goat serum at RT for 30 min, followed by incubation with rabbit anti-fluorescein IgG (cat. no. A889, Thermo Fisher Scientific, MA, USA) at 4°C for O/N and Alexa 647 goat anti-rabbit IgG secondary antibody at RT for 1h. Nuclei were counterstained with DAPI at 1 µg/ml. The coverslips were mounted on glass slides with Fluoromount-G (Electron Microscopy Sciences, PA, USA), imaged using confocal microscopy (Olympus FV1200MPE, Tokyo, Japan) and analyzed using the FV10-ASW4.2 software.

### In vitro AgNP binding and internalization studies

To assess the binding and uptake of silver nanoparticles functionalized with Cerepep, Neuro2A were cultured in 24-well plates on polylysine coated glass coverslips until 80-90% confluency. Biotinylated Cerepep peptide or biotin as a non-targeted control and CF555 dye were conjugated to neutravidin-AgNPs prepared as described previously (Tobi et al., 2021). Some samples were pre-incubated with 100 µM Cerepep peptide (Ac-SRRVISRAKLAAAL-OH (SEQ ID NO:9), where Ac - acetylation) at 37°C for 1h to assess whether the binding and internalization of particles is peptide-dependant. 10 µl (OD=100) of CP-AgNPs or control biotin-AgNPs were added to cells in 500 µl of media and incubated at 37°C for 1h. In some samples, the cell surface-bound AgNPs were removed with a freshly prepared etching solution containing 10 mM of Na2S2O3 and K3Fe(CN)6 in PBS, which was applied for 3 min. All the samples were washed with DPBS to remove unbound AgNPs. The cells were fixed in methanol, stained with DAPI and analyzed with confocal microscopy as described above.

To study the colocalization of CP-AgNPs with markers of endocytosis, Neuro2A cells were incubated with CP-AgNPs for 60 or 180 min and the samples were treated with an etching solution, fixed in methanol, blocked, stained with mouse anti-Rab5a, rat anti-Rab7a or rabbit anti-TGN46 IgG primary antibodies and Alexa 647 goat anti-mouse, anti-rat or anti-rabbit IgG secondary antibodies, respectively. Nuclei were stained with DAPI and the samples were analyzed with confocal microscopy.

For characterization of the CP-AgNP uptake pathway, the Neuro2A cells were cultured in 6-well plates until 80-90% confluency and pre-incubated at 37°C for 30 min with the following endocytosis inhibitors added to growth media: 50 µM Nystatin, 50 µM 5-(N-ethyl-N-isopropyl)-Amiloride (EIPA), 4 µM Cytochalasin D, and 30 µM Chlorpromazine. The CP-AgNPs or control biotin-AgNPs were then added, and the cells were incubated at 37°C for 1h, treated with an etching solution to remove accessible surface-bound AgNPs and subsequently washed with DPBS. The fluorescence of the labeled AgNPs was detected using the BD Accuri flow cytometer by monitoring the 555 nm channel FL2 and analyzed using FCS Express 7 software.

### Proximity labelling

To assess the efficiency and specificity of proximity labelling, WT GBM cells were cultured in 24-well plates on polylysine coated glass coverslips until 80-90% confluency. Cerepep and horseradish peroxidase complex (CP-HRP) was prepared by conjugating biotinylated Cerepep to streptavidin-HRP. Control biotin-HRP complex was prepared by conjugating biotin to streptavidin-HRP. 4 µM CP-HRP or biot-HRP in DPBS was added to the cells and incubated at 37°C for 1 h. The cells were washed with DPBS. 500 µM biotin-phenol (Iris Biotech #41994-02-9) in DPBS was added to the cells and incubated at 37°C for 30 min. To start the reaction, H2O2 with the final concentration of 1 mM was added to the cells and and incubated for 2 min at RT. The reaction was stopped by washing with 100 U/ml catalase in DPBS. Cells were fixed in 4% PFA and biotinylated proteins were detected with Streptavidin-Dylight 550 conjugate (1:500 in DPBS). Nuclei were stained with DAPI and the biotinylation of cells was analyzed with confocal microscopy.

To biotinylate the receptor of Cerepep peptide to be detected with mass spectrometry, the proximity labelling was performed on WT GBM and Neuro2A cells in suspension following the same protocol as with attached cells. End-over-end rotation was used for all the incubations with cells in suspension and for the washes, the cells were centrifuged at 300×g, RT for 5 min. 3x10⁶ of cells were used per sample. The biotinylation of the cells was confirmed by adding Streptavidin-Dylight 550 conjugate and detecting the signal using the BD Accuri flow cytometer by monitoring the 555 nm channel FL2.

### In vivo play-off

To determine which amino acid positions are important for brain homing of Cerepep, *in vivo* play-off method was used. A pool of T7 peptide-phages was created, where each amino acid of Cerepep sequence was changed one-by-by to alanine and alanines were changed to leucines. These clones, original Cerepep peptide-phage clone and control insertless phage were mixed in equimolar ratio and 5×10⁹ pfu of phage mix was injected *i.v*. into male Balb/C mice (n =2). Following 1h circulation the animals were anesthetized and perfused with PBS. The prevalence of phage clones in the brain was determined by high throughput sequencing.

### Statistical analysis

Prism 8 software was used to perform statistical analyses. The results are presented as mean with error bars indicating ±SD. For comparison of groups, One-Way ANOVA test was used. P-values were considered as follows: *p ≤ 0.05, **p ≤ 0.01, ***p ≤ 0.001 and p****≤0.0001.

### Results

### Identification of blood-brain barrier penetrating peptide Cerepep using in vivo phage display

To identify peptides that accumulate in mouse brain, cross the blood-brain barrier and reach the brain parenchyma cells, *in vivo* T7 phage display was used. For round 1 of biopanning, naïve CX7C T7 peptide-phage library was injected into mice *i.v*. The phages accumulated in brain were rescued to be used as input in a subsequent round of biopanning. A consistent increase in the % of injected phage accumulating in the brain tissue through rounds 1...5 of biopanning was observed (**Fig. 1A**; To assess the % of injected phage that accumulated in the brain throughout the five rounds of biopanning (R1-R5), Peptide-phages were injected *i.v*. and following 30 min circulation mice were perfused with PBS. The phages rescued from the brain were reamplified and injected for a subsequent round of biopanning). The 5^{th} round of bio-panning was then repeated with an additional step of isolating CD31+ brain endothelial cells from CD31- brain cells to detect peptides that not only accumulate in the brain, but also penetrate the endothelial cell layer lining the brain blood vessels (**Fig. 1B**; The total amount of phage rescued from mouse brain CD31+ and CD31- cell fractions after R5 with the additional step of isolating brain endothelial (CD31+) cells from all the other brain cells (parenchyma cells; CD31-)). The peptides displayed on the surface of the phages rescued from the brain in rounds 1-5 and from the brain endothelial or parenchyma cell fractions were identified using Ion-Torrent HTS.

One peptide - Pep 1 - was the most represented peptide sequence in the whole brain after round 5 of bio-panning as well as in CD31+ and CD31- brain cell fractions (**Fig. 1C**; The prevalence of Pep1 sequence in high throughput sequencing data, shown as a percentage of all reads). Pep1 is not a CX7C peptide, instead it was created through a frame shift mutation resulting in a longer peptide displayed on the surface of the phage (**Table 1A**). A complete listing of peptides that were identified from Ion Torrent high throughput sequencing from bio-panning Round 5 is set forth in **Table 1B.** Therefore, to determine the shortest sequence necessary for efficient brain homing, shorter versions of Pep1 were displayed on the surface of the phage (**Table 1A**) and tested their accumulation in the brain (**Fig. 1D**; CP is the shortest fragment of Pep1 peptide that retains a high accumulation in the brain. 5×10⁹ pfu of phage was injected *i.v*. and following 1 h circulation mice were perfused with PBS. The amount of phage accumulated in the brain and remaining in the blood was determined by titering). The shortest peptide still achieving a high accumulation in the brain was determined to be 14 aa long peptide SRRVISRAKLAAAL (SEQ ID NO:9), which was termed "Cerepep".

**Table 1A: The sequence of Peptides , including Pep 1 (the most prevalent peptide sequence displayed on the surface of phage rescued from brain after R5 from the whole brain,CD31- and CD31+ cell fractions) and shorter versions of the peptide tested in vivo.**

| |
|---|
| **Pep1**- M L G D P - - - - - - I L A **S R R V I S R A K L A A A L** E (SEQ ID NO:4) |
| **BP1**- M L G D P N S G G **- - - - - S R R V I S R A K L A A A L -** (SEQ ID NO:5) |
| **Pep17-** M L G D P N S G G S P I L A S R R V I S R A K L A A A **- -** (SEQ ID NO:65) |
| **Pep13**- M L G D P N S G G **- - - - -** S - R V I S R A K L A A A L E (SEQ ID NO:7) |

**Table 1B: The sequence of Peptides identified in Ion torrent sequencing following Round 5 of biopanning, arranged accoring to the numbers of reads associated with each sequence.**

| | | | |
|---|---|---|---|
| total reads in sample | 218048 | 135061 | |
| **Round 5 mice →** | **326** | **325** | |
| MLGDPILASRRVISRAKLAAALE | 132268 | 80229 | (SEQ ID NO:4) |
| MLGDPNLARRRVISRAKLAAALE | 8755 | 4716 | (SEQ ID NO:99) |
| MLGDPILASRRVISRAKLAAAL | 2334 | 1495 | (SEQ ID NO:36) |
| MLGDPNSCTEGTRVTC | 2339 | 1533 | (SEQ ID NO:60) |
| MLGDPNSSS | 2197 | 237 | (SEQ ID NO:61) |
| MLGDPNFAIRRVISRAKLAAALE | 825 | 543 | (SEQ ID NO:37) |
| MLGDPNSSSVDKLAAALE | 588 | 674 | (SEQ ID NO:38) |
| MLGDPILAGRRIVSRAKLAAALE | 480 | 315 | (SEQ ID NO:39) |
| MLGDPILASRRVISRAKLAAA | 399 | 350 | (SEQ ID NO:40) |
| MLGDPNIVAKLAAALE | 368 | 271 | (SEQ ID NO:41) |
| MLGDPILASRRVISRAKLADALE | 359 | 142 | (SEQ ID NO:42) |
| MLGDPILASRRVISRAKLAAVLE | 292 | 51 | (SEQ ID NO:43) |
| MLGDPILASRRVISRAKLAA | 286 | 254 | (SEQ ID NO:44) |
| MLGDPILASRRVISRAKLAAAFE | 269 | 46 | (SEQ ID NO:45) |
| MLGDPILASRRVISRAKLEAALE | 251 | 129 | (SEQ ID NO:46) |
| MLGDPNSC | 216 | 84 | (SEQ ID NO:47) |
| MLGDPILASRRVISRAKLAAALS | 194 | 54 | (SEQ ID NO:48) |
| MLGDPNLARRRVISRAKLAAAL | 188 | 89 | (SEQ ID NO:49) |
| MLGDPILASRRVISRAKLA | 172 | 150 | (SEQ ID NO:50) |
| M LGDPILASRRVISRAKLAAASS | 169 | 47 | (SEQ ID NO:51) |
| MLGDPILASRRVISRAKLAATLE | 143 | 39 | (SEQ ID NO:52) |
| MLGDPILASRRVISRAKLAAHSS | 142 | 84 | (SEQ ID NO:53) |
| MLGDPILASRRVISRAKLAAALD | 119 | 34 | (SEQ ID NO:54) |
| MLGDPILASRRVISRAKL | 116 | 68 | (SEQ ID NO:55) |
| MLGDPILASRRVISRAKLAAALG | 108 | 33 | (SEQ ID NO:56) |
| MLGDPILASRRVISRAK | 100 | 67 | (SEQ ID NO:57) |
| MLGDPNS | 84 | 57 | (SEQ ID NO:58) |
| MLGDPILASRRVISR | 76 | 77 | (SEQ ID NO:59) |

### Cerepep phage achieves high and selective accumulation in mouse and rat brain

To study brain accumulation and biodistribution of SRRVISRAKLAAAL (SEQ ID NO:5) peptide in mice and rats, it was displayed on the surface of T7 phage. The peptide-phage was injected *i*.*v*. and following 1h circulation, the mice were perfused, brain and control organs were collected and phage titer in each tissue was determined. Insertless T7 phage was used as a negative control and a phage displaying BBB penetrating peptide Angiopep-2 (TFFYGGSRGKRNNFKTEEY; SEQ ID NO:97) was used for comparison. Cerepep phage achieved approximately 300-fold higher accumulation in mouse brain compared to control and Angiopep-2 phages (**Fig. 2A**; Peptide-phage accumulation in the brain. 5×10⁹ pfu of phage was injected *i.v*. and following 1 h circulation mice were perfused with PBS. The amount of phage accumulated in the brain was determined by titering). To assess the brain distribution of SRRVISRAKLAAAL (SEQ ID NO:9), the mouse brain was dissected to separate the olfactory bulbs, cerebellum, brain stem and the hemispheres before determining the titer in the tissue. Compared to control phage, SRRVISRAKLAAAL (SEQ ID NO:9) had a high accumulation in all tested brain regions as well as in the spinal cord (**Fig. 2B**; peptide-phage distribution in different regions of central nervous system. 5×10⁹ pfu of CP or insertless control phage was injected *i.v*. and following 1 h circulation mice were perfused with PBS. The amount of phage accumulated in the different regions of central nervous system, liver and blood was determined by tittering.).

The brain selectivity of SRRVISRAKLAAAL (SEQ ID NO:9) phage following a systemic administration was studied in mice and rats by determining the titer of SRRVISRAKLAAAL (SEQ ID NO:9) and control phage in multiple control tissues. In both mice and rats, SRRVISRAKLAAAL (SEQ ID NO:9) showed the highest accumulation in the brain and low accumulation in all other tested tissues (**Fig. 2C-2D**; CP peptide-phage biodistribution in male mice and female rats - CP or insertless control phage was injected *i.v*. and following 1 h circulation mice were perfused with PBS. The amount of phage accumulated in the tissues was determined by titering.)

### Cerepep-functionalized silver nanoparticles home to mouse brain

Peptide-coated isotopically barcoded AgNPs were used to assess whether Cerepep (SRRVISRAKLAAAL (SEQ ID NO:9)) can be used to increase the brain homing of synthetic nanoparticles. Biotinylated Cerepep was conjugated to neutravidin-109AgNPs (CP109AgNPs), biotin-blocked neutravidin-107AgNPs (biot107AgNPs) were used as a control and neutravidin-107AgNPs conjugated to biotinylated Angiopep-2 (Ang107AgNPs) were used for comparison. Equimolar mixture of 107/109AgNPs was injected *i.v*. in mice; after 3 h circulation, animals were sacrificed, and AgNPs mapped in brain.

To visualize silver nanoparticles functionalized with CP accumulated in the mouse brain, distribution of isotopically barcoded CP-109AgNPs and control biotin-107AgNPs nanoparticles were mixed in equimolar ratio and 200 µl of AgNPs (OD=100) and injected *i.v*. Following 3 h of circulation the mice were perfused with DPBS. The amount of isotopically barcoded targeted and control AgNPs in the tissues was determined with LA ICP-MS. In addition, CP-109AgNPs, Angiopep-2-107AgNPs and control nanoparticles were mixed in equimolar ratio and 200 µl of AgNPs (OD=100) and injected *i.v*. Following 3 h of circulation the mice were perfused with DPBS. The amount of isotopically barcoded targeted and control AgNPs in the tissues was determined with LA ICP-MS.) and liver sections using LA-ICP-MS.

The ratio of 109/107AgNPs in the control organ (liver) was similar to the ratio of the particles in the input mixture (~1) (**Fig. 3**; quantification of CP-AgNP accumulation in the brain and liver was compared to biotin-NPs and Angiopep-2-AgNPs).

The AgNPs functionalized with SRRVISRAKLAAAL (SEQ ID NO:9) achieved 6.72-fold (95% CI 4.28 to 9.16) higher accumulation in the brain compared to control AgNPs and 7.11-fold (95% CI 5.98 to 8.24) higher accumulation compared to AgNPs functionalized with Angiopep-2. Areas positive for SRRVISRAKLAAAL (SEQ ID NO:9) nanoparticle signal were evenly distributed between different brain regions of the brain section.

### Cerepep binds to glioblastoma and neuroblastoma cells

Binding of SRRVISRAKLAAAL (SEQ ID NO:9) phage to a panel of different cell lines was studied in vitro. Three of the assayed cell lines showed at least 50-fold higher binding of SRRVISRAKLAAAL phage compared to control insertless phage (**Fig. 4**; Binding of CP peptide-phage to different cell lines shown as fold over insertless phage. Data are shown as mean, n=2.). The highest ratio of SRRVISRAKLAAAL (SEQ ID NO:9) to control binding was seen with P3 stem cells (Dirkse, *et al.,* 2019), which is a human glioblastoma cell line; followed by mouse glioblastoma cell line WT GBM (Blouw, *et al.,* 2003) and commercially available mouse neuroblastoma cell line Neuro2A.

Next, the interaction of cultured Neuro2A cells with neutravidin-AgNPs labeled with NHS-CF555 dye and functionalized with biotinylated SRRVISRAKLAAAL (SEQ ID NO:9) (CP-AgNPs) was tested. Confocal imaging demonstrated that CP-AgNPs bound to Neuro2A cells, whereas the control biotin-AgNPs showed only background binding; 10 µl of OD=100 CP-AgNPs or control biotin-AgNPs were added to Neuro2a cells, with the cells in some samples preincubated with 100 µM free CP peptide for 1h at 37°C; after one hour of incubation with AgNPs at 37°C, some samples were etched to remove AgNPs bound to the surface of the cells. CP-AgNP signal was also present after treatment with a cell-impermeable etching solution (+Etch) to dissolve extracellular AgNPs, suggesting internalization. Binding and internalization of CP-AgNPs was abolished when the cells were pre-incubated with free SRRVISRAKLAAAL (SEQ ID NO:9), suggesting that the binding and uptake of CP-AgNPs by Neuro2A cells is peptide-dependent.

The binding (at 4°C) and uptake (at 37°C) of synthetic FAM-Cerepep by Neuro2A cells was also studied, comparing it to a control FAM-RPAR peptide which has a similar charge. FAM-Cerepep bound to and was taken up by Neuro2A cells, whereas the control FAM-RPAR showed only background binding; the binding (at 4°C) and internalization (at 37°C) of CP peptide to Neuro2A cells was visualized using cells incubated with 30 µM FAM-CP or a control peptide Fam-RPAR for 1h; Fam was detected with anti-fluorescein antibody).

### Cerepep endocytosis pathway

To study how treatment with endocytic inhibitors (nystatin, EIPA, cytochalasin D and chlorpromazine) affects the uptake of CP-AgNPs in Neuro2A cells, flow cytometry was used. Nystatin was used to block caveolae/lipid-mediated endocytosis, EIPA to block micropinocytosis, cytochalasin D to block actin polymerization and micropinocytosis and chlorpromazine to block clathrin-mediated endocytosis. Nystatin was the only inhibitor that did not have a statistically significant inhibitory effect on the uptake of CP-AgNPs (**Fig. 5**; Neuro2A cells were pre-incubated with inhibitors of endocytic pathways, 50 µM nystatin, 1 mM 5-(N-ethyl-N-isopropyl)-amiloride (EIPA); 30 µM chlorpromazine; 4 µM cytochalasin D and untreated cells as control at 37°C for 30 min. CP-AgNPs were added to the cells and incubated for 60 min, cells were treated by etching to remove membrane-bound particles. The cells were analyzed with flow cytometry for inhibition of uptake), suggesting that caveolae/lipid-mediated endocytosis is not contributing to CP-AgNP uptake by Neuro2A cells. EIPA, cytochalasin D and chlorpromazine had a similar and statistically significant inhibitory effect on the uptake of CP-AgNPs, suggesting that more than one endocytotic pathway is used for the uptake of CP-AgNPs by Neuro2A cells.

To map the route of endocytosis of CP-AgNP, the colocalization of CP-AgNPs was tracked with compartmental markers: early endosome marker Rab5a, late endosome marker Rab7a and trans-Golgi network marker TGN46. After 60 and 180 min, the CP-AgNPs showed endo-lysosomal uptake: colocalization with late endosomes positive for Rab7a and early endosomes positive for Rab5a.

### Identification of Cerepep receptor candidates

To identify potential receptor candidates of Cerepep (SRRVISRAKLAAAL (SEQ ID NO:9)) that facilitate blood-brain barrier penetration of the peptide, proximity labelling method was used that has been optimized for this purpose (unpublished data) on cultured WT GBM and Neuro2A cells. CP-HPR complex or control biotin-HRP complex was added to the cells and in the presence of peroxide, the cell surface proteins in proximity of the peptide-HRP complex were labelled with biotin from biotin-phenol. To confirm the efficiency and specificity of the method, the biotinylation of WT GBM cells was detected with fluorescent streptavidin conjugate and confocal microscopy.

The surface of cells treated with CP-HRP was extensively biotinylated, while cells treated with control biotin-HRP complex showed only background signal; to determine Cerepep receptor candidates, proximity ligation method with Cerepep-HRP complex and control biotin-HRP complex was used on WT GBM or Neuro2A cells to biotinylate the proteins on the surface of the cells that come into close contact with the complex. Biotin was detected with Streptavidin DyLight550. The WT GBM cells were analyzed with confocal microscopy. The samples of WT GBM and Neuro2A cells were also analyzed with flow cytometry, which confirmed that cells treated with CP-HRP had more biotin signal compared to control samples treated with biotin-HRP, although the signal of the latter was somewhat higher compared to samples where no HRP was added (**Figs. 6A-6B**).

The biotinylated proteins in the samples were pulled down and identified with mass spectrometry. **Table 2** and **Table 3** contain a list of proteins identified with mass spectrometry that have the highest spectral counts (sorting is based on the spectral counts in CP-HRP sample) in WT GBM and Neuro2A samples, respectively. Proteins that have a higher spectral count in CP-HRP sample and lower or no spectral counts in control samples with biotin-HRP an without HRP complex are considered as potential receptor candidates of Cerepep (highlighted in blue). There is a lot of overlap of potential receptor candidates when comparing the results of two cell lines, but LRP1 was considered the most probable candidate, as it was one of the top hits and has already been described as a receptor for numerous BBB penetrating peptides, including Angiopep-2 (Demeule, *et al.,* 2008).

### Cerepep binds to LRP1

Whether Cerepep (SRRVISRAKLAAAL (SEQ ID NO:9)) binds to human LRP1 purified from placenta was tested using surface plasmon resonance analysis. Cerepep-neutravidin complex generated higher response compared to LRP1 binding BBB penetrating peptide Angiopep-2-neutravidin complex. Furthermore, binding of CP-neutravidin complex to LRP1 is inhibited by RAP and EDTA, as can be expected with LRP1 ligands.

### Arginines and isoleucines are crucial for brain homing of Cerepep

To determine which amino acid positions are important for brain homing of SRRVISRAKLAAAL (SEQ ID NO:9), a pool of peptide-phage clones was compiled, where every amino acid position was one-by-one changed to alanine (alanines were changed to leucines). The peptide-phages were mixed in equimolar ratio, the Cerepep phage was included as a positive control and insertless as a negative control. The phage mix was injected into mice and following a 1h circulation the amount of different phage clones in the brain was determined using HTS. The results indicated that changing any of the three arginines or the isoleucine to alanine abolished the brain accumulation of the phage, suggesting that these amino acids are crucial for efficient brain homing. The addition of C-terminal alanine did not decrease brain homing of the phage, suggesting that Cerepep does not require a free C-terminus for efficient brain homing. Surface plasmon resonance analysis with human LRP-1 immobilized on a BIAcore sensor chip was used to assess whether CP-neutravidin complex directly interacts with human LRP-1. Angiopep-2-neutravidin complex was used as a positive control (**Figs. 7A-7B**).

**Tables 2 and 3:** The biotinylated proteins on the surface of WT GBM pulled down and identified with mass spectrometry

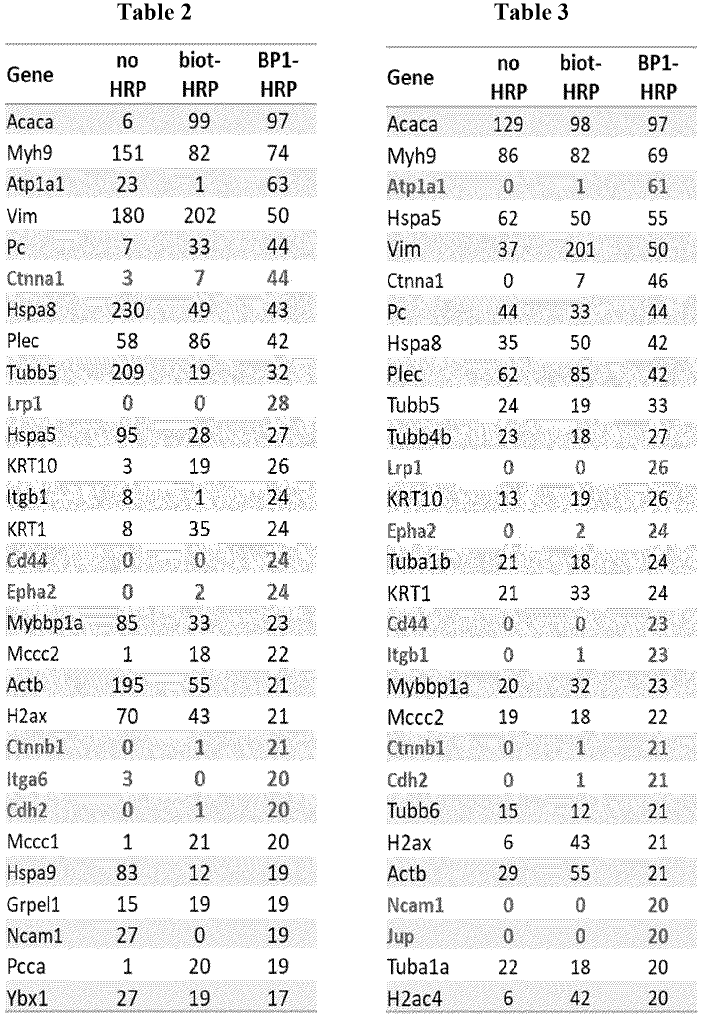

### The binding of Cerepep (CP)-neutravidin complex to LRP1 is inhibited by RAP and EDTA.

Based on studies involving in vivo play-off analyses, it was determined that Arginines and isoleucine are crucial for the brain homing of Cerepep (CP) peptide. Every position of CP peptide was one-by one mutated to alanine (alanines were changed to leucines) and the brain homing of each mutated peptide-phage was assessed using *in vivo* play-off method (**Fig. 7C**). A table of different phage-peptide sequences, showing the corresponding fold brain homing over a control phage is shown in **Figure 7D**.

### Cerepep cell recruitment mechanism)

To identify the receptor for the Cerepep peptide, it was demonstrated that binding of AgNP-Cerepep to P3 human stem cells is reduced by inhibitors of several LDL-receptor (low density lipoprotein receptor) proteins, Receptor Associated Protein (RAP) and anti-LRP-1 (Low density lipoprotein receptor-related protein 1, also known as alpha-2-macroglobulin receptor, apolipoprotein E receptor, or cluster of differentiation (CD)91 antibodies.

Briefly, P3 human stem cells were incubated with the indicated antibody (AB) or RAP (diluted in 20% of glycerol) at 4°C for 1 h, after cells were exposed to AgNP-control or AgNP-Cerepep for 1 h at 4°C, then the cells were analyzed by FACS. Data are shown in **Figs. 18A-18B** as fold over AgNP-Cerepep binding, where **Fig. 18A** shows incubation with anti-LRP-1 antibody, while **Fig. 18B** shows incubation with RAP, LDLR antibody, and both anti-LRP-1 and anti-LDLR antibodies. The data demonstrate that anti-LRP-1 antibody blocks binding of Cerepep with its receptor.

Next, experiments were carried out to assess if and how Cerepep interacts with LRP-1 cluster II and cluster III.

Briefly, 50 µl of Myone Dynabeads were washed multiple times and incubated with 100 µl of 2 mM biotinylated peptide at room temperature for 2h. Beads were washed twice with PBS, 0.01% Tween 20-DPBS, and 0.01% PBS-BSA. The beads werethen incubated overnight with 10 nM LRP-1 cluster II, or LRP-1 cluster III, and Alexa Fluor 647 anti-human LRP-1 antibody (1:200) in PBS with 0.1% BSA (**Figs. 19A-****19B**).

The beads were washed several times and analyzed by FACS. Samples are indicated as follows:
Negative control (-ve control): beads incubated with biotin (non-peptide), LRP-1 cluster III protein, and Alexa Fluor 647 anti-human antibody;
Positive control for the cluster II expts: beads incubated with biotinylated-Angiopep2 peptide, LRP-1 cluster II protein and Alexa Fluor 647 anti-human LRP-1 antibody (see **Fig.19A**); and
Positive control for cluster III expts: beads incubated with biotinylated-RPAR peptide, Neuropilin-1 b1b2 protein, (1:200) anti-neuropilin-1 antibody, (1:200) and Alexa Fluor 647 anti-rabbit antibody (see **Fig.19B**).

10,000 events per condition were analyzed. Experient was performed in three replicates per condition and t-test compared scramble sequence (SARVISRAKLARAL (SEQ ID NO:70)) with Cerepep.

### Cerepep-AgNPs colocalize with lysosomes

P3 stem cells were grown on coverslips and incubated with AgNP-control or AgNP-Cerepepat 37°C for 1 h, fixed with methanol, and stained using anti-LAMP1 antibody and DAPI. Micrographs were viewed under a confocal microscope with a 60x objective. Colocalization analysis of the images was carried out using Colormap script ImageJ plugin. The data are depicted in **Fig. 20****,** with each bar representing the mean correlation index (Icorr, ImageJ output). Experient was performed in three replicates and the error bars indicate the standard deviation. The data show colocalization of AgNP-Cerepep with lysosomes.

### The LRP1 antibody inhibits the binding of Cerepep in the brain.

To assess the ability of mAbs to inhibit Cerepep activity in brain, 25 µg of LRP1 (N-terminal) antibody (Sigma L2295) was injected into the tail vein of mice, after 1 h of circulation, phages were injected and after 30 min the animals were anesthetized and perfused. Brain and liver were collected and homogenized in LB + 1% NP40, the phages titers were determinate in each organ (see **Figs. 21A-21B**). Raw data present phage accumulation in the liver and brain (**Fig. 21A**) and the fold-over control phage is shown for brain (**Fig. 21B**), with datapoints indicated on the bar. The data show LRP1 antibody inhibits the binding of Cerepep.

### FAM-Cerepep peptide crosses the blood-brain barrier.

To assess the ability of FAM-conjugated to Cerepep to travesrse the BBB, 100 µl of 2 mM FAM-Cerepep or FAM-scrambled Control peptide (SARVISRAKLARAL (SEQ ID NO:70)) was injected into the tail vein of mice n=3. After 2.5 h of circulation, the animals were anesthetized and perfused, the brains were collected, cryosected, fixed, permeabilized, and incubated with CD31-rat and anti-fluorescein-rabbit primary antibodies at room temperature for 2 hours. After washes, sections were incubated with Alexa Fluor 456 anti-rat and Alexa Fluor 647 anti-rabbit secondary antibodies, washed and stained with DAPI. Images were taken using a confocal microscope in 4 different areas of the brain (cortex, cerebellum, hippocampus, brainstem). FAM-Cerepep could be detected in the brain tissues.

### FAM-Cerepep colocalizes with mature neurons.

To assess the ability of FAM-conjugated to Cerepep to colocalize with mature neurons, 100 µl of 2 mM FAM-Cerepep or FAM-scramble (SARVISRAKLARAL (SEQ ID NO:70)) was injected into the tail vein of mice n=3. After 2.5 h of circulation, the animals were anesthetized and perfused, the brains were collected, cryosected, fixed, permeabilized, and incubated with NeuN-mouse and anti-fluorescein-rabbit primary antibodies for 2 hours at room temperature. After washes, sections were incubated with Alexa Fluor 456 anti-mouse and Alexa Fluor 647 anti-rabbit secondary antibodies, sections were washed and stained with DAPI. Pictures were taken under a confocal microscope in 4 different areas of the brain (cortex, cerebellum, hippocampus, brainstem). FAM-Cerepep could be detected associated with neurons in the brain tissues.

### FAM-Cerepep colocalizes with astrocytes

To assess the ability of FAM-conjugated to Cerepep to colocalize with astrocytes,100 µl of 2 mM FAM-Cerepep or FAM-scramble (SARVISRAKLARAL (SEQ ID NO:70)) was injected into the tail vein of mice n=3. After 2.5 h of circulation, the animals were anesthetized and perfused, the brains were collected, cryosected, fixed, permeabilized, and incubated with GFAP-rat and anti-fluorescein-rabbit primary antibodies for 2 hours at room temperature. After washes, sections were incubated with Alexa Fluor 456 anti-rat and Alexa Fluor 647 anti-rabbit secondary antibodies, sections were washed and stained with DAPI. Pictures were taken under a confocal microscope in 4 different areas of the brain (cortex, cerebellum, hippocampus, brainstem). FAM-Cerepep could be detected associated with astrocytes in the brain tissues.

### FAM-Cerepep colocalizes with microglia.

To assess the ability of FAM-conjugated to Cerepep to colocalize with microglia, 100 µl of 2 mM FAM-Cerepep or FAM-scramble (SARVISRAKLARAL (SEQ ID NO:70)) was injected into the tail vein of mice n=3. After 2.5 h of circulation, the animals were anesthetized and perfused, the brains were collected, cryosected, fixed, permeabilized, and incubated with CD11b-rat and anti-fluorescein-rabbit primary antibodies at room temperature for 2 hours. After washes, sections were incubated with Alexa Fluor 456 anti-rat and Alexa Fluor 647 anti-rabbit secondary antibodies, sections were washed and stained with DAPI. Pictures were taken under a confocal microscope in 4 different areas of the brain (cortex, cerebellum, hippocampus, brainstem). FAM-Cerepep could be detected associated with microglia in the brain tissues.

To quantify FAM-Cerepep in different regions of the brain, expts were set up to assess fluorescence intensity. Briefly, 100 µl of 2 mM FAM-Cerepep or FAM-scrambled peptide (SARVISRAKLARAL (SEQ ID NO:70)) was injected into the tail vein of mice n=3. After 2.5 h of circulation, the animals were anesthetized and perfused, the brains were collected, cryosected, fixed, permeabilized, and incubated with anti-fluorescein-rabbit primary antibody for 2 hours at room temperature. After washes, sections were incubated with Alexa Fluor 647 anti-rabbit secondary antibody, sections were washed and stained with DAPI. Images were taken under a confocal microscope in 4 different areas of the brain (cortex, cerebellum, hippocampus, brainstem), and fluorescence intensity in each brain region was measured and quantified using ImageJ, as depicted in **Figs. 22A-22E**.

### HER2-FAM-Cerepep antibody crosses the blood-brain barrier

To assess whether Cerepep conjugated to a cargo molecule, such as an antibody crosses the blood-brain barrier, HER2-FAM-scrambled (SARVISRAKLARAL (SEQ ID NO:70)) or HER2-FAM-Cerepep antibodies were injected into the tail vein of mice. After 24 h of circulation, the animals were anesthetized and perfused, the brains were harvested and divided into two hemispheres, half of the brain of the mice was cryosectioned, fixed, permeabilized and incubated with CD31-rat and fluorescein-rabbit primary antibodies at room temperature for 2 h. After washes, sections were incubated with Alexa Fluor 456 anti-rat and Alexa Fluor 647 anti-rabbit secondary antibodies, washed again and stained with DAPI. Pictures were taken under a confocal microscope in 3 different areas of the brain (cortex, hippocampus and brainstem). Fluorescence intensity signal from FAM was measured in each area using ImageJ, as depicted in **Fig. 23**.

### Cerepep library accumulates in the brain

Accumulation of the Cerepep library in the brain of mice was detected in two second rounds of biopanning. The constrained Cerepep-based library containing critical residues identified in alanine scan (XRRXIXRAXLAXXX (where x is a random amino acid; SEQ ID NO:76)) was prepared and 5×10⁹ pfu of library phages were injected into the tail vein of mice, after 1 h of circulation, animals were anesthetized and perfused.

Brain, lung and liver were collected and homogenized in LB + 1% NP40, the phages titers were determinate in each organ, while phage pools recovered from brains were amplified in semi-solid media, purified, and injected into the tail vein of mice for the second round of biopanning; raw data present phage accumulation in the brain after rounds 1 and 2 of biopanning (**Fig. 24A**). The fold-over control phage is shown for all three organs analyzed in **Fig. 24B**, and the data is indicated on the bar. Experiment was performed in three replicates per condition and the error bars show the standard deviation. The data indicated accumulation of the Cerepep library in the brain tissues. Peptides from constrained library screen (XRRXIXRAXLAXXX (where x is a random amino acid; SEQ ID NO:76)) were ranked by high throughput DNA sequencing based representation in the brain (ranked based on Round 2 to Round 1 ratio) and weblogo based on top ranking 20 peptides, as depicted in **Fig. 25**.

The 20 top peptides that were identified as being accumulated in the brain based on the bio-panning experiments are set forth in **Table 7**, below.

**Table 7: Top 20 peptides identified from constrained library screen**

| **Peptide Sequence** | **SEQ ID NO:** |
|---|---|
| GRRVIGRASLAQDE | 95 |
| NRRVIDRAALASL | 77 |
| SRRVISRAGLADNL | 78 |
| PRRVIGRAGLASTA | 79 |
| GRRVIGRASLAPDS | 80 |
| GRRIIERAALALED | 81 |
| GRRPISRANLANTD | 82 |
| PRRIISRAQLAQTS | 83 |
| GRRVISRAGLASDD | 84 |
| ARRIINRAILASDP | 85 |
| PRRIITRATLAPPV | 86 |
| TRRVIDRAGLANEK | 87 |
| ARRVISRAGLAQDT | 88 |
| ARRIIPRAPLADR | 89 |
| ARRVISRAELARNG | 90 |
| ARRTISRAALAQ | 91 |
| QRRVIPRAKLALEE | 92 |
| ARRVISRANLANPT | 93 |
| NRRVIPRAGLAENQ | 94 |
| NRRVIPRAGLASND | 96 |

### Example 2. Identification and Development of Brain Penetrating Peptide CVG Methods

### Peptides

Fluorescent peptides [5(6)-carboxyfluoresceine (FAM)-labeled and with 6-aminohexanoic acid spacer attached to the N-terminus of the peptides] and biotinylated peptides were ordered from a commercial suppliers (TAG Copenhagen, Denmark and LifeTein, USA)

### Cell lines

22 different cell-lines derived from human or mouse tumors or normal tissues were used in the experiments (**Table 4**).

**Table 4. Cell-lines used in the in vitro and in vivo experiments.**

| **Cell lines** | |
|---|---|
| PPC1 | Human prostate adenocarcinoma |
| PC-3 | Human prostate adenocarcinoma |
| M21 | Human melanoma |
| B16-F-10 | Mouse skin melanoma |
| 4T1 | Mouse mammary gland tumor |
| MDA-MB-231 | Human mammary gland adenocarcinoma |
| MCF10CA1a | Human mammary gland carcinoma |
| U87MG | Human glioblastoma |
| WT GBM | Mouse glioblastoma |
| VEGFko GBM | Mouse glioblastoma |
| P3 stem-like | Human glioblastoma |
| NCH421k | Human glioblastoma |
| Neuro2A | Mouse neuroblastoma |
| HT1080 | Human fibrosarcoma |
| RCC-MF | Human kidney carcinoma |
| CT26 | Mouse colon carcinoma |
| MKN45P | Human gastric adenocarcinoma |
| HepG2 | Human hepatocellular carcinoma |
| HBVP | Human Brain Vascular Pericytes |
| RC-124 | Human renal cell line |
| HEK293 | Human embryonic kidney cell line |
| RAW264.7 | Mouse macrophage cell line |

### Peptide-phage bio-panning

Play-off screen was carried out with 10 previously published BBB penetrating/CNS homing peptide-phages. DNA oligonucleotides that encode a peptide displayed on the phage surface were purchased from Integrated DNA Technologies, Inc. (USA). Oligonucleotides were annealed and ligated into the phage genome using T7Select 415-1 cloning kit (Novagen) and the T7Select System Manual. Each peptide-phage was separately amplified in liquid bacterial culture, precipitated with PEG, recovered in PBS and titered. Phages were mixed in equimolar ratios and purified with CsCl-ultracentrifugation and dialysis. Titer of the play-off pool was determined and mice were intravenously injected with 1x10¹⁰ pfu of phage pool. After one hour of circulation time, mice were perfused with acid buffer (500 mM NaCl, 0.1 M glycine, 1% BSA, pH2.5) and detergent buffer (1%NP40 in PBS), collected organs were homogenized and phage pools amplified for high-throughput sequencing.

*In vivo* phage display with CX4CYX library was performed in three selection rounds. Phage library was injected intravenously to Balb/c mice and allowed to circulate for one hour. After circulation, the animals were anesthetized and perfused intracardially with acid and detergent buffers. Brain and control organs were collected and homogenized in 1%NP40-LB. Bound phages were rescued by amplification in *E*. *coli* and their genomic DNA was sequenced by Ion Torrent next generation DNA sequencing system.

### Alanine

Phage pool for alanine-leucine scanning contained original CAGALCY (SEQ ID NO:21) peptide-phage and phages where each amino acid in the CAGALCY (SEQ ID NO:21) peptide was substituted with either alanine or leucine. Phage pool was *i.v*. injected to mice, at one hour time point mice were perfused, brains collected and phage pools from each mouse amplified, purified and sequenced with HTS.

### Phage binding experiments on cell lines

To evaluate the CVGTNCY (SEQ ID NO:2) peptide-phage binding on cell lines, the CVGTNCY (SEQ ID NO:2) peptide-phage and control G7 phage were incubated on attached cells (except NCH421k cells that grow as speroids) in DMEM-0.5% BSA for 1 hour at +4 °C. Thereafter, the cells were washed four times with DMEM-0.5% BSA and phage was released in 1%NP40-LB to determine the titer of the samples.

### Animal experiments

Animal experimentation procedures were approved by the Estonian Ministry of Agriculture, Committee of Animal Experimentation, Project #48 and #159. Balb/c mice and Athymic nude mice (HD) were housed in a pathogen-free environment at the Animal Facility of the Institute of Biomedicine and Translational Medicine, University of Tartu (Tartu, Estonia). For induction of orthotopic GBM xenografts in nude mice, 7 x 10⁵ (WT-GBM, VEGFko GBM) cells were implanted intracranially in the right striatum of the brain (ccordinates: 2 mm laterally and 2 mm posteriorly from bregma and at 2.5 mm depth). Intracranial tumors developed for 6-7 days (WT-GBM) and 12-14 days (VEGFko GBM) before performing experiments.

### Isotopic silver nanoparticle synthesis and functionalization

Isotopically-barcoded silver nanoparticles (AgNP) were synthesized using a modified Lee and Meisel citrate method. Isotopically pure (107Ag or 109Ag) AgNO3 (50.4 mg) was dissolved in the dark in 1 ml of Milli-Q water (MQ, resistivity 18 MQ cm) and added to 500 ml of water heated at 65 °C in a glass flask cleaned with Piranha solution (H2SO4 and H2O2; Caution: highly oxidizing acid solution). Tannic acid (1.2 mg) was dissolved in 10 ml of MQ and 200 mg of citrate tribasic dihydrate was added to the solution. This mixture was added to the AgNO3 solution in the reaction vessel. The mixture was vigorously stirred at ~70 °C and the reaction was allowed to proceed for 3 min, at which point the solution turned yellow. The flask was then transferred to a pre-heated hot-plate, boiled for 20 min, and left to cool down to RT. The boiled-off volume was reconstituted with fresh MQ.

The particles were functionalized with NeutrAvidin (NA) to allow conjugation with biotinylated-peptides and lipoic acid-polyethylene glycol (1 k)-NH2 (PEG). The terminal amines of PEG were used for coupling of CF555-N-hydroxysuccinimide-dye (NHS-dye). Biotin-X-CVGTNCY (SEQ ID NO:2)-OH (X, aminohexanoic acid) peptide was coated on 107AgNPs, and 109AgNPs were blocked with free D-biotin. The AgNPs were washed to remove free peptides by centrifugation at 7000G, decanting, and resuspension in fresh buffer (0.1 M HEPES pH 7.2, 0.1 M NaNO3, 0.005% Tween-20) with sonication.

### In vivo biodistribution studies

CVGTNCY (SEQ ID NO:2) peptide-phage or control phage were injected iv into mice, after one hour circulation animals were perfused with 20 ml PBS, organs were collected, weighed, and dropped to 1% NP40-LB solution. Organs were homogenized and titer of each tissue was determined. FAM-labeled peptides (2 mM of peptide in PBS) were injected intravenously and 3 hours later the animals were perfused with 20 mL of PBS. The organs were excised, snap-frozen in the vapor of liquid nitrogen, and stored at -80 °C. For confocal microscopy, the tissues were sectioned at 15 µm and stained with antibodies and DAPI.

For *in vivo* homing of isotopically barcoded AgNPs, the 107Ag and 109Ag particles were suspended at equimolar ratio in 200 µl PBS and injected into tail vein of Balb/c mice. After 3 hours circulation, the mice were perfused via the left ventricle of the heart with 20 mL PBS. Organs were snap-frozen in the vapor of liquid nitrogen and stored at -80 °C until sectioning.

### Laser ablation ICP-MS analysis

For ratiometric laser ablation (LA) ICP-MS-based biodistribution studies, the organs from mice injected with Ag-NPs were collected, snap-frozen, cryo-sectioned in slices of 30 µm thickness on Superfrost Plus slides and air-dried. Determination of 109Ag/107Ag ratio in tissue sections was performed using Cetac LSX-213 G2+ laser ablation system (Teledyne Cetac Technologies, USA) using a HelEx 2-volume ablation cell on Agilent 8800 ICP-MS system. The LA-ICP-MS setup was optimized using NIST 612 glass slides. The same reference sample was used to monitor the ThO/Th ratio during the analytical run, which remained under 0.3%. 13C, 107Ag and 109Ag isotopes were monitored with dwell times of 47.5 ms, 95 ms and 95 ms respectively, corresponding to a duty cycle of 0.25 s. 13C was used as internal standard to account for differences in the carbon content of the ablated tissue.

### In vitro binding of AgNPs and microscopy

WT-GBM and VEGFko cells were grown in MEM with Earl's salts (Capricorn Scientific, Germany); 100 IU/ml of Pen/Strep; 1% sodium pyruvate, 0.01 M HEPES, 0.6% glucose (Applichem, USA) and 5% of heat-inactivated fetal bovine serum (GE Healthcare, UK). Microscopy experiments were carried out with wtAgNPs with CF555 dye. The culture medium of cells was aspirated, the cells were washed twice with warm medium, and fresh medium was added along with CVGTNCY (SEQ ID NO:2)-targeted AgNPs or biotin-AgNPs at 0.2 nM. The cells were incubated with NPs at 37 °C for one hour. After incubation with AgNPs the NP containing medium was aspirated, the cells were washed, then fixed with cold methanol, and washed with PBS. Nuclei were stained with DAPI at 1 µg mL⁻¹. The coverslips were mounted onto glass slides with Fluoromount-G (Electron Microscopy Sciences) imaged using a Olympus FV1200MPE confocal microscope (Olympus, Germany) and the images were processed and analyzed using the FV10-ASW 4.2 Viewer image software (Olympus, Germany).

### Immunohistochemical staining of tissues and microscopy

Cryosections (15 µm) on Superfrost Plus slides were fixed in cold methanol, washed in PBS, treated with 3% hydrogen peroxide, and blocked in PBS containing 0.05% Tween-20, 5% BSA, and 5% goat serum (GE Healthcare, UK) for 1 h. The sections were immunostained with rabbit anti-fluorescein (cat #A889, Thermo Fisher Scientific, MA, USA) antibody overnight at +4 °C. Immunohistochemical staining was achieved with Tyramide SuperBoost Kit (Invitrogen) and Pierce DAB Substrate Kit (Thermo Scientific). Sections were imaged with Leica Aperio VERSA microscope slide scanner. Imaged were processed with Aperio Image Scope (v12.4.3.5008).

### Binding study on ELISA plate

Reelin protein (#8546-MR-050; R&D Systems), NRP-1 b1b2 (wt and mutant) or 5 % BSA was immobilized on Costar 96-Well ELISA plate (#3590, Corning Life Sciences, Tewksbury, MA, USA). The multiwell plates were coated with 20 µg/ml recombinant protein in 100 µl of PBS overnight at +4 °C, followed by blocking with 1% bovine serum albumin (BSA) in PBS overnight at +4 °C. The phage (5x10⁸ pfu in 100 µl of PBS-BSA) was incubated overnight at +4 °C, followed by 6 washes with PBS containing 1% BSA and 0.1% Tween-20 (washing buffer) to remove background phages. BLT5615 OD₆₀₀=0.5 containing 2mM IPTG was used to recover the phages and determine the titer of samples.

### Results

### Identification of a novel CNS homing peptide

Novel BBB penetrating and CNS homing peptide was identified in a step by step process including a phage play-off screens, alanine scanning mutagenesis and phage display with unique library. The first selection step included a phage play-off screen with ten previously published CNS homing peptides (**Table 5**). To ensure collecting BBB penetrating and CNS homing peptides, phage play-off pool was intravenously (*iv)* injected into the mice and perfusion was done with acid and detergent buffers to remove unbound phages and brake the endothelial layer of blood vessels. One peptide CAGALCY (SEQ ID NO:21) showed the highest representation in the brain based on the Ion Torrent high throughput sequencing (HTS) reads in the screen and it was selected for further modifications (**Figure 8A**; *in vivo* play-off phage display screen with 10 published CNS homing/BBB penetrating peptides revealed high representation of CAGALCY (SEQ ID NO:21) in the brain). In next step, each amino acid in CAGALCY (SEQ ID NO:21) peptide was substituted with either alanine or leucine to determine the essential amino acids for CNS homing. Substitution of both cysteines (Cys1 and Cys6) and tyrosine (Tyr7) in CAGALCY (SEQ ID NO:21) peptide led to the most significant decrease in brain homing demonstrating the importance of these amino acids for the peptide's ability to reach to the target site (**Figure 8B**; Alanine-leucine screening of CAGALCY (SEQ ID NO:21) peptide illustrated the relevance of both cysteines, glycine and tyrosine for brain homing.

A novel phage library CX4CYX was constructed based on the alanine-leucine screen to find new brain homing peptides). This knowledge directed us to construct a novel phage library CX4CYX (X - any amino acid), where Cys1, Cys6 and Tyr7 remained in their intact positions, and the four amino acid variable-domain laid between the cysteines, and one amino acid variability after Tyr7. Three rounds of phage bio-panning was performed to allow enrichment of peptides in the brain. Again acid and detergent buffers were used for perfusion to remove non-specifically bound phages from the brain after systemic administration of phage library (round 1) and recovered brain phage pools (round 2 and 3). HTS reads from round 1 to round 3 indicated decent enrichment of in-frame peptides in the brain (**Table 6**). The first peptide in the list, CVGTNCY (SEQ ID NO:2), showed about 40 times more reads in the brain than the original CAGALCY (SEQ ID NO:21) peptide in round 3 (**Table 6**; selection of identified peptides from *in vivo* phage display screen with CX4CYX phage library, with peptides listed according to the ratio of sequencing reads between round 3 and round 1). Homing of the four peptides; CVGTNCY (SEQ ID NO:2), CEGALCY (SEQ ID NO:24), CTGSLCY (SEQ ID NO:12) and CDGALCY (SEQ ID NO:34)) that showed the highest ratio between round 3 and round 1, were evaluated in additional play-off screen (**Figure 8C**; *in vivo* play-off screen with 4 potential brain homing peptides identified in CX4CYX phage display in comparison with the published original CAGALCY (SEQ ID NO:21) peptide. Peptide CVGTNCY (SEQ ID NO:2) demonstrates the highest representation in the brain and was selected for detailed validation as a BBB penetrating/CNS homing peptide). Compared to the initial CAGALCY (SEQ ID NO:21) peptide, the CVGTNCY (SEQ ID NO:2) showed the highest representation in the brain and was chosen for detailed characterization as a novel BBB penetrating and CNS homing peptide.

**Table 6: Selection of identified peptides from in vivo phage display screen with CX4CYX phage library. Peptides are listed according to the ratio of sequencing reads between round 3 and round 1.**

| **Peptide** | **R1 reads** | **R2 reads** | **R3 reads** | **R3** > **R1** | |
|---|---|---|---|---|---|
| C**V**GTNC**Y** | 12 | 1474 | 37038 | 3087 | (SEQ ID NO:2) |
| C**E**GALC**Y** | 17 | 1370 | 39863 | 2345 | (SEQ ID NO:24) |
| C**E**GS**L**C**Y** | 3 | 52 | 2833 | 944 | (SEQ ID NO:11) |
| CTGS**L**C**Y** | 64 | 3013 | 43464 | 679 | (SEQ ID NO:12) |
| CAGS**M**C**Y** | 14 | 750 | 9077 | 648 | (SEQ ID NO:13) |
| C**V**GT**L**C**Y** | 21 | 1295 | 11631 | 554 | (SEQ ID NO:14) |
| CKGSNC**Y** | 4 | 100 | 2037 | 509 | (SEQ ID NO:15) |
| C**V**GQ**L**C**Y** | 12 | 504 | 5355 | 446 | (SEQ ID NO:16) |
| CAGP**L**C**Y** | 3 | 208 | 1232 | 411 | (SEQ ID NO: 17) |
| CQGA**L**C**Y** | 27 | 1410 | 8612 | 319 | (SEQ ID NO: 18) |
| C**V**GANC**Y** | 24 | 813 | 7359 | 307 | (SEQ ID NO: 19) |
| CQGSNC**Y** | 4 | 287 | 1202 | 301 | (SEQ ID NO:20) |
| CAGA**L**C**Y** | 3 | 71 | 880 | 293 | (SEQ ID NO:21) |
| CTGS**M**C**Y** | 3 | 63 | 841 | 280 | (SEQ ID NO:22) |
| C**M**G**DL**C**Y** | 3 | 119 | 837 | 279 | (SEQ ID NO:23) |

### Biodistribution of CVGTNCY peptide in rodents

To describe the utility of CVGTNCY (SEQ ID NO:2) peptide as a systemic CNS homing peptide, the biodistribution of CVGTNCY (SEQ ID NO:2) peptide-phage as well as peptide-functionalized AgNPs were analyzed in rodents. High level of CVGTNCY (SEQ ID NO:2) peptide-phage was detected in mouse CNS and eyes compared to the control G7 phage when injected i.v., to animals, whereas in numerous other analyzed organs CVGTNCY (SEQ ID NO:2) did not show a trend of homing. In the CNS, CVGTNCY (SEQ ID NO:2) was distributed throughout the nervous tissue, but showed the tendency to home more to the hippocampus, frontal cortex and cerebellum (**Figure 9**; Biodistribution analysis of CVGTNCY (SEQ ID NO:2) peptide-phage in mice shows homing in CNS; CVGTNCY (SEQ ID NO:2) peptide-phage or control phage G7 was injected *iv*, into the mouse for one hour circulation, perfusion was performed with acid and detergent buffer, and tissues of interest were collected to determine the titers. Graph shows the ratio of CVGTNCY (SEQ ID NO:2) phage counts to control G7 counts. Significantly higher CVGTNCY (SEQ ID NO:2) accumulation was observed in various CNS regions compared to control phage. No significant difference between CVGTNCY (SEQ ID NO:2) and G7 titers was determined in the rest of the analyzed organs/tissues). Comparative biodistribution analysis between mice and rats proved that in both species CVGTNCY (SEQ ID NO:2) homes preferentially to the CNS than to other organs (**Figures 10A** **and** **10B**; CVGTNCY (SEQ ID NO:2) peptide-phage homes to CNS in both mice and rats. CVGTNCY (SEQ ID NO:2) peptide-phage or control phage (G7 in mice and Insertless in rats) was intravenously injected to the rodents, one hour later animals were perfused with PBS and tissues of interest collected to determine the titers. Graphs represent the ratio of CVGTNCY (SEQ ID NO:2) phage counts over the control counts in CNS and control tissues of mice (**Fig. 10A**) and rats (**Fig. 10B**).

Laser-Ablation Inductively-Coupled Plasma Mass Spectrometry (LA-ICP-MS) allows ultrasensitive detection of AgNPs in the tissues of interest with a possibility to quantify *in vivo* biodistribution of peptide functionalized AgNPs. To study CVGTNCY (SEQ ID NO:2)-functionalized AgNP distribution in the brain, a cocktail of isotopically barcoded CVGTNCY (SEQ ID NO:2)-Ag107NPs and control-Ag109NPs was i.v. coinjected into the Balb/c mice. AgNPs were allowed to circulate for 3 hours, thereafter the animals were perfused and cryosections prepared from snap-frozen brains and livers. LA-ICP-MS mapping of Ag107/Ag109 ratio in the brain showed distribution throughout the brain with some heterogeneity, meaning that some areas had Ag107/Ag109 ratio of 30 and above. The homing of CVGTNCY (SEQ ID NO:2)-Ag107NPs was brain specific, as the Ag107/Ag109 ratio in the control organ (liver) remained close to the input 1:1 ratio (**Figures 10C** and **10D**; Ratiometric Laser Ablation-Inductively Coupled Plasma Mass Spectrometry (LA-ICP-MS) profiling of CVGTNCY (SEQ ID NO:2)-AgNP on brain sections was used to identify tissue homing in Balb/c mice injected i.v. with a mixture of CVGTNCY (SEQ ID NO:2)-Ag107NPs (OD=100) and control biotin-Ag109NPs (OD=100). At 3-hour time point mice were perfused, organs (brain and liver) collected, snap-frozen, and sectioned at 30 µm. Laser ablation line scans data indicated that CVGTNCY (SEQ ID NO:2)-Ag107NPs were distributed throughout the brain, and that Control-Ag109NPs show tendency to accumulate in the choroid plexus due to leaky blood vessels. The results demonstrate preferential homing of CVGTNCY (SEQ ID NO:2) to the CNS and not to other organs/tissues).

### CVG peptide targets specially cortical and hippocampal neurons

To confirm CVGTNCY (SEQ ID NO:2) as a BBB penetrating and CNS homing peptide, the presence of CVGTNCY (SEQ ID NO:2) -AgNPs in the cleared brain was analyzed by light-sheet microscopy, and confocal microscopy, respectively. Mice were i.v. injected twice with either CVGTNCY (SEQ ID NO:2)-AgNPs or control-AgNPs with 12-hour interval, one hour before sacrifying the animals were i.v. injected with Wheat Germ Agglutinin-Alexa Fluor 555 conjugate to visualize the blood vessels in the CNS. Animals were perfused and processed for imaging. Light-sheet microscopy imaging detected CVGTNCY (SEQ ID NO:2)-AgNPs outside of the brain blood vessels and quantification of AgNPs in the whole volume of the brain (**Figure 10E**), and specifically in the white matter (**Figure 10F**) showed significantly more CVGTNCY (SEQ ID NO:2)-AgNPs in both domains compared to control-AgNPs. Altogether the results prove CVGTNCY (SEQ ID NO:2) crosses the BBB and homes to the brain parenchyma.

Next, a co-localization analysis was carried out to identify the CVGTNCY (SEQ ID NO:2) targeting cell types in the CNS. CNS sections from mice injected with CVGTNCY (SEQ ID NO:2)-AgNPs or control-AgNPs were co-stained with cell type specific markers, such as pan-neuronal NeuN, astrocytic GFAP and oligodendrocytic Olig2. CVGTNCY (SEQ ID NO:2)-AgNPs showed co-localization with NeuN-positive cells in the CNS, in contrast colocalization of control-AgNPs with neurons was not evident (CVGTNCY (SEQ ID NO:2)-AgNPs colocalize with neuronal cells in the cortex and hippocampus. CVGTNCY (SEQ ID NO:2)-functionalized AgNPs (OD=100) were i.v. injected twice with 12-hour interval, after perfusion of the animals, organs were collected and processed for IF stainings. Colocalization analysis of CVGTNCY (SEQ ID NO:2)-functionalized AgNPs and pan-neuronal marker NeuN shows colocalization on confocal microscopy images). Quantification of CVGTNCY (SEQ ID NO:2)-AgNPs in NeuN-positive cells shows significant colocalization compared to control-AgNPs in hippocampus CA3, hippocampus GD, cortex and corpus callosum; significant colocalization between CVGTNCY (SEQ ID NO:2)-AgNPs and NeuN-positive cells was not detected in cerebellum). Quantification of AgNPs showed significantly more CVGTNCY (SEQ ID NO:2)-AgNPs associated with neurons in the hippocampus CA3 and dentate gyrus (GD) regions, as well as in the cortex and corpus callosum (**Figures 11A-11E**). CVGTNCY (SEQ ID NO:2)-AgNPs showed also a tendency to colocalize with cerebellar neurons, but the difference with control-AgNPs was not significant. Additionally, CVGTNCY (SEQ ID NO:2)-AgNPs showed association with blood vessel walls on the brain parenchyma, co-localizing especially with GFAP-positive astrocytes. CVGTNCY (SEQ ID NO:2)-AgNPs were detected in microglia cells, and in the spinal cord neurons (motoneurons). Taken together, the results confirm that CVGTNCY (SEQ ID NO:2) is a BBB penetrating peptide that targets astrocytes, neurons and occasionally microglia in the CNS.

**Table 5. CNS homing/BBB penetrating peptides for in vivo play-off**

| **Peptide (abbreviation)** | **Identification** | **Receptor** | **Evidence for BBB penetration** | **Reference** | **SEQ ID NO** |
|---|---|---|---|---|---|
| **CAGALCY** | *In vivo* phage display for mouse brain homing peptides | Unknown | n.a. | Fan et al. Pharmaceutical Research 2007, 24, 5, 868-879 | 21 |
| **TPSYDTYAAELR (TPS)** | *In vivo* phage display for rat brain homing peptides | Unknown | Peptide-phage recovered from the rat CSF | Li et al., Amino Acids 2015, 47, 2, 401-405 | 33 |
| **CLSSRLDA (SRL)** | *In vivo* phage display for mouse brain homing peptides | LDL receptor-related protein | Barrier penetration in an *in vitro* cell-based rat BBB model | Pasqualini, Ruoslahti; Nature 1996, 380, 364-366 Zarebkohana et al. European Journal of Pharmaceutical Sciences 2015, 78, 19-30 | 25 |
| **LSSRLDAC** | *In vivo* phage display for mouse brain homing peptides | LDL receptor-related protein | Barrier penetration in an *in vitro* cell-based rat BBB model | Pasqualini, Ruoslahti; Nature 1996, 380, 364-366 Zarebkohana et al. European Journal of Pharmaceutical Sciences 2015, 78, 19-30 | 26 |
| **ACSYTSSTMCGGGS (ACSYTSS)** | *In vivo* phage display for rat brain homing peptides | Unknown | Presence in the rat brain parenchyma after capillary depletion | Smith, Gumbleton; Drug Discov Today 2010;15:1113 | 27 |
| **CGHKAKGPRK (B6)** | *In vitro* phage display on extracellular domain of hTfR | Transferrin receptor | Neuronal delivery of therapeutic payload in *mouse* Alzheimer's disease model | Liu et al. Bioconjugate Chem. 2013, 24, 997-1007 Xia et al. Journal of Virology 2000, 74, 23, 11359-11366 | 28 |
| **RLSSVDSDLSGC (RLSSVDS)** | *In vivo* phage display for rat brain homing peptides | Unknown | Peptide-phage present in the CSF of a rat | Urich et al. Scientific Reports 2015, 5,14104 | 29 |
| **THRPPMWSPVWP (THRPPMW)** | *In vitro* phage display on cells that constitutively express the hTfR | fransferrin receptor | Barrier penetration in an *in vitro* rat/bovine cell-based BBB model Optical demonstration of BBB penetration by transmission electron microscopy on rat brain sections | Lee et al. Eur. J. Biochem. 2001, 268, 7, 2004-2012 Prades et al. Biomaterials 2012, 33, 29, 7194-7205 | 30 |
| **TFFYGGSRGKRNNFKTEEYC (Angiopep-2)** | Neurotropic endogenous protein, derived from the Kunitz domain of aprotinin | Low density lipoprotein receptor | *In vivo* delivery of paclitaxel to mouse model of glioblastoma | Demeule et al. Br. J. Pharmacol. 155: 185, 2008. | 31 |
| **CMPRLRGC** | *In vitro* phage display on extracellular domain of human LDLR | Low density lipoprotein receptor | Optical demonstration of BBB penetration by multiphoton microscopy in live mice | Malcor et al. Journal of Medicinal Chemistry 2012;55(5):2227-41 | 32 |

### Secreted extracellular matrix protein Reelin is a receptor candidate of CVG

An *in vitro* proximity labeling method was planned to use for the identification of the CVGTNCY (SEQ ID NO:2) peptide receptor. To accomplish receptor search, binding of CVGTNCY (SEQ ID NO:2) peptide-phage on 22 different cell-lines isolated from various tumors and normal tissues was tested. Only minimal binding of CVGTNCY (SEQ ID NO:2) peptide-phage was observed on all the tested cell-lines (**Figure 12**; CVGTNCY (SEQ ID NO:2) peptide-phage and CVGTNCY (SEQ ID NO:2)-functionalized AgNP show low binding *in vitro*. CVGTNCY (SEQ ID NO:2) peptide-phage binding was tested on various tumorigenic and non-tumorigenic cell lines. The binding efficiency was evaluated based on the titer (PFU/ml) of each sample and shows low CVGTNCY (SEQ ID NO:2) ratio over the control phage in tested cell lines). That minimal binding was too low to carry out a proximity labeling of receptor proteins *in vitro*. In addition to low peptide-phage binding, binding of CVGTNCY (SEQ ID NO:2)-AgNPs was not detected with two GBM cell lines, WT-GBM and VEGFko *in vitro*. Moreover, when CVGTNCY (SEQ ID NO:2) peptide-phage binding was tested *ex vivo* on Balb/c brain homogenite, no binding was detected.

These results suggest that CVGTNCY (SEQ ID NO:2) receptor/binding partner is not accessible in artificial *in vitro* and *ex vivo* conditions and can be available only *in vivo*.

Next, an affinity chromatography was performed to identify a receptor of the CVGTNCY (SEQ ID NO:2), and selected a receptor candidate, which is a secreted extracellular matrix protein Reelin. CVGTNCY (SEQ ID NO:2) binding to Reelin protein was tested by cell free method on ELISA plate. Recombinant human Reelin protein (Ser1221-Gln2666; 163 kDa) was used to coat the ELISA plate and the CVGTNCY (SEQ ID NO:2) peptide-phage was allowed to bind to it. After several washes, the titer of the experiment was determined to rate on the binding efficiency. CVGTNCY (SEQ ID NO:2) showed specific binding to Reelin, while no binding was detected to NRP-1 and mutant NRP1 proteins (**Figure 13****;** CVGTNCY (SEQ ID NO:2) receptor candidate.

Reelin was identified from affinity chromatography proteomics data. Recombinant human Reelin protein (Ser1221-Gln2666; 8546-MR-050 R&D Systems) was coated to ELISA plate and CVGTNCY (SEQ ID NO:2) peptide-phage was incubated with the protein to allow binding. The binding efficiency was evaluated based on the titer (PFU/ml) of each sample. Control proteins were NRP1, mutant NRP1 and BSA; control phage was G7. CVGTNCY (SEQ ID NO:2) shows binding to Reelin protein, and not to the other tested proteins). This preliminary result suggests that Reelin acts as a receptor of CVGTNCY (SEQ ID NO:2).

### CVG peptide targets GBM cells

Homing of CVGTNCY (SEQ ID NO:2) was analyzed in angiogenic WT-GBM and infiltrative VEGFko GBM models. First, CVGTNCY (SEQ ID NO:2) peptide-phage homing was evaluated based on titer after systemic injection into the glioma-bearing mice and perfusion with PBS. CVGTNCY (SEQ ID NO:2) showed accumulation into both, the WT-GBM and VEGFko GBM, being 6 times and 2 times more present in respective GBMs compared to normal brain parenchyma (**Figures 14A-14C**; CVGTNCY (SEQ ID NO:2) peptide-phage was injected *iv* into mice bearing either WT-GBM or VEGFko GBM, and to mice without a tumor. At one hour time point mice were perfused, brains and control organs collected and titered; **Fig. 14A** shows CVGTNCY (SEQ ID NO:2) level in GBM compared to normal brain tissue, middle graph demonstrates the control G7 phage level in GBM and normal brain tissue, and right graph shows the ratio of CVGTNCY (SEQ ID NO:2) over the control phage in GBM and normal brain). At the same time the control G7 phage showed also binding with the GBM tissue *in vivo* like the CVGTNCY (SEQ ID NO:2) phage, G7 showed also a tendency to bind more to tumor than to the normal brain (**Fig. 14B**). On the contrary, the ratio of CVGTNCY (SEQ ID NO:2) over the G7 is higher in the normal brain parenchyma than in GBMs (**Fig. 14C**) indicating to the enhanced permeability and retention (EPR) effect in tumors.

The synthetic CVGTNCY (SEQ ID NO:2) peptide or scrambled CVGTNCY (SEQ ID NO:2) homing in WT-GBM and VEGFko GBM-bearing mice was analyzed after *i.v*. injection and perfusion of the animals. Cryosections of glioma-bearing brains were stained with anti-FAM antibody and tyramide amplification kit to enhance the signal of the peptide. CVGTNCY (SEQ ID NO:2) peptide was detected in the brain parenchyma and the tumor tissue in both GBM models. Scrambled CVGTNCY (SEQ ID NO:2) peptide was not detectable in the brain parenchyma, however binding was detected in the GBM (Distribution of CVGTNCY (SEQ ID NO:2) peptide in GBM and normal brain parenchyma was detected as follows: CVGTNCY (SEQ ID NO:2) peptide or its scrambled version (CVYTCNG (SEQ ID NO:68)) peptide was *i.v*. injected to glioma bearing mice, after one hour circulation mice were perfused and tissues of interest snap-frozen in liquid nitrogen. Immunohistochemical HRP-DAB staining was used to illustrate peptide targeting on brain cryosections. Images demonstrated that CVGTNCY (SEQ ID NO:2) peptide targets cortical neurons in brain parenchyma and activated microglia in GBM. The scrambled peptide showed no targeting to brain parenchyma, however it targets to GBM cells). In a closer look, the CVGTNCY (SEQ ID NO:2) peptide showed binding to the cortical neurons in the normal brain and to the activated microglia in GBM.

*CVG peptides have an adaptor protein in the blood plasma.*

It was demonstrated that CVGTNCY (SEQ ID NO:2) peptide-phage homes to the brain parenchyma after iv injection (*in vivo*) (**Fig. 15A**) On the contrary, CVG (CVGTNCY; SEQ ID NO:2) peptide-phage binding to brain tissue homogenate of perfused mouse is significantly disrupted (*ex vivo*). Control peptide-phage G7 shows higher binding to brain tissue homogenate *ex vivo* than *in vivo.* This result suggests that CVGTNCY (SEQ ID NO:2) peptide binding to brain parenchymal cells is dependent on the presence of a blood plasma protein.

Next, it was demonstrated that CVG peptide-phage binding to brain tissue homogenate *ex vivo* is restored in the presence of whole blood sample. Incubation of brain tissue homogenate with blood sample containing CVG peptide-phage significantly increase binding of the peptide to brain parenchymal cells (**Fig. 15B**). This result proves that CVG has an adaptor protein in the blood plasma that directs peptide targeting with the brain parenchymal cells. N=3; * p<0.05.

To assess the saturation of CVG receptor in the brain, CVG peptide-streptavidin complex (SA-CVG) was injected i.v in different amounts (0 µg, 10 µg, 30 µg, 100 µg). In 2 min, CVG peptide-phage as i.v injected. 10 min after phage injection, mice were perfused with PBS, brains homogenized and titered. Angiopep2 peptide-streptavidin complex (SA-Angiopep) and G7 phage were used as controls (**Fig. 16**).

It was demonstrated that an anti-Reelin antibody can cross the BBB after i.v administration. Reelin antibody (PA5-78413; Invitrogen) or control antibody (in-house made) was i.v. injected to mouse for 3-hour circulation. After cardiac perfusion, tissues were collected and snap-frozen in isopentane. Antibody was detected in brain on tissue sections with a proper secondary antibody.

Our observation that two different classes of Reelin ligands (peptides and antibodies) show increased brain tropism and BBB penetration indicate that Reelin can act as a bona fide BBB shuttle protein and that systemic Reelin ligands of different classes (e.g., antibodies, peptides, aptamers, LMW compounds) can be used to transport cargoes across BBB.

### CVG peptide homes to glioblastoma cells in brain

To assess homing of CVG peptide in glioblastoma, CVGTNCY (SEQ ID NO:2) peptide-phage was injected iv into mice bearing either WT-GBM, VEGFko, U87MG or NCH421k GBM, and to mice without a tumor. At one hour time point mice were perfused, brains and control organs collected and titered. The data showed that CVG PFU per mg of GBM was greater than normal brain tissue, and that the ratio of CVG over the G7 control phage in GBM was lower than in normal brain. (**Figs. 17A-17B**) Distribution of CVG peptide in GBM and brain parenchyma was also assessed. CVGTNCY (SEQ ID NO:2) peptide or its scrambled version (CVYTCNG; (SEQ ID NO:68)) was injected iv into glioma bearing mice, after 1 hour circulation mice were perfused and tissues of interest snap-frozen in liquid nitrogen. Immunohistochemical HRP-DAB staining was used to illustrate peptide targeting on brain cryosections, indicating that CVG peptide homed to brain parenchyma and GBM, whereas the Scrambled peptide showed no targeting to brain parenchyma,

*CVG peptide is engulfed by activated microglia cells in glioblastoma*.

WT GBM glioma- bearing mice were injected *iv* with FAM-labelled CVG peptide for 1 hour circulation. Mice were perfused and tissues of interest were snap-frozen in the vapor of liquid nitrogen. Immunofluorescent staining was carried out with the following antibodies: FAM, CD68, CD11b and CD206. CVG peptide showed colocalization with activated microglia markers CD68, CD11b and CD206 cells in the glioma edge and core. Scrambled CVG peptides showed also colocalization with CD68, CD11b and CD206. This result is suggesting that the peptide is engulfed by the activated microglia cells in glioma-bearing animals.

Finally, Ratiometric Laser Ablation-Inductively Coupled Plasma Mass Spectrometry (LA-ICP-MS) profiling of CVG (CVGTNCY; SEQ ID NO:2)-AgNP was carried out on glioma-bearing brain sections. VEGFko, NCH421k and U87-MG tumors were intracranially induced in nude mice. A mixture of CVGTNCY-Ag107NPs (OD=100) and control biotin-Ag109NPs (OD=100) was injected *iv* into tumor-bearing animals for 3-hour circulation. Thereafter, mice were perfused, organs (brain and liver) collected, snap-frozen in the vapor of nitrogen, and sectioned at 30 µm. The results showed that the ratio of CVG-Ag107NPs to control-Ag109NPs is higher in the brain parenchyma than in glioma.

### Embodiments

Embodiment 1. A brain penetrating peptide that is capable of traversing the blood brain barrier (BBB) of a subject *in vivo,* comprising a BBB-traversing domain comprising the amino acid sequence RRVISRAKLAAAL (SEQ ID NO:1), or a functional variant thereof.

Embodiment 2. A brain penetrating peptide that is capable of traversing the blood brain barrier (BBB) of a subject *in vivo,* comprising a BBB-traversing domain comprising the amino acid sequence of CVGTNCY (SEQ ID NO:2), or a functional variant thereof.

Embodiment 3. The brain penetrating peptide of embodiment 1, wherein the BBB-traversing domain is at least 13 amino acids in length.

Embodiment 4. The brain penetrating peptide of embodiment 1 or 3, wherein the BBB-traversing domain comprises between 5-20 contiguous amino acids of one or more of SEQ ID NOs:3-10, or a functional variant thereof.

Embodiment 5. The brain penetrating peptide of embodiment 1 or 3 or 4, wherein the BBB-traversing domain comprises a functional variant of any one of SEQ ID NOs:1, or 3-10, having an amino acid sequence at least 70%, 75%, 80%, 85%, 90%, 95%, or 99% identical to any one of SEQ ID NOs:1 or 3-10.

Embodiment 6. The brain penetrating peptide of embodiment 2, wherein the BBB-traversing domain is at least 7 amino acids in length.

Embodiment 7. The brain penetrating peptide of embodiment 2 or 6, wherein the BBB-traversing domain comprises between 5-10 contiguous amino acids of one or more of SEQ ID NOs:11-20, 22-23, or a functional variant thereof.

Embodiment 8. The brain penetrating peptide of any one of embodiments 1-7, wherein the amino acid sequence of the BBB-traversing domain is not CAGALCY (SEQ ID NO:21).

Embodiment 9. The brain penetrating peptide of any one of embodiments 1-8, wherein the brain penetrating peptide selectively homes to the brain parenchyma, endothelial cells, or the whole brain.

Embodiment 10. The brain penetrating peptide of embodiment 9, wherein the BBB-traversing domain is or comprises SRRVISRAKLAAAL (SEQ ID NO:9); SRRVISRAKLAAALE (SEQ ID NO:3); or MLGDPILASRRVISRAKLAAALE (SEQ ID NO:4), and
wherein the brain penetrating peptide selectively homes to the brain parenchyma cells.

Embodiment 11. A peptide conjugate comprising
(a) the brain penetrating peptide of any one of embodiments 1-10; and
(b) a cargo molecule,

wherein the cargo molecule is directly or indirectly conjugated to or complexed with the brain penetrating peptide, and
wherein the cargo molecule does not traverse the BBB in the absence of the brain penetrating peptide.

Embodiment 12. The peptide conjugate of embodiment 11, wherein the cargo molecule comprises one or more active agent selected from the group consisting of a therapeutic agent, a diagnostic agent, a prophylactic agent and a nutraceutical agent.

Embodiment 13. The peptide conjugate of embodiment 11 or 12, wherein the cargo molecule is encapsulated within or conjugated to a carrier, optionally wherein the carrier is conjugated to the brain penetrating peptide.

Embodiment 14. The peptide conjugate of embodiment 13, wherein the carrier comprises a polymeric particle, a lipid particle, a liposome, a gel, an inorganic particle, a viral particle, a nucleic acid nanostructure, and a virus-like particle.

Embodiment 15. The peptide conjugate of embodiment 13, wherein the carrier is conjugated to the brain penetrating peptide by one or more linkers,
optionally wherein the one or more linkers are cleavable linkers.

Embodiment 16. The peptide conjugate of any one of embodiments 12-15, wherein the active agent is a therapeutic agent selected from the group consisting of a nucleic acid, a peptide, a lipid, a glycolipid, a glycoprotein, and a small molecule.

Embodiment 17. The peptide conjugate of embodiment 16, wherein the therapeutic agent is a nucleic acid selected from the group consisting of antisense molecules, aptamers, ribozymes, triplex forming oligonucleotides, external guide sequences, RNAi, CRISPR/Cas, zinc finger nucleases, and transcription activator-like effector nucleases (TALENs).

Embodiment 18. The peptide conjugate of embodiment 16, wherein the therapeutic agent is a small molecule.

Embodiment 19. The peptide conjugate of any one of embodiments 16-18, wherein the therapeutic agent is selected from the group consisting of an anti-cancer agent, an anti-inflammatory agent, and an antimicrobial agent.

Embodiment 20. A pharmaceutical composition comprising the peptide conjugate of any one of embodiments 16-19, and a pharmaceutically acceptable excipient for administration.

Embodiment 21. The pharmaceutical composition of embodiment 20, wherein the composition is suitable for mucosal, pulmonary, intravenous, or intramuscular delivery.

Embodiment 22. A method of treating or preventing one or more symptoms of a disease or disorder in the brain or CNS of a subject comprising
administering to the subject an effective amount of a pharmaceutical composition of embodiment 20 or 21 to prevent or reduce one or more symptoms of a disease or disorder in the brain or CNS of a subject in the subject.

Embodiment 23. The peptide conjugate of any one of embodiments 12-15, wherein the active agent is a diagnostic agent selected from the group consisting of a dye, a radionuclide, a fluorescent tag, a magnetic tag, and a nanoparticle.

Embodiment 24. A pharmaceutical composition comprising the peptide conjugate of embodiment 23 and a pharmaceutically acceptable excipient for administration.

Embodiment 25. The pharmaceutical composition of embodiment 24, wherein the composition is suitable for mucosal, pulmonary, intravenous, or intramuscular delivery.

Embodiment 26. A formulation of the peptide conjugate of any one of embodiments 11-19 for treating or preventing one or more symptoms of a disease or disorder in the brain or CNS of a subject, or a pharmaceutical composition thereof for use in treating or preventing one or more symptoms of a disease or disorder in the brain or CNS of a subject are also provided.

Embodiment 27. Use of the peptide conjugate of any one of embodiments 11-19 in the manufacture of a medicament for treating or preventing one or more symptoms of a disease or disorder in the brain or CNS of a subject, wherein the conjugates including one or more therapeutic agents.

Embodiment 28. A method of detecting or monitoring a disease or disorder in the brain or CNS of a subject comprising
administering to the subject an effective amount of a pharmaceutical composition of embodiment 24 or 25 to detect or monitor a disease or disorder in the brain or CNS of a subject in the subject.

Embodiment 29. The method of any one of embodiments 22 or 28, wherein the subject is a human.

Embodiment 30. The method of any one of embodiments 22 or 28 or 29, wherein the subject has, or is suspected as having a disease selected from the group consisting of cancer, an inflammatory disease, a neuronal disorder, HIV/AIDS, a diabetes, a cardiovascular disease, an infectious disease (including a viral, a protozoan, a bacterial disease, and an allergy), an autoimmune disease and an autoimmune disease, Alzheimer's disease, Parkinson's disease, ischemia, a neurodegenerative disorder, and a genetic disorder.

Embodiment 31. The method of embodiment 30, wherein the disease is a cancer.

Embodiment 32. The method of embodiment 30, wherein the disease is Alzheimer's disease.

Embodiment 33. The method of embodiment 30, wherein the disease is Parkinson's disease.

Embodiment 34. The method of embodiment 30, wherein the disease is a neurodegenerative disorder.

Embodiment 35. The method of any one of embodiments 22 or 28-34, wherein the subject has an infection.

Embodiment 36. A brain penetrating peptide comprising the amino acid sequence of any one of SEQ ID NOs:77-96.

Embodiment 37. The peptide of embodiment 36, further comprising a cargo molecule,
wherein the cargo molecule is directly or indirectly conjugated to or complexed with the brain penetrating peptide, and
wherein the cargo molecule does not traverse the BBB in the absence of the brain penetrating peptide.

Embodiment 38. A brain penetrating peptide conjugate that is capable of traversing the blood brain barrier (BBB) of a subject *in vivo,* comprising
(a) a BBB-traversing domain comprising the amino acid sequence RRVISRAKLAAAL (SEQ ID NO:1), or a functional variant thereof; and
(b) an active agent selected from the group consisting of a therapeutic agent, a diagnostic agent and a prophylactic agent;
wherein the active agent is conjugated with the BBB-traversing domain, optionally wherein the active agent is conjugated *via* a linker.

Embodiment 39. A brain penetrating peptide conjugate that is capable of traversing the blood brain barrier (BBB) of a subject *in vivo,* comprising
(a) a BBB-traversing domain comprising the amino acid sequence CVGTNCY (SEQ ID NO:2); and
(b) an active agent selected from the group consisting of a therapeutic agent, a diagnostic agent and a prophylactic agent;
wherein the active agent is conjugated with the BBB-traversing domain, optionally wherein the active agent is conjugated *via* a linker.

Embodiment 40. A brain penetrating peptide conjugate that is capable of traversing the blood brain barrier (BBB) of a subject *in vivo,* comprising
(a) a BBB-traversing domain comprising the amino acid sequence of any one of (SEQ ID NO:77-96); and
(b) an active agent selected from the group consisting of a therapeutic agent, a diagnostic agent and a prophylactic agent;
wherein the active agent is conjugated with the BBB-traversing domain, optionally wherein the active agent is conjugated *via* a linker.

Embodiment 41. A method of delivering an active agent selectively to one or more structures of the brain of a subject, comprising administering to the subject the composition of any one of embodiments 38-40,
wherein the composition is administered to the subject *via* a systemic route.

Embodiment 42. The method of embodiment 41, wherein the active agent comprises a therapeutic and/or prophylactic agent in an amount effective to treat or prevent one or more symptoms of a disease or disorder in the subject.

Embodiment 43. The method of embodiment 42, wherein the therapeutic and/or prophylactic agent comprises one or more of a protein, a carbohydrate, a lipid, a small molecule or a nucleic acid.

Embodiment 44. The method of embodiment 43, wherein the protein comprises an immunoglobulin, or an antigen-binding fragment thereof.

Embodiment 45. The method of any one of embodiments 42-44, wherein the therapeutic and/or prophylactic agent does not traverse the BBB in the absence of the brain penetrating peptide.

Embodiment 46. The method of any one of embodiments 42-45, wherein the one or more structures of the brain is selected from the group consisting of the cortex, the hippocampus and the brain stem.

Embodiment 47. The method of any one of embodiments 42-46, wherein the effective amount of the therapeutic and/or prophylactic when administered to the subject as the conjugate with the BBB-traversing domain is less than the effective amount of the same therapeutic and/or prophylactic agent in the absence of the BBB-traversing domain.

Embodiment 48. The method of any one of embodiments 42-47, wherein the BBB-traversing domain comprises the amino acid sequence RRVISRAKLAAAL (SEQ ID NO:1), and
wherein the active agent is selectively delivered to one or more of astrocytes, microglia or mature neurons in the brain of the subject.

Embodiment 49. The method of any one of embodiments 42-47, wherein the BBB-traversing domain comprises the amino acid sequence CVGTNCY (SEQ ID NO:2); and
wherein the active agent is selectively delivered to activated microglia in the brain of the subject.

Embodiment 50. The method of embodiment 49, wherein the subject has cancer, and
wherein the active agent is selectively delivered to the region of a tumor and/or brain damage within the brain of the subject.

Embodiment 51. The method of embodiment 49 or 50, wherein the tumor is a glioblastoma.

Embodiment 52. The method of any one of embodiments 49-52, wherein the active agent is a chemotherapeutic agent.

The disclosures in European patent application number 23764718.5, from which this application is divided, and in US provisional patent application number 63/373,160, from which this application claims priority, are incorporated by reference herein in their entirety.

### References

Blouw, B, Song, H, Tihan, T, Bosze, J, Ferrara, N, Gerber, H-P, Johnson, RS, and Bergers, G (2003). The hypoxic response of tumors is dependent on their microenvironment. Cancer Cell 4, 133-146.
Braun, GB et al. (2014). Etchable plasmonic nanoparticle probes to image and quantify cellular internalization. Nat Mater 13, 904-911.
Demeule, M, Régina, A, Ché, C, Poirier, J, Nguyen, T, Gabathuler, R, Castaigne, J-P, and Béliveau, R (2008). Identification and design of peptides as a new drug delivery system for the brain. J Pharmacol Exp Ther 324, 1064-1072.
Dirkse, A et al. (2019). Stem cell-associated heterogeneity in Glioblastoma results from intrinsic tumor plasticity shaped by the microenvironment. Nat Commun 10, 1787.
Pleiko, K, Põšnograjeva, K, Haugas, M, Paiste, P, Tobi, A, Kurm, K, Riekstina, U, and Teesalu, T (2021). In vivo phage display: identification of organ-specific peptides using deep sequencing and differential profiling across tissues. Nucleic Acids Res 49, e38-e38.
Põšnograjeva, K, Pleiko, K, Haugas, M, and Teesalu, T (2022). New Tools for Streamlined In Vivo Homing Peptide Identification. In: Cell Penetrating Peptides, ed. Ü Langel, New York, NY: Springer US, 385-412.
Tobi, A, Willmore, AA, Kilk, K, Sidorenko, V, Braun, GB, Soomets, U, Sugahara, KN, Ruoslahti, E, and Teesalu, T (2021). Silver Nanocarriers Targeted with a CendR Peptide Potentiate the Cytotoxic Activity of an Anticancer Drug. Adv Ther 4, 2000097.
Toome, K, Willmore, A-MA, Paiste, P, Tobi, A, Sugahara, KN, Kirsimäe, K, Ruoslahti, E, Braun, GB, and Teesalu, T (2017). Ratiometric in vivo auditioning of targeted silver nanoparticles. Nanoscale 9, 10094-10100.

## Claims

1. A reelin-binding ligand for transporting cargo across the blood brain barrier (BBB), comprising a BBB-traversing domain, wherein the BBB-traversing domain is a polypeptide capable of traversing the BBB of a subject *in vivo.*

2. The reelin-binding ligand of claim 1, wherein the ligand is a peptide, an antibody, an aptamer or a low-molecular-weight compound.

3. The reelin-binding ligand of claim 1 or 2, wherein the BBB-traversing domain is or includes a polypeptide having a sequence of between 5 and 25 contiguous amino acids of one or more of SEQ ID NOs:2, 11-20, and 22-23.

4. The reelin-binding ligand of any preceding claim, wherein the BBB-traversing domain selectively homes to the hippocampus, frontal cortex, cerebellum, brain parenchyma, or the whole CNS.

5. A brain penetrating peptide that is capable of traversing the blood brain barrier (BBB) of a subject *in vivo,* comprising a BBB-traversing domain comprising the amino acid sequence RRVISRAKLAAAL (SEQ ID NO:1), or a functional variant thereof,
optionally wherein the brain penetrating peptide selectively homes to the brain parenchyma, endothelial cells, or the whole brain.

6. The brain penetrating peptide of claim 5, wherein the BBB-traversing domain is or comprises SRRVISRAKLAAAL (SEQ ID NO:9); SRRVISRAKLAAALE (SEQ ID NO:3); or MLGDPILASRRVISRAKLAAALE (SEQ ID NO:4), and
wherein the brain penetrating peptide selectively homes to the brain parenchyma cells.

7. A peptide conjugate comprising
(a) the reelin-binding ligand of any of claims 1 to 4, wherein the ligand is a peptide or the brain penetrating peptide of claim 5 or 6;
(b) a cargo molecule,
wherein the cargo molecule is directly or indirectly conjugated to or complexed with the peptide, and
wherein the cargo molecule does not traverse the BBB in the absence of the peptide.

8. The peptide conjugate of claim 7, wherein the peptide is a reelin-binding ligand, wherein the peptide conjugate comprises one or more targeting moieties, and wherein the targeting moiety selectively binds reelin.

9. The peptide conjugate of claim 7 or 8, wherein the cargo molecule comprises one or more active agents selected from the group consisting of:
a therapeutic agent, optionally wherein the therapeutic agent is selected from the group consisting of a nucleic acid, a peptide, a lipid, a glycolipid, a glycoprotein, and a small molecule, optionally wherein when the therapeutic agent is a nucleic acid, the nucleic acid is selected from the group consisting of antisense molecules, aptamers, ribozymes, triplex forming oligonucleotides, external guide sequences, RNAi, CRISPR/Cas, zinc finger nucleases, and transcription activator-like effector nucleases (TALENs); or wherein: the therapeutic agent is selected from the group consisting of an anti-cancer agent, an anti-inflammatory agent, and an antimicrobial agent;
a prophylactic agent;
a nutraceutical agent; and
a diagnostic agent, optionally wherein the diagnostic agent is selected from the group consisting of a dye, a radionuclide, a fluorescent tag, a magnetic tag, and a nanoparticle.

10. The peptide conjugate of claim 7, 8 or 9, wherein the cargo molecule is encapsulated within or conjugated to a carrier, and wherein the carrier is conjugated to the peptide.

11. The peptide conjugate of claim 10, wherein the carrier comprises a polymeric particle, a lipid particle, a liposome, a gel, an inorganic particle, a viral particle, a nucleic acid nanostructure, or a virus-like particle; or wherein:
the carrier is conjugated to the peptide by one or more linkers, optionally wherein the one or more linkers are cleavable linkers.

12. The peptide conjugate of any one of claims 7 to 11 for use in a method of delivering an active agent selectively to one or more structures of the brain of a subject, wherein the active agent comprises a therapeutic and/or prophylactic agent in an amount effective to treat or prevent one or more symptoms of a disease or disorder in the subject.

13. The peptide conjugate of any one of claims 7 to 12, wherein the cargo molecule comprises an active agent and the active agent is a therapeutic agent selected from the group consisting of a nucleic acid, a peptide, a lipid, a glycolipid, a glycoprotein, and a small molecule, optionally wherein when the therapeutic agent is a nucleic acid, the nucleic acid is selected from the group consisting of antisense molecules, aptamers, ribozymes, triplex forming oligonucleotides, external guide sequences, RNAi, CRISPR/Cas, zinc finger nucleases, and transcription activator-like effector nucleases (TALENs); or wherein:
the therapeutic agent is selected from the group consisting of an anti-cancer agent, an anti-inflammatory agent, and an antimicrobial agent.

14. A pharmaceutical composition comprising the peptide conjugate of any one of claims 7 to 13, and a pharmaceutically acceptable excipient for administration, optionally wherein the composition is suitable for mucosal, pulmonary, intravenous, or intramuscular delivery, the pharmaceutical composition optionally being for use in a method of treating or preventing one or more symptoms of a disease or disorder in the brain or CNS of a subject, or for detecting or monitoring a disease or disorder in the brain or CNS of a subject, wherein the subject is a human;
the pharmaceutical composition optionally for use where the subject has, or is suspected as having a disease selected from the group consisting of cancer, an inflammatory disease, a neuronal disorder, HIV/AIDS, diabetes, a cardiovascular disease, an infectious disease (including a viral, a protozoan, a bacterial disease, and an allergy), an autoimmune disease, Alzheimer's disease, Parkinson's disease, ischemia, a neurodegenerative disorder, and a genetic disorder.

15. A brain penetrating peptide conjugate that is capable of traversing the blood brain barrier (BBB) of a subject *in vivo,* comprising
(a) a brain penetrating peptide as claimed in claim 5 or 6; and
(b) an active agent selected from the group consisting of a therapeutic agent, a diagnostic agent and a prophylactic agent;
wherein the active agent is conjugated with the BBB-traversing domain, optionally wherein the active agent is conjugated *via* a linker, optionally wherein the brain penetrating peptide conjugate is for use in a method of delivering an active agent selectively to one or more structures of the brain of a subject.
